# EUROPEAN PATENT SPECIFICATION

(11) **EP 4 365 906 B1**
(45) Date of publication and mention of the grant of the patent: **15.10.2025**
(21) Application number: 22853140.6
(22) Date of filing: 04.08.2022
(51) Int. Cl.: G16H 20/60

(54) **PROGRAM, INFORMATION PROCESSING METHOD, AND INFORMATION PROCESSING DEVICE**
PROGRAMM, INFORMATIONSVERARBEITUNGSVERFAHREN UND INFORMATIONSVERARBEITUNGSVORRICHTUNG
PROGRAMME, PROCÉDÉ DE TRAITEMENT D'INFORMATIONS ET DISPOSITIF DE TRAITEMENT D'INFORMATIONS

(30) Priority: 04.08.2021 JP 2021128356
(43) Date of publication of application: 08.05.2024
(73) Proprietor: Mizkan Holdings Co., Ltd., Handa-shi, Aichi 475-8585 (JP)
(72) Inventor: KISHI Mikiya, Handa-shi, Aichi 475-8585 (JP); ISHII Shou, Handa-shi, Aichi 475-8585 (JP); TSUCHIYA Yoshihiro, Handa-shi, Aichi 475-8585 (JP); IHARA Junichiro, Handa-shi, Aichi 475-8585 (JP); NAKANISHI Isao, Handa-shi, Aichi 475-8585 (JP)
(74) Representative: Hasegawa, Kan
(86) International application number: PCT/JP2022/029902
(87) International publication number: WO 2023/013722

(56) References cited:
- WO-A1-2020/158686
- WO-A1-2020/158686
- CN-A- 113 140 287
- JP-A- 2013 190 915
- JP-A- 2013 190 915
- JP-A- 2020 504 362
- JP-A- 2020 504 362
- US-A1- 2018 300 460
- US-A1- 2018 300 460

## Description

### [Technical Field]

The present invention relates to a program, an information processing method, and an information processing device.

### [Background Art]

With the spread of the Internet, various kinds of information have been provided through the Internet. In regard to technologies of providing various kinds of information through the Internet, a technology that enables a situation where a user can more easily receive information for behavior support which is generated is known (see JP 2020 170217 A, for example).

US 2018/300460 A1 discloses a system for improving appetite through positive memory association. Information is received including text content related to a user. A positive user sentiment is defined using sentiment analysis. Text analysis is applied on the text content to identify a factor in the information that correlates with the positive user sentiment. The factor is ranked based on a strength of the correlation. Responsive to determining that the strength of the correlation is above a predefined threshold, the factor is stored as a positive token. A list of food recipes is accessed to identify a food recipe that incorporates the positive token. The food recipe is displayed to the user.

### [Summary of Invention]

### [Technical Problem]

However, according to a conventional system as disclosed in Patent Literature 1, it is difficult to accurately grasp whether a factor has affected real feelings, such as which input information has been effective or has not been effective for a user, in regard to everyday input information in which food (details of meals) that the user has had, behaviors, and the like are mixed in a complicated manner. Furthermore, there is a problem that it is difficult to verify whether an effect given by such factors is an effect that gives a medium-to-long-term real feeling over a specific period of time rather than an effect that gives transitory pleasure to the user, without investigation of the factors for each region or each group.

Additionally, in clinical tests that have been conventionally done in order to verify health effects of foods, it is necessary to provide an intake period and a non-intake period of a specific food and to verify the effects in an unusual environment in which conditions of test objects, such as prohibition of alcohol and smoking, are adjusted. The conventional clinical tests have not used a method of picking up a target food, and in the first place, they have not used a method for verifying an effect of raising a user's long-term real feeling score such as life satisfaction.

The present invention was made in view of such circumstances, and an object thereof is to provide a program and the like capable of providing, to a user, information regarding a food intake mode that gives a medium-to-long-term real feeling over a specific period of time.

### [Solution to Problem]

The invention is defined by the appended claims. A program according to an aspect of the present disclosure causes a computer to: acquire intake information regarding foods that a user has taken; acquire one or more pieces of information from among feeling information, biological information, and behavior information of the user and/or real feeling score information derived from such information; output, to an information processing unit, the acquired intake information, the one or more pieces of information from among the feeling information, the biological information, and the behavior information, and/or the real feeling score information derived from such information, and identification information of the user; and acquire, from the information processing unit, advice information including information regarding a correlation between the intake information and the real feeling score information.

### [Advantageous Effects of Invention]

According to an aspect of the present invention, it is possible to provide, to a user, information regarding a food intake mode that gives a medium-to-long-term real feeling over a specific period of time.

### [Brief Description of Drawings]

FIG. 1 is a schematic view illustrating a configuration example of an information processing system.
FIG. 2 is a block diagram illustrating a configuration example of a server and a user terminal.
FIG. 3 is a schematic view illustrating a configuration example of a DB stored in the server.
FIG. 4 is a schematic view illustrating the configuration example of the DB stored in the server.
FIG. 5 is a schematic view illustrating the configuration example of the DB stored in the server.
FIG. 6 is a flowchart illustrating an example of a user information registration processing procedure.
FIG. 7 is a flowchart illustrating the example of the user information registration processing procedure.
FIG. 8A is a schematic view illustrating a screen example of the user terminal.
FIG. 8B is a schematic view illustrating a screen example of the user terminal.
FIG. 9A is a schematic view illustrating a screen example of the user terminal.
FIG. 9B is a schematic view illustrating a screen example of the user terminal.
FIG. 10A is a schematic view illustrating a screen example of the user terminal.
FIG. 10B is a schematic view illustrating a screen example of the user terminal.
FIG. 11 is a flowchart illustrating an example of an advice providing processing procedure.
FIG. 12 is a schematic view illustrating a screen example of the user terminal.
FIG. 13 is a schematic view illustrating a configuration example of a learning model.
FIG. 14 is a flowchart illustrating an example of a user information registration processing procedure according to a second embodiment.
FIG. 15A is a schematic view illustrating a screen example of a user terminal.
FIG. 15B is a schematic view illustrating a screen example of the user terminal.
FIG. 16 is a schematic view illustrating a configuration example of a target amount DB.
FIG. 17A is a schematic view illustrating a registration screen example according to a third embodiment.
FIG. 17B is a schematic view illustrating a target information input screen example.
FIG. 18 is a flowchart illustrating an example of an advice providing processing procedure according to the third embodiment.
FIG. 19A is a schematic view illustrating a screen example of a user terminal.
FIG. 19B is a schematic view illustrating a screen example of the user terminal.

### [Description of Embodiments]

Hereinafter, a program, an information processing method, and an information processing device according to the present disclosure will be described in detail on the basis of the drawings illustrating embodiments thereof.

### (First embodiment)

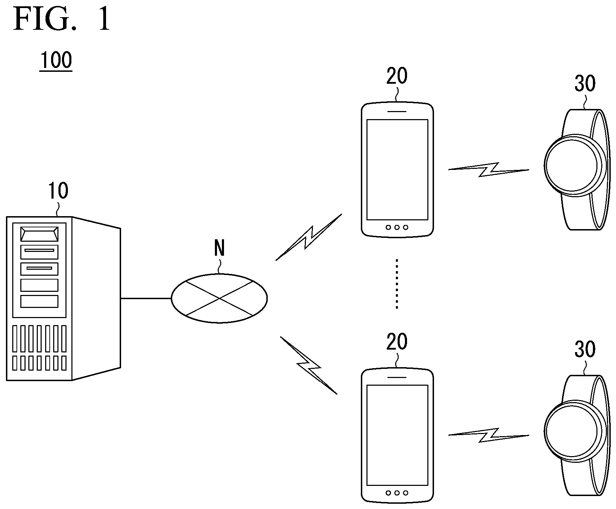

FIG. 1 is a schematic view illustrating a configuration example of an information processing system according to the present embodiment. An information processing system 100 according to the present embodiment includes a server 10, a plurality of user terminals 20, and the like, and the server 10 and the user terminals 20 are connected via a network N such as the Internet. The server 10 is an information processing device capable of performing various kinds of information processing and transmitting and receiving information and is, for example, a server computer, a personal computer, or the like. A plurality of servers 10 may be provided, or a server 10 may be implemented by a plurality of virtual machines provided in one server device or may be implemented by using a cloud server.

The user terminals 20 are terminals of users who take foods, are information processing devices (computers) such as smartphones, tablet terminals, or personal computers, and may be configured by dedicated terminals. Also, the information processing system 100 according to the present embodiment includes a wearable device 30 used by each user, and the corresponding user terminal 20 and the wearable device 30 can perform wireless communication.

The wearable device 30 is configured to be able to measure biological information of each user such as a body temperature, a blood pressure, a heart rate, a pulse rate, sweating, brain waves, emotional hormones, and stress hormones, motion information such as a number of steps, a moving distance, and a moving time, and sleep information such as a sleeping time. For the wearable device 30, a user terminal 20 as a communication counterpart is set in advance, and the wearable device 30 transmits various kinds of measured information to the user terminal 20 as a communication counterpart. The wearable device 30 may be configured as a watch type as illustrated in FIG. 1 or may be configured as a glasses type, a ring type, or the like. Note that the information processing system 100 according to the present embodiment may be configured by a block chain (a distributed ledger technology or a distributed network) including the plurality of user terminals 20 as nodes without including the server 10.

In the information processing system 100 according to the present embodiment, the user terminals 20 perform various kinds of information processing such as processing of receiving various kinds of information regarding the users input by the users and processing of transmitting received information to the server 10. The server 10 performs various kinds of information processing such as processing of registering information regarding the users received from the user terminals 20 and processing of providing advice based on the registered information to the respective users.

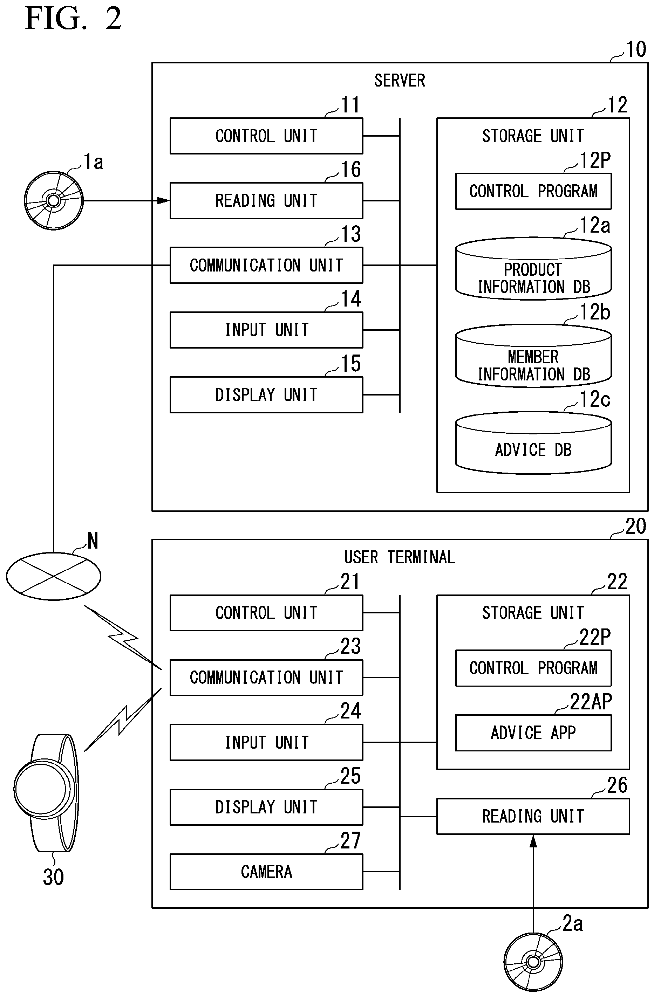

FIG. 2 is a block diagram illustrating a configuration example of the server 10 and the user terminal 20. The server 10 includes a control unit 11, a storage unit 12, a communication unit 13, an input unit 14, a display unit 15, a reading unit 16, and the like, and each of these components is connected to the others via a bus. The control unit 11 includes one or more processors such as a central processing unit (CPU), a micro-processing unit (MPU), or a graphics processing unit (GPU). The control unit 11 executes various kinds of information processing, control processing, and the like that are to be performed by the server 10, by appropriately executing a control program 12P stored in the storage unit 12.

The storage unit 12 includes a random access memory (RAM), a flash memory, a hard disk, a solid state drive (SSD), or the like. The storage unit 12 stores the control program 12P to be executed by the control unit 11 and various kinds of data and the like that are necessary to execute the control program 12P in advance. Also, the storage unit 12 temporarily stores data and the like generated when the control unit 11 executes the control program 12P. Moreover, the storage unit 12 stores a product information database (DB) 12a, a member information DB 12b, an advice DB 12c, and the like, which will be described later. The product information DB 12a, the member information DB 12b, and the advice DB 12c may be stored in another storage device connected to the server 10 or may be stored in another storage device with which the server 10 can communicate.

The communication unit 13 is an interface for establishing connection to the network N through wired communication or wireless communication and transmits and receives information to and from another device via the network N. The input unit 14 includes, for example, a mouse, a keyboard, and the like, receives operation inputs by a manager who manages the server 10, and sends a control signal corresponding to operation details to the control unit 11. The display unit 15 is a liquid crystal display, an organic EL display, or the like and displays various kinds of information in response to an instruction from the control unit 11. The input unit 14 and the display unit 15 may be an integrally configured touch panel.

The reading unit 16 reads information stored in a portable storage medium 1a including a compact disc (CD)-ROM, a digital versatile disc (DVD)-ROM, a universal serial bus (USB) memory, a secure digital (SD) card, or the like. The control program 12P and the various kinds of data stored in the storage unit 12 may be read by the control unit 11 from the portable storage medium 1a via the reading unit 16 and may be stored in the storage unit 12. Also, the control program 12P and the various kinds of data stored in the storage unit 12 may be downloaded from another device via the communication unit 13 by the control unit 11 and may be stored in the storage unit 12.

The user terminal 20 includes a control unit 21, a storage unit 22, a communication unit 23, an input unit 24, a display unit 25, a reading unit 26, a camera 27, and the like, and each of these components is connected to the others via a bus. Each of the components 21 to 26 of the user terminal 20 has a configuration similar to that of each of the components 11 to 16 of the server 10, and detailed description of the configurations will thus be omitted. Note that the storage unit 22 of the user terminal 20 stores, in addition to the control program 22P to be executed by the control unit 21, an advice application program 22AP (hereinafter, referred to as an advice APP 22AP) which is a program according to the present disclosure and is for performing processing of acquiring, from the server 10, an appropriate advice in accordance with information regarding foods that the user has taken (hereinafter, simply referred to as "intake information" or "user intake information" in some cases), one or more pieces of information from among information specifying a feeling of the user (hereinafter, simply referred to as "feeling information" or "user feeling information" in some cases), information specifying a living body of the user (hereinafter, simply referred to as "biological information" or "user biological information" in some cases), and information specifying a behavior of the user (hereinafter, simply referred to as "behavior information" or "user behavior information" in some cases), and/or information (hereinafter, simply referred to as "real feeling score information" or "user real feeling score information" in some cases) derived from such information (the user intake information and the one or more pieces of information from among the user feeling information, the user biological information, and the user behavior information). The "user XX information" used for explanation of the present invention represents a meaning of "XX information which is characteristic information of the user" rather than a meaning representing an origin of the XX information and has the same meaning as that of just "XX information".

The "user real feeling score information" in the present embodiment represents a score derived from information with a high correlation with a middle-to-long-term feeling (real feeling) over a specific period of time from among the "user feeling information", the "user biological information", and the "user behavior information". The "user real feeling score information" in the present embodiment may be data obtained by representing, as a score, information with a high correlation with the medium-to-long-term feeling over the specific period of time from among the "user feeling information", the "user biological information", and the "user behavior information" or may be a total score derived by multiplying such one or more pieces of data by a coefficient in accordance with the correlation.

Also, the storage unit 22 of the user terminal 20 stores score information of each of the items of the "user feeling information", the "user biological information", and the "user behavior information" when the "user real feeling score information" is acquired, a point for converting the score information of each item into user real feeling score information, a conversion coefficient, and a chart (a biorhythm chart, a radar chart, a color chart, or the like).

As an example of the user real feeling score, a numerical value of a positive point or a negative point is selected on the basis of one or more pieces of information from among user feeling information, user biological information, and user behavior information input to the input unit 24 of the user terminal 20 and a keyword in each of feeling information, biological information, and behavior information stored in the storage unit 22, an arithmetic average of the selected numerical value is obtained by the control program 22P, and results of arithmetic averages are summed, thereby deriving the user real feeling score. Here, the selected numerical value may be multiplied by a correction coefficient specified for each user. Also, a method of calculating a real feeling score by multiplying a numerical value selected from any of the acquired "user feeling information", "user biological information", and "user behavior information" by a correction coefficient may be employed, a neural network calculation method of calculating a real feeling score via a plurality of temporary scores obtained by multiplying the numerical value by a correction coefficient may be used, or furthermore, a deep learning method of calculating a real feeling score via multiple stages of calculating temporary scores may be employed. Also, a numerical value of each item, a positive point, and a negative point may be plotted on a radar chart, and an area value of the radar chart obtained by the plotting may be calculated.

As an example of the user real feeling score, the user real feeling score is derived by calculating and using a value multiplied by a correlation coefficient calculated for each explanatory variable (user feeling information, user biological information, user behavior information) through multivariate analysis using a medium-to-long-term feeling (real feeling) score over a specific period of time as a target variable. Here, only a highly correlated explanatory variable, an absolute value of a correlation coefficient f which is equal to or greater than a specific value, may be used as needed.

Specifically, a questionnaire survey when vinegar is taken every day for XX days is conducted, information such as an "age", a "sex", an "occupation", a "disease history", and the like is acquired as attribute information of the user, information such as "XX mL of vinegar was taken at breakfast (lunch, snack, or dinner)", "XX was taken in breakfast (lunch, snack, or dinner)", and the like is acquired as intake information, information such as "concentration is enhanced", "bright mood is felt", "vitality is felt", and the like is acquired as user feeling information, information such as a "user feeling information when XX mL is taken every day", a "weight", a "BMI", a "body fat percentage", a "blood pressure", and the like are acquired as user biological information, and information such as "XX km running is performed at night", "XX minutes walking is performed", and "XX minutes stretching is performed in early morning", and the like is acquired as behavior information, and logistic regression analysis is conducted on the obtained information. As a result, a correlation of the user feeling information such as "concentration is enhanced" or "bright mood is felt" XX days after the start of intake with user behavior information "running" is stored as a negative point, and a correlation of the user feeling information "vitality is felt" XX days after the start of intake with "running" as user behavior information is stored as a positive point, for example, in the storage unit. On the other hand, a correlation with "stretch" as user behavior information with respect to user feeling information "concentration is enhanced" XX days after the start of intake is stored as a positive point, for example, in the storage unit. Also, analysis (for example, XGBoost, LightGBM, or RandomForest) using a determination tree model from the same data is performed, an explanatory variable with high importance both as an explanatory variable obtained as a result of the logistic regression analysis and in the determination tree model regression analysis is used, and it is thus possible to perform analysis with high accuracy.

Next, information such as "10 km running is performed in the morning" is acquired as behavior information of the user who uses the advice in the present embodiment other than the target on which this questionnaire has been conducted, and information such as "concentration is not enhanced when a work task is started" is acquired as feeling information of the user, and the information is output to the storage unit. Then, intake information "XX mL of vinegar is taken every day" and "running" as behavior information which is a negative point, and "stretching" as a positive point are selected for the feeling information "concentration is enhanced" from information regarding analysis results through the questionnaire from the storage unit, advice that "a real feeling point of enhancing concentration will increase by XX if you run 5 km in the morning instead of 10 km morning running, perform stretching for 5 minutes, and drink 150 mL of vinegar drink" can be selected in the advice DB 12c or generated, acquired, output to the user terminal, and displayed on the user terminal. As described above, the user who uses the advice in the present embodiment can perform "drinking XX mL of vinegar every day" and "stretching" while continuing "running" which is previous behavior information without forcing himself/herself and can obtain the effect that "concentration is enhanced". On the other hand, the user who does not use the advice in the present embodiment cannot enhance concentration if he/she continues "running" or stops "running" and cannot enhance concentration as well. In this manner, although each causal relationship or the like is not clear, it is possible to obtain unexpected effects that feeling information is enhanced by successfully combining behaviors and diet that contribute to a plurality of feeling elements in opposite directions to enhance total real feeling score without forcibly recommending and encouraging continuation of specific behavior for each user.

Also, it is possible to evaluate regression analysis accuracy and to always employ an analysis result with high prediction accuracy by calculating accuracy from data acquired from the user who uses the advice other than the target on which this questionnaire has been conducted and performing logistic regression analysis again by using all pieces of data obtained until then in a case where the numerical value is equal to or less than a predetermined value. Particularly, it is possible to verify a model in a arbitrarily selected group with higher homogeneity in terms of statistical analysis model (a logistic regression analysis model, for example) and a learning model and to more finely evaluate regression analysis accuracy as compared with a case where a test sample is randomly selected, by calculating accuracy from user data in a case where the proposal has been followed and user data in a case where the proposal has not been followed and performing logistic regression analysis again by using all pieces of data obtained until then in a case where the numerical value is equal to or less than the predetermined value.

In other words, the present embodiment includes a program, an information processing method, or an information processing device adapted to acquire one or more pieces of information from among feeling information regarding a feeling of a user who has followed advice information and/or a user who has not followed the advice information after the advice information is output at an arbitrary timing, biological information, and behavior information and/or real feeling score information derived from such information, output, to an information processing unit, the acquired intake information, the one or more pieces of information from among the feeling information, the biological information, and the behavior information and/or the real feeling score information derived from such information and identification information of the user, and acquire, from the information processing unit, properness verification information for information regarding a correlation when the advice information is output.

Also, the present embodiment includes a program, an information processing method, or an information processing device adapted to newly perform information processing by using one or more pieces of information from among feeling information regarding a feeling of a user who has followed advice information or a user who has not followed the advice information, biological information, and behavior information and/or real feeling score information derived from such information, and further a program, an information processing method, or an information processing device adapted to newly acquire, from an information processing unit, advice information including information regarding a correlation between intake information and a real feeling score information.

In regard to the feeling information, the display unit 25 is caused to display a color that can be selected using a color chart, an illustration including a color, or the like, and the color selected by the user is converted into a numerical value by a data sequence in which each color stored in the storage unit 22 is expressed as a numerical value. Alternatively, in regard to the feeling information, a negative point, a positive point, and other numerical values of each of the user feeling information, the user biological information, and the user behavior information may be calculated by numerical values obtained by positions represented by three-axis coordinates of an x axis, a y axis and a z axis, or the positions represented by the three-axis coordinates may be represented as vectors. For example, coordinates for representing effects, such as a "reference", a "model", a "target", an "ideal", an "expectation", an "assumption", an "improvement", and the like may be set in regard to the feeling information, and the feeling information may be calculated as an approximation rate between the coordinates of the user at the current point and the coordinates of the "reference" or the like. Alternatively, in regard to the feeling information, a vector obtained from the set coordinates may be obtained, and the feeling information may be calculated as an approximation rate between the vector of the user at the current point and the vector of the "reference" or the like.

Correlations of the thus acquired intake histories of a plurality of users, one or more pieces of information from among feeling histories, biological histories, and behavior histories, and/or user real feeling score information derived from such information (the one or more pieces of information from among the feeling histories, the biological histories, and the behavior histories) (which may be referred to as "information regarding a correlation between user intake information and user real feeling score information" in the present embodiment) are obtained. In other words, the "information regarding the correlation between the intake information and the real feeling score information (which may be referred to as "information regarding the correlation between the intake information and the real feeling score information")" may be any information regarding the correlation between the intake information acquired from an assumed user who uses the advice (which may be simply referred to as a "user") in the present embodiment and the user real feeling information which is scheduled to be provided to the user and is not necessarily information based on data obtained from the user.

For example, in a case where information regarding a specific correlation that "real feeling score information is enhanced (by the number of users from which high values are obtained in regard to feeling information for a long period of time increasing)" is obtained when other users (user group) not including the user take a predetermined amount of insoluble dietary fiber from foods, the effect of the present embodiment is achieved by providing information regarding the correlation that "the group with high real feeling score information takes a predetermined amount of insoluble dietary fiber" or "feeling information is enhanced by taking a predetermined amount of insoluble dietary fiber or advice including the information to users for which effectiveness or relevance is presumed from the correlation, such as users with similar intake information (for example, users who take insoluble dietary fiber) or users for which an improvement in real feeling score information is required (for example, users who exhibited low feeling information for a long period of time). In other words, the "information regarding the correlation between the intake information and the real feeling score information" in the present embodiment may be information regarding a correlation based on data obtained from a user group including assumed users who will use the advice or may be information regarding a correlation based on data obtained from a user group that does not include the assumed users.

Also, the storage unit 22 may be configured to store user intake information, user feeling information, user biological information, and user behavior information input via the input unit 24, for example. Also, the communication unit 23 of the user terminal 20 has an interface for performing wireless communication with the wearable device 30 in addition to the interface for establishing connection to the network N. Note that the communication unit 23 may be configured to communicate with the wearable device 30 through wired communication via a cable.

The camera 27 has a lens, an imaging element, and the like, performs photoelectric conversion on light, which has been incident via the lens, by the imaging element, and acquires image data. The camera 27 performs imaging in accordance with an instruction from the control unit 21, successively sends the acquired image data (captured image) to the storage unit 22, and causes the storage unit 22 to store it. Note that the user terminal 20 may be configured to include a camera connection unit to which an external camera can be connected instead of having the configuration in which the camera 27 is incorporated therein and may be configured to include a camera communication unit that performs wireless communication with the external camera. In this case, the camera connection unit or the camera communication unit receives an input of image data acquired by the external camera, successively sends the input image data to the storage unit 22, and causes the storage unit 22 to store it.

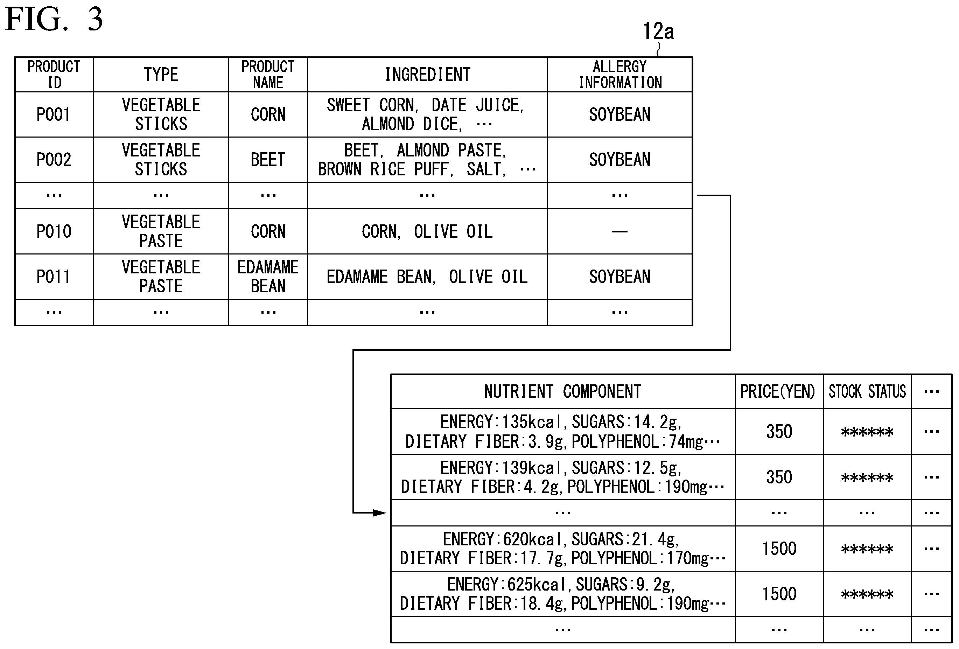

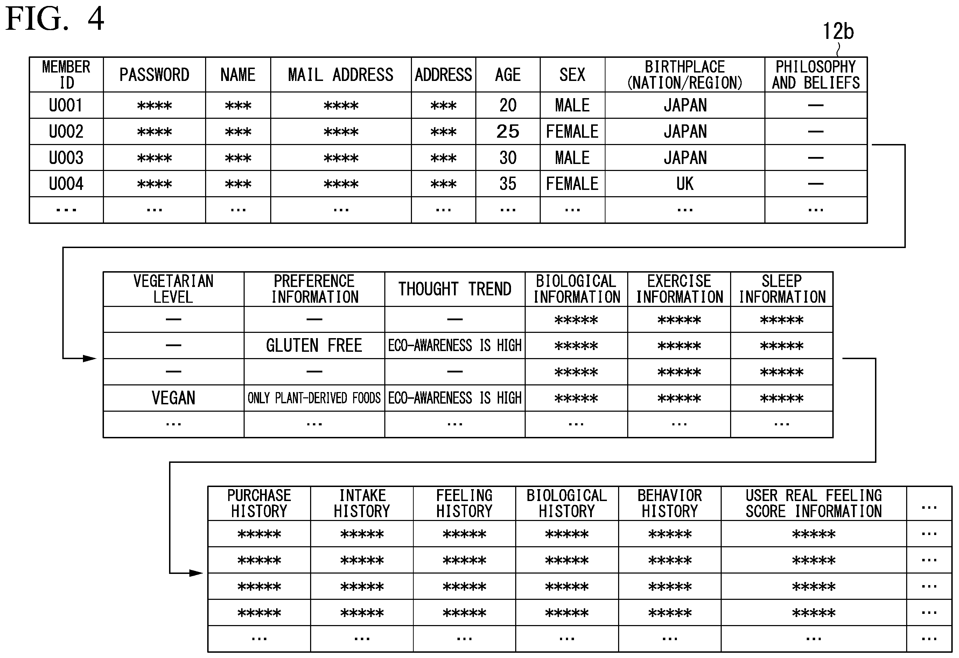

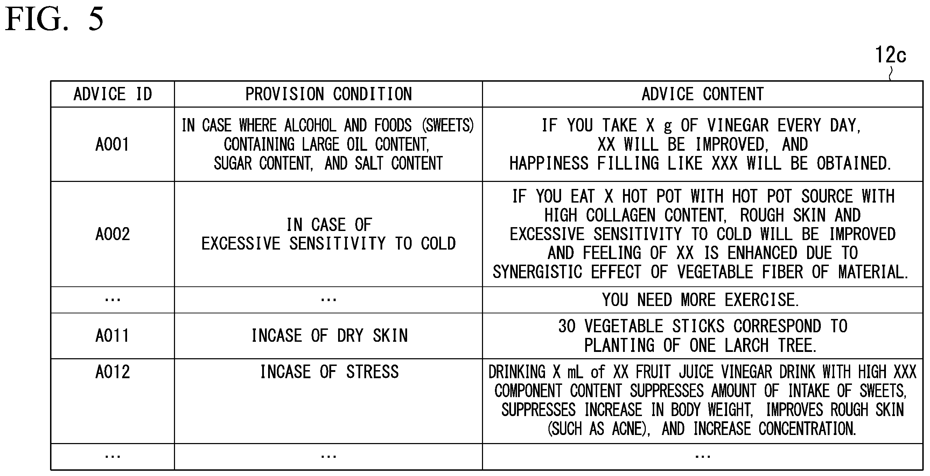

FIGS. 3 to 5 are schematic diagrams illustrating a configuration example of DBs 12a to 12c stored in the server 10. FIG. 3 illustrates a product information DB 12a, FIG. 4 illustrates a member information DB 12b, and FIG. 5 illustrates an advice DB 12c, respectively.

The product information DB 12a stores information regarding products. The product information DB 12a illustrated in FIG. 3 includes a product ID column, a type column, a product name column, an ingredient column, an allergy information column, a nutrient component column, a price column, a stock status, and the like and stores information regarding products in association with the product IDs. The product ID column stores identification information (product ID) allocated to the respective foods that are sales targets. The type column stores types by which the products are categorized by forms, ingredients, manufacturing methods, and the like of the foods and stores, for example, vegetable sticks, vegetable paste, noodles, fruit sticks, fruit paste, and the like. The product name column stores names applied to the products, the ingredient column stores names of materials contained in the products, and the allergy information column stores names of allergen materials (materials that may cause allergy) contained in the products. The nutrient component column stores energy and component names of nutrient components obtained by taking the products and amounts of intake in an associated manner.

Note that as the amounts of intake of energy and nutrient components, the amounts of intake with respect to a predetermined amount of products may be used and, for example, the amount of intake per stick food or the amount of intake per 100 g of paste-like food may be used. The price column stores prices of the products, and the stock status column stores the numbers of stocks of the products.

The product IDs stored in the product information DB 12a are issued by the control unit 11 and are then stored when information regarding new products that are sales targets is registered. Information regarding the types, the product names, the ingredients, the allergy information, the nutrient components, and the prices stored in the product information DB 12a is stored by the control unit 11 in a case where the control unit 11 acquires information of the new products that are sales targets via the communication unit 13 or the input unit 14, for example.

In regard to the stock status stored in the product information DB 12a, the number of manufactured products is added by the control unit 11 in a case where the control unit 11 receives the number of manufactured products via the communication unit 13 or the input unit 14, for example, and the number of sales is subtracted therefrom by the control unit 11 in a case where the control unit 11 receives the number of sold products via the communication unit 13 or the input unit 14, for example. The details stored in the product information DB 12a are not limited to those in the example illustrated in FIG. 3, and various kinds of information regarding the foods that are sales targets can be stored. For example, information regarding preservation methods, best- before dates, manufacturing companies, and manufacturing processes of the food may be stored in the product information DB 12a, for example.

The member information DB 12b stores information regarding users who have been registered as members in order to receive various kinds of advice from the server 10. The member information DB 12b illustrated in FIG. 4 includes a member ID column, a password column, a name column, a mail address column, an address column, an age column, a sex column, a birthplace column, a philosophy and beliefs column, a vegetarian level column, a preference information column, a thought trend column, a biological information column, an exercise information column, a sleep information column, a purchase history column, an intake history column, a feeling history column, a biological history column, a behavior history column, and the like and stores information regarding users who are members in association with the member IDs.

The member ID column stores identification information (member ID) allocated to each of the users who have been registered as members, and the password column stores passwords that the user has set at the time of registering as members or after registering as members. The name column, the mail address column, the address column, the age column, the sex column, the birthplace column, and the philosophy and belief column store names, mail addresses, addresses, ages, sexes, birthplaces (nations or regions where the users were born, home countries), and philosophy and beliefs (religions, for example) which are attribute information that the users have input (designated) at the time of registering as members or after registering as members, respectively.

Note that birth dates may be stored instead of ages, sexes that user recognizes may be stored as sexes, nations (or regions or home country or power distance index (PDI) values thereof) where the user was born, nations (or regions or nationalities or PDI values thereof) where the user was brought up, and the like can be used as birthplaces, and religions and the like in which the users have belief can be used as philosophy and beliefs.

The vegetarian level column, the preference information column, the thought trend column, the biological information column, the exercise information column, and the sleep information column store vegetarian levels, preference information, thought trends, feeling information, biological information, and behavior information (exercise information, sleep information) that the users have designated at the time of registering as members or after registering as members, respectively. The vegetarian levels are information indicating vegetarian levels of the users and include, for example, vegans indicating total vegetarians who do not eat any animal-derived foods, lacto-vegetarians who eat dairy products, ovo-vegetarians who eat eggs, and the like. The preference information is information indicating trends of taste (likes and dislikes) of the users related to foods, materials, and the like and include a type (gluten-free) of users not taking gluten, a type of users not taking animal-derived foods (only plant-derived foods), a type of users not taking additives, and the like. The thought trends are information indicating trends of how to think in user's everyday lives or daily habits. Examples include a type (high eco-awareness) of users who are aware of reduction of waste such as leftovers, a type of users who desire to purchase products with high quality even if they are expensive, a type of users who place priority on prices, and the like.

The feeling information includes information specifying feelings when users eat products. Examples of the information specifying feelings may be users' impressions (including free text answers and schemes of selecting feelings by check boxes, radio buttons, icons, and the like), may be information on the basis of which it is possible to directly estimate feelings such as users' facial expressions, or may be information on the basis of which it is possible to indirectly estimate feelings such as texts, utterances, and voice tones.

Also, it is possible to exemplify, as specific types of the feeling information, apparent feelings such as "satisfied", "worried", "happy", and "sense of accomplishment", desire such as "appetite" and "a desire to purchase a product", and subconscious mind such as "a desire to lose weight", "a desire to look young", and "a desire to recommend something to others" in addition to joy, anger, sadness, and pleasure. Furthermore, intermediate-to-long-term feeling information may be calculated by recording such feeling information with time in the feeling history column.

The biological information includes various kinds of information regarding user's body conditions, may include, for example, a height, a weight, a body fat percentage, a body temperature, a blood pressure, a heart rate, a pulse rate, sweating, brain waves, emotional hormones, stress hormones, and the like, and may include, as information regarding body conditions (which may simply referred to as "body information"), types and amounts of medicines that are currently being taken, pre-existing conditions (disease history), frequencies and amounts of smoking and drinking, test results of various tests including a blood test and a urine test conducted in medical facilities, test results of various tests obtained by health check or complete medical checkup, and the like, a life expectancy (particularly, healthy life expectancy), the number of teeth, joint pain, a disease history, results of complete medical checkup, a hospitalization history, a period of bedridden period, whether or not the user can independently walk, whether or not the user can carry out everyday behaviors (meals, changing clothes, excretion, taking bath, and the like) without any assistance, and the like as information regarding the user's living body.

Furthermore, each piece of information that is measured with time in relation with the feeling information may be categorized into positive and negative biological information, each of them may be weighted for each time and each item, and information with a comprehensively high correlation with the intermediate-to-long-term feeling information over a specific period of time may be used to derive user real feeling score information as information based on the fact that it is possible to potentially estimate feelings from unconsciously expressed phenomena. For example, it is preferable to use brain waves, emotional hormones, stress hormone, information regarding the body, and the like with high correlations with the intermediate-to-long-term feeling (real feeling) over a specific period of time.

The exercise information is information regarding exercise that the user has conducted and includes types (content) of exercise and an exercise time, for example. The sleep information is information regarding user's sleep and includes information such as a sleep time, a clock time at which the user goes to bed (sleep start clock time), a clock time at which the user gets up (sleep end clock time), and a pulse during sleeping, for example.

The behavior information may include, in addition to information regarding an amount and a frequency of exercise and a time and quality of sleep, information regarding user behavior including an amount and a frequency of smoking, an amount and a frequency of night drinking, a marital status, and a frequency of fighting between a married couple and how hard it is, a frequency of masturbation and sexual activities, presence/absence of menopausal symptoms or dementia for a senior user, a timing and a frequency of menstruation and ovulation for a female user, whether or not the user is pregnant and months of pregnancy, and whether or not the user has experienced menopause. Furthermore, each piece of information measured with time in relation with the feeling information is categorized into positive and negative behavior information, each of them is weighted for each time and each item, and information with a comprehensively high correlation with the intermediate-to-long-term feeling information over a specific period of time may be used to derive user real feeling score information as information based on the fact that it is possible to potentially estimate feelings from unconsciously expressed phenomena. For example, it is preferable to use the amount and the frequency of exercise, the time and the quality of sleep, the amount and the frequency of smoking and the amount and the frequency of night drinking (with high negative correlations), and the like with high correlations with the intermediate-to-long-term feeling (real feeling) over a specific period of time.

The purchase history column stores purchase information in which information regarding products that the user has purchased and purchase dates are associated. The intake history column stores intake information in which information regarding products that the user has eaten from among products that the user has purchased and the dates and times (intake dates and times) when the user has eaten the products are associated.

The biological history column stores information specifying a living body when the user has eaten products. The behavior history column stores information specifying behaviors when the user has eaten products.

Note that the intake information may include information regarding energy and nutrient components that the user has taken by eating products, instead of information regarding products that the user has eaten. Examples thereof include nutrient component information stored in the product information DB 12a, information obtained from websites or the like that companies that sell products provide, information based on "Japanese Food Ingredients Chart 2015 (seventh revision)", and information based on "Dietary Reference Intakes for Japanese (2015)".

The member ID stored in the member information DB 12b is issued by the control unit 11 and is stored when information of a user who is newly registered as a member is registered. Other information stored in the member information DB 12b is stored in the control unit 11 in a case where the control unit 11 acquires each piece of information of the user via the communication unit 13 or the input unit 14, for example, and is changed by the control unit 11 in a case where a change instruction is acquired via the communication unit 13 or the input unit 14. Note that each of attribute information, biological information, exercise information, sleep information, a purchase history, an intake history, a feeling history (feeling information), a biological history, a behavior history, and user real feeling score information stored in the member information DB 12b is accumulated (additionally stored) in the member information DB 12b by the control unit 11 every time the control unit 11 acquires each piece of information from the user terminal 20 via the communication unit 13.

The content stored in the member information DB 12b is not limited to those in the example illustrated in FIG. 4, and various kinds of information regarding users who have registered as members may be stored for each of the users. For example, a family structure of the user, user's residence (the name of the nation or the PDI value), a user's occupation, information regarding foods or materials by which the user causes allergy reactions, hobbies, targets in everyday life, and the like may be stored in the member information DB 12b. Also, the biological information, the exercise information, the sleep information, the intake history, the feeling history (feeling information), the biological history, the behavior history, the user real feeling score information, and the like may be stored in a predetermined region in the storage unit 12 or another storage device in addition to the storage in the member information DB 12b.

Also, for the user real feeling score information (or the user feeling information, the user biological information, and the user behavior information used to derive the user real feeling score information), data on which information processing has been done as member information may be registered, or data including the user feeling information, the user biological information, and the user behavior information used to derive the user real feeling score information may be registered and calculated as needed. In this case, each of the biological information column, the exercise information column, the sleep information column, the intake history column, the feeling history column, the biological history column, and the behavior history column stores information (for example, a file name indicating a data storage location) to read each piece of information. The member information DB 12b is not limited to the configuration as one DB and may be configured such that each information is stored in a split manner in a plurality of DBs.

The advice DB 12c stores information regarding advice to be provided to the user (user terminal 20). The advice DB 12c illustrated in FIG. 5 includes an advice ID column, a provision condition column, an advice content column, and the like and stores advice provision conditions and advice content in association with the advice ID. The advice ID column stores identification information (advice ID) allocated to each advice. The provision condition column stores provision conditions under which advice is to be provided and stores a condition regarding information related to a correlation between intake information and the real feeling score information, a condition regarding a nutrient component, a condition regarding a feeling, a condition regarding a living body, a condition regarding a behavior, a condition regarding a timing (a season, a time zone, and the like) at which the advice is to be provided, for example. In this manner, it is possible to register different advice content in the advice DB 12c in accordance with the user's eating habits and user's real feeling and in accordance with the advice providing timing. Also, the information regarding the correlation between the intake information and the real feeling score information stored in the advice DB 12c may be information regarding a correlation based on knowledge of professionals, may be information regarding a correlation obtained by a learning model based on current or past data of an assumed user who will use the advice, may be information regarding a correlation obtained by a learning model based on current or past data obtained from a user group arbitrarily including the assumed user, or may be information regarding a correlation obtained by a model obtained by combining them. Particularly, it is preferable to generate advice information by using "information regarding a correlation between intake information and real feeling score information" obtained by the learning model based on data obtained from the user group (which is preferably a group with the same attribute as that of the user, in particular) that does not include the assumed user in the past because it is possible to quickly provide advice to the user by using data on which analysis regarding the correlation has already been done. Also, it is preferable to generate advice information by using "information regarding a correlation between intake information and real feeling score information" obtained by the learning model based on current or past data obtained from a user group including the assumed data because the advice exhibited high self-fulfilling effect.

For example, it is possible to output advice ("it is believed that intake of vinegar makes body movement better", "it is believed that vinegar poured into ramen noodles make body movement better", "it is believed that vinegar contained in sweet and sour pork makes body movement better") including information regarding a correlation between intake information and real feeling score information for a user (for example, "who takes a small/large amount of vinegar", "who prefers ramen noodles that is a cooked food for which vinegar can be used", or "who has taken sweet and sour pork which is a cooked food with high relevance with vinegar") who is determined to have a high relevance on the side of intake information on the basis of the correlation between arbitrary intake information (intake of "vinegar" which is a food, for example) and the real feeling score information (for example, feeling information that "body movement is good" has been collected from many of a certain group and the real feeling score is high), and it is possible to output advice ("it is believed that intake of vinegar makes body movement better") including the information regarding the correlation between the intake information and the real feeling score information for the user ("who has had poor body movement recently", for example) who is determined to have a high relevance on the side of the real feeling score information.

Furthermore, it is possible to provide a more effective advice by using member information, attribute information, and the like when the provision conditions are determined. For example, since a user in a nation with a relatively low PDI value places priority on review information, it is possible to cause the self-fulfilling effect of the present invention to be more strongly exhibited by outputting, to such a user, advice including the information regarding a correlation between intake information and real feeling score information such as "intake of vinegar makes body motion better in XXX (nation)". Also, a user in a relatively high PDI value places priority on information from professionals, it is possible to cause the self-fulfilling effect of the present invention to be more strongly exhibited by outputting advice including a correlation between intake information and real feeling score information from professionals such as "according to professionals, intake of vinegar makes body movement better" to such a user.

The "advice information including the correlation between the intake information and the real feeling score information" in the present embodiment represents information itself regarding a correlation between specified user intake information and the "real feeling score" obtained by specifying user intake information with a positive or negative correlation with the "real feeling score", or advice information using the correlation. In other words, it is possible to provide, to the user, information regarding a food intake mode that gives an intermediate-to-long-term real feeling over a specific period of time rather than giving a transitory pleasure to the user by specifying the intake information (intake modes such as the type, the amount, the intake timing, the season, the eating combination, the cooking method, the contained components, and the like of the food) with positive or negative correlation with an intermediate-to-long-term feeling (real feeling) over a specific period of time and providing the information itself regarding the correlation between the specified intake information and the "real feeling score" or advice information using the correlation.

Here, the "information regarding the correlation between the intake information and the real feeling score information" may be information regarding a correlation based on knowledge of professionals, may be information regarding a correlation based on data obtained from a user group including an assumed user who will use the advice, or may be information regarding a correlation based on data obtained from a user group that does not include the assumed user. In other words, in a case where high (or low) real feeling score information is exhibited in a user group arbitrarily including the user, it is possible to obtain the effect of the present embodiment by specifying one or more pieces of intake information (the intake modes such as the type, the amount, the intake timing, the season, the eating combination, the cooking method, the contained components, and the like) as factors of the real feeling score information and providing information regarding the correlation to the user from which effectiveness or relevance is presumed from the correlation, such as a user with the similar intake information, a user for which an improvement in real feeling score information is required, or the like.

It is possible to expect the effect of enhancing the user real feeling score from the user who has taken the food with the information as compared with a case where the food is simply taken. This is because information such as "a real feeling score is high in a certain region" or "a real feeling score is high in a certain group" itself is valuable for the user, and the effect of enhancing the real feeling score is achieved in a self-fulfilling manner due to a so-called placebo effect or a user's awareness of that "his/her behavior is good for health" even in a case where the same food is taken in the same intake form.

In other words, the present embodiment can enhance the effect of the food itself by provided information by specifying food intake information that enhances the user real feeling score in a specific region and with a specific means and providing not only the food but also information that there is a correlation with the real feeling score as well, and utilization for realizing food intake to enhance user's quality of life is expected. Here, it has not been possible to specify food intake modes that lead to user's real feelings, and information that enhances user's real feelings for foods in a synergistic manner has not been provided at all even though it is possible to verify foods (or effective components in the foods) that affect living bodies in a test system that verifies effects in a comparative test manner in a short period of time as in a conventional clinical test.

Also, it is possible to expect an effect of enhancing a value of a food and increasing a real feeling score by information that "the real feeling score is high in a certain region" or "a real feeling score is high in a certain group" being information in a group with the same attribute as that of the user himself/herself. For example, information is preferably based on data obtained in a nation with a relatively similar PDI value, which will be described later, or in the same nation as the to which the user who receives the information belong, and more specifically, the information is preferably based on data obtained in a nation with a PDI value within ±20. This is because such information in the group with the same attribute is likely to cause a so-called placebo effect or a user's awareness that "the user's behavior is good for health" and the effect of enhancing the real feeling score in a self-fulfilling manner in the present invention is further exhibited. It is preferable to provide such information in the group with the same attribute in a nation with a relatively low PDI value in which there is a trend that review information is highly rated.

Also, in a case where nations are categorized into nations with relatively low PDI values (equal to or greater than 0 and less than 30), which will be described later, nations with middle PDI values (equal to or greater than 30 and less than 60), nations with relatively high PDI values (equal to or greater than 60 and less than 90), and nations with high PDI values (equal to or greater than 90 and less than 120), product intake information that enhances user real feeling score is preferably information obtained in the nation belonging to the same category as the nation to which the user who receives the information belongs, and is particularly preferably information in the same nation. The information in the group with the same attribute is likely to cause a so-called placebo effect and a user's awareness that "the user's behavior is good for health", the effect of enhancing the real feeling score in a self-fulfilling manner in the present invention is further exhibited, and it is thus possible to obtain a higher effect.

Also, although the food intake information is preferably information obtained in a nation with a relatively low PDI value (equal to or greater than 0 and less than 30) or a nation with a middle PDI value (equal to or greater than 30 and less than 60), the food intake information that enhances the user real feeling score is preferably information obtained in the nation with the middle PDI value (equal to or greater than 30 and less than 60) in terms of how easily the data can be obtained. Furthermore, it is preferrable to provide such information to a user in a nation with a relatively low PDI value (equal to or greater than 3 and less than 30) or a nation with a middle PDI value (equal to or greater than 30 or less than 60) because the effect of enhancing the real feeling score in a self-fulfilling manner is more strongly exhibited due to a so-called placebo effect or a user's awareness that the user's behavior is good for health".

Further surprisingly, there is an effect of further enhancing the above real feeling score in a case where a food as a target of intake information is a general food or a cooked menu thereof as compared with a case where the food as a target of the intake information is a highly functional food with an increased effective component. Although the principle is not clear, this is considered to be because a value of information that "the real feeling score is high in a certain region" or "the real feeling score is high in a certain group" is highly rated for a general food or a cooked menu, an action mechanism of which cannot be easily understood by the user who accesses the information, and the self-fulfilling effect is strongly expressed.

Furthermore, the user in the present embodiment is preferably a user in a nation with a small power difference. The "power difference" in the present embodiment is in a level in which people in the nation accept unfairness of power, and the proportion increases as a level in which the people accept the unfairness of power increases. Also, this is considered to be because while users in nations with large power differences tend to place importance in reliability of company professionals, users in nations with small power differences (Japan, the U.S., U.K., Germany, and the like) tend to place importance on sense of intimacy with other users, and thus a value of information that "the real feeling score is high in a certain region" or "the real feeling score is high in a certain group" derived from the users with the same attribute as that of the users themselves is thus rated high, and the self-fulfilling effect is strongly expressed.

The "nations with small power differences" in the present embodiment may be defined as nations in which numerical values of power distance indexes (which may be simply referred to as PDI or a PDI value) in "Hofstede index" obtained by Geert Hofstede quantitatively measuring cultures (national mentalities) of various nations and representing the cultures and the national mentalities of the nations by numerical values are equal to or less than specific values (for example, see data in "6-dimensions-for-website-2015-08-16.xls" in https://geerthofstede.com/research-and-vsm/dimension-data-matrix/ or https://web.archive. org/web/20180222070021/http: geerthofstede.com/research-and-vsm/dimension-data-matrix/). Specifically, the PDI values in the "nations with small power differences" is preferably equal to or less than 80, is further preferably equal to or less than 75, is particularly preferably equal to or less than 70, or equal to or less than 65. Also, it may be defined by the PDI values being equal to or less than an arithmetic average value (78 in the above file, or 59 as a national average value) in all the nations from which data has been able to be obtained. Although the lower limit is not particularly limited and is equal to or greater than 0. Specifically, nations with small power differences include Japan (54), the U.S. (40), Germany and UK (35), Finland (33), Norway and Sweden (31), Switzerland (in German speaking area) (26), New Zealand (22), Austria (11), and the like.

On the other hand, it is preferable to feed back the information along with professionals' comments to users in nations with large power differences because the effect of the present embodiment is enhanced. The "nations with large power differences" in the present embodiment represent nations other than the aforementioned "nations with small power differences", and specifically, the nations with large power differences include Malaysia, Slovakia (104), Guatemala and Panama (95), the Filippines (94), Russia (93), and the like.

The advice content column stores an advice message to be provided to the user in regard to the daily habits such as meals, a message to be provided as a notification to the user in regard to contribution to the global environment through eating of a food, a message including virtual user intake information that is intake information for increasing the user real feeling score per unit time, a message including virtual user intake information that is intake information that increases the user real feeling score per unit time while including a food having a relatively low user real feeling score but highly required in terms of user preference and/or daily habits, a message including virtual user intake information that is intake information that increases the user real feeling score per unit time without any change in behavior information, a message that includes virtual user behavior information that is behavior information for increasing the user real feeling score per unit time, and the like.

As advice regarding a meal, advice proposing intake amounts of various nutrient components such as dietary fiber and protein, for example, effects of intake, and menus to obtain a certain effect. As advice regarding exercise, advice proposing the type of exercise and the exercise time, a message that promotes the user to do exercise, and the like are included.

Also, as a message regarding contribution to the global environment, a message regarding the global environment that can be protected in accordance with intake amounts of foods (the amounts by which the foods have been eaten) is included, and for example, a message that provides a notification of the amount of reduction of the amount of discharged CO2 that can be reduced in accordance with intake amounts of foods is included. Note that the advice message includes various kinds of advice regarding daily habits including meals, exercise, and the like from professionals such as doctors, nurses, pharmacists, nutritionist, sport trainers, researchers, and the like. The advice ID stored in the advice DB 12c is issued and stored by the control unit 11 when a new advice is registered. Other information stored in the advice DB 12c is stored by the control unit 11 in a case where the control unit 11 acquires various kinds of information regarding a new advice via the communication unit 13 or the input unit 14, for example. Content stored in the advice DB 12c is not limited to the example illustrated in FIG. 5, advice messages with various kinds of content may be stored therein, and various kinds of information regarding advice can be stored therein.

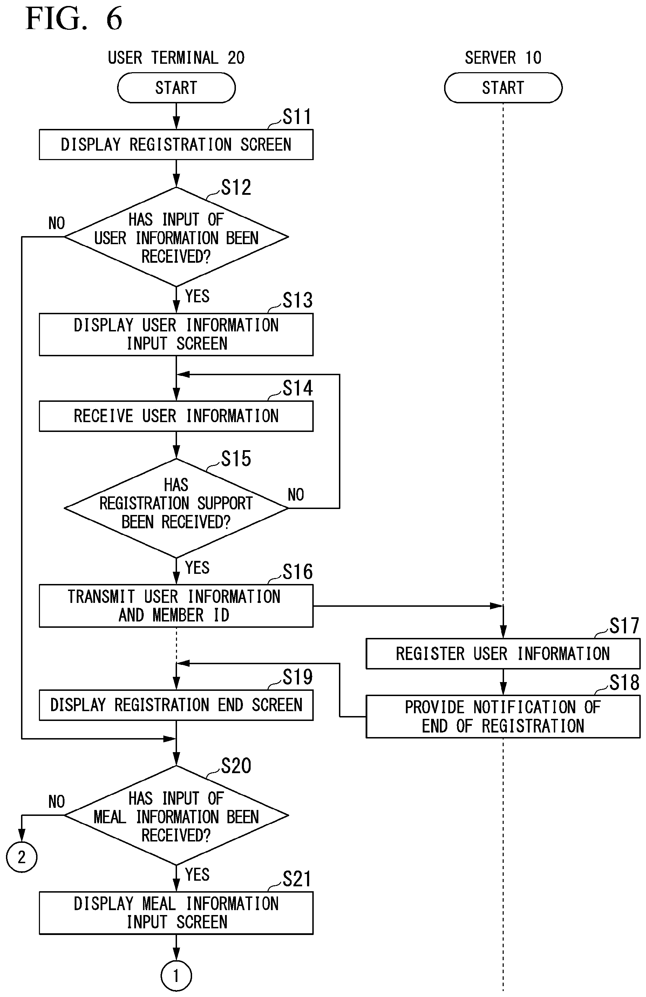

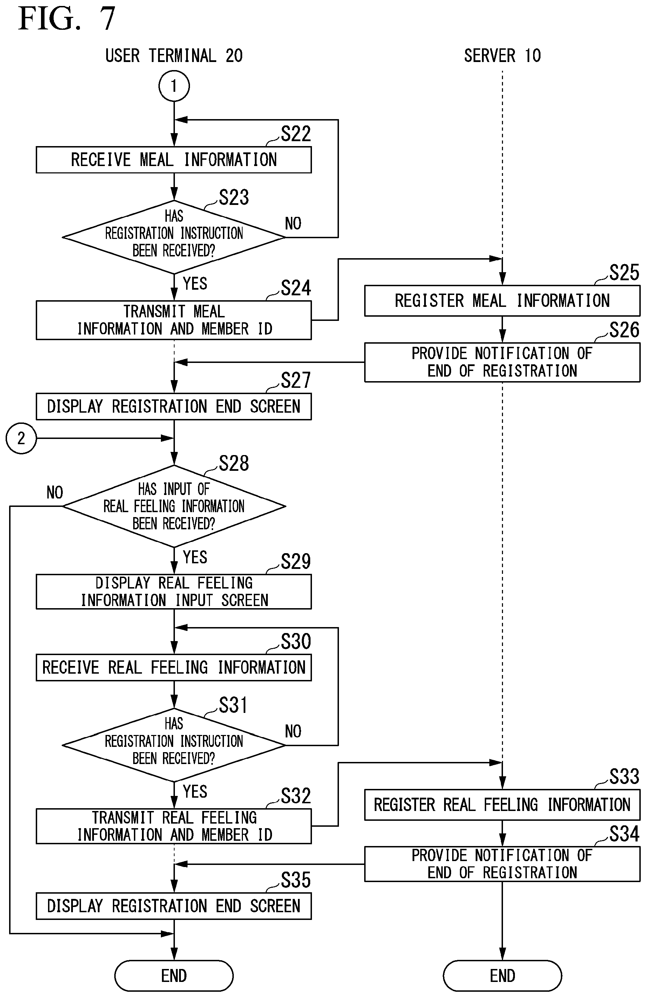

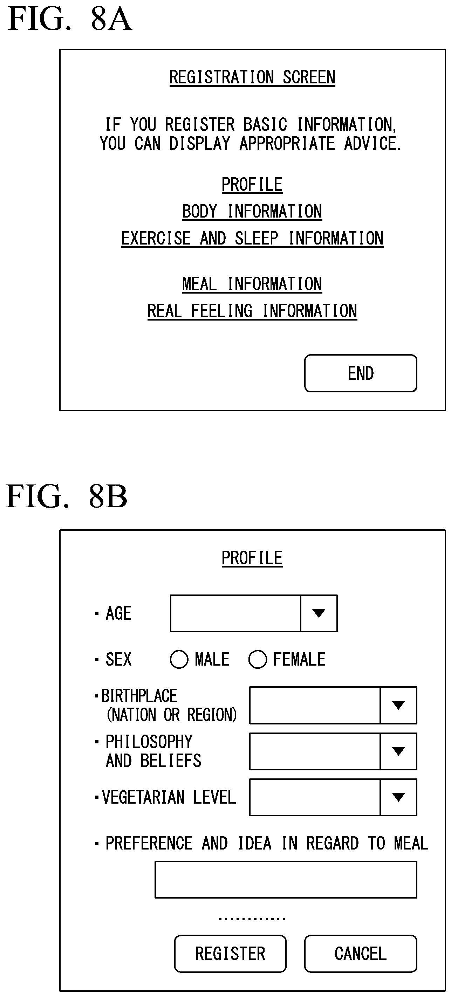

Hereinafter, processing performed by each device when the user registers information (user information) regarding the user in the server 10 by using the user terminal 20 in the information processing system 100 according to the present embodiment will be described. FIGS. 6 and 7 are flowcharts illustrating an example of user information registration processing procedure, FIGS. 8A to 10B are schematic views illustrating screen examples of the user terminal 20. In FIGS. 6 and 7, processing performed by the user terminal 20 is illustrated on the left side, and processing performed by the server 10 is illustrated on the right side, respectively. The following processing is executed by the control unit 21 in accordance with the control program 22P and the advice APP 22AP stored in the storage unit 22 of the user terminal 20 and is executed by the control unit 11 in accordance with the control program 12P stored in the storage unit 12 of the server 10. Apart of the following processing may be realized by a dedicated hardware circuit. In the following processing, it is assumed that the user is a user who has been registered as a member to acquire advice from the server 10 and has performed processing of logging in the server 10 by using a member ID and a password of the user himself/herself. Note that in a case where the user has been registered as a member, the control unit 11 (registration unit) of the server 10 acquires individual information required for member registration such as a name, a password, a mail address, an address, and the like from the user terminal 20 and register each piece of acquired information in the member information DB 12b. At that time, the control unit 11 issues a member ID and stores each piece of information in association with the member ID.

In the information processing system 100 according to the present embodiment, the user inputs user information and transmits it to the server 10 by using the user terminal 20 and causes the server 10 to register it in a case where the user desires to register or change information (user information) regarding himself/herself. Note that the user causes the user terminal 20 to display a user information input screen (registration screen) by causing the user terminal 20 to activate the advice APP 22AP and inputs the user information via the input screen. Also, in a case where the user terminal 20 stores a web browser for viewing a website via the network N in the storage unit 22, the user may access the server 10 by causing the user terminal 20 to activate the browser and acquire the user information input screen (registration screen) from the server 10.

The control unit 21 of the user terminal 20 causes the advice APP 22AP to be activated in a case where an instruction to activate the advice APP 22AP is received from the user via the input unit 24. Thereafter, in a case where an instruction to register or change the user information is received via the input unit 24, the control unit 21 displays a registration screen as illustrated in FIG. 8A on the display unit 25 (S11). FIG. 8A illustrates an initial screen example of the input screen (registration screen) for inputting the user information, and the screen illustrated in FIG. 8A is a selection screen for receiving a selection of a type of user information to be input (registered). The screen illustrated in FIG. 8A is configured to be able to select any of a user's profile, body information, exercise/sleep information, meal information, and real feeling information as user information to be input (registered). In a case where any of the profile, the body information, the exercise/sleep information, and the meal information has been selected via the input unit 24 on the screen illustrated in FIG. 8A, the control unit 21 receives an instruction to input the selected user information.

First, the control unit 21 determines whether or not an instruction to input user information regarding any of the profile, the body information, and the exercise/sleep information has been received on the screen illustrated in FIG. 8A (S12). In a case where it is determined that the instruction to input any of the user information has been received (S12: YES), the control unit 21 displays the input screen for the selected user information on the display unit 25 (S13). Note that in a case where the profile has been selected on the screen illustrated in FIG. 8A, the control unit 21 displays a profile input screen as illustrated in FIG. 8B. The input screen illustrated in FIG. 8B includes an input section for inputting each item of profile information including attribute information including an age and a sex of the user, a birthplace of the user, philosophy and beliefs, a vegetarian level, a taste (preference) for meals (foods and materials), principles and ideas (thoughts), and the like.

A pull-down menu from which it is possible to select arbitrary one of a predetermined age or a predetermined age zone is provided in the input section for an age, and it is possible to input an arbitrary age or age zone by using the pull-down menu. A radio button with which it is possible to select male or female is provided in the input section for a sex, and it is possible to input a sex by using the radio button.

A pull-down menu from which it is possible to select any one out of predetermined birthplaces (nations or regions) is provided in an input section for a birthplace, and it is possible to input an arbitrary birthplace by using the pull-down menu. A pull-down menu from which it is possible to any one out of predetermined philosophy and beliefs (religions, for example) is provided in an input section for philosophy and beliefs, and it is possible to input arbitrary philosophy and beliefs by using the pull-down menu.

A pull-down menu from which it is possible to select arbitrary one from predetermined information (vegan, lacto-vegetarian, or the like) indicating a vegetarian level is provided in the input section for a vegetarian level, and it is possible to input an arbitrary vegetarian level by using the pull-down menu. An arbitrary comment can be input to an input section for a taste and an idea in regard to meals via the input unit 24. Note that the control unit 21 may perform processing of extracting the user's taste and idea in regard to meals from a viewing history of a website (a recipe site for cooking, for example) that the user has viewed by using the user terminal 20 via the network N, for example. In this case, information that the control unit 21 has extracted from the viewing history is input to the input section for a taste and an idea in regard to meals. The profile input screen is not limited to the configuration illustrated in FIG. 8B, and each input section may be configured such that arbitrary information can be input via the input unit 24, and a pull-down menu may be provided such that arbitrary one can be selected from among predetermined options.

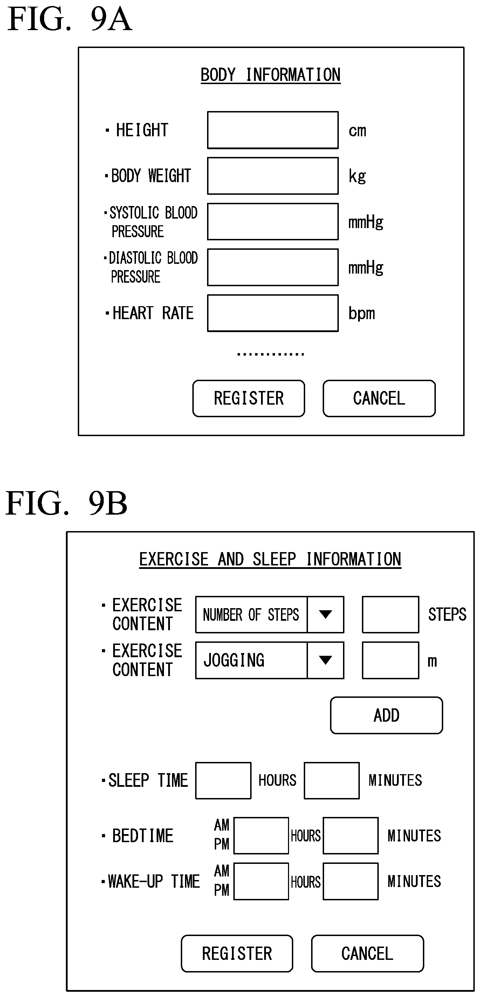

Also, in a case where body information is selected on the screen illustrated in FIG. 8A, the control unit 21 displays a body information input screen as illustrated in FIG. 9A. The input screen illustrated in FIG. 9A includes an input section for inputting each piece of biological information (information regarding a body condition) such as a height, a weight, a blood pressure (a systolic blood pressure and a diastolic blood pressure), a heart rate, a body fat rate, a pulse rate, a body temperature, a defecation frequency, a blood glucose level, sweating, brain waves, emotional hormones, stress hormones, and the like of the user. Although it is possible to input an arbitrary numerical value to each input section for biological information via the input unit 24, a pull-down menu from which arbitrary one can be selected from a predetermined options may be provided in each input section. Also, the user terminal 20 can acquire measurement values of biological information that can be measured by the wearable device 30 from the wearable device 30, and measurement data of the biological information acquired from the wearable device 30 is input to each input section for biological information on the input screen illustrated in FIG. 9A.

Specifically, measurement values such as a body temperature, a blood pressure, a heart rate, a pulse rate, measurement values of an intraoral sensor or the like that can be measured by the wearable device 30 are input to and displayed in the respective input sections. Note that the user terminal 20 may be configured to acquire measurement values obtained by measurement devices other than the wearable device 30, and in this case, the measurement values acquired from the measurement devices are displayed in the corresponding input sections. Note that as the measurement devices, it is possible to use a height meter to measure a height, a weight scale to measure a weight, visceral fat, and a body fat percentage, a pulse meter to measure a pulse, a blood glucose meter to measure a blood glucose value, a skin sensor, and the like.

The body information input screen is not limited to the configuration illustrated in FIG. 9A and may include input sections for types and amounts of medicines that are currently being taken, pre-existing conditions (disease history), frequencies and amounts of smoking and drinking, test results of various tests including a blood test and a urine test conducted in medical facilities, test results of various tests obtained by health check or complete medical checkup, and the like. Note that a configuration in which test result data is acquired by the camera 27 imaging a paper on which the test results are described may be employed.

In this case, the user terminal 20 reads the test results by imaging the paper on which the test results are described by the camera 27 and generating text data through optical character recognition (OCR) from the obtained captured image. Then, the user terminal 20 can acquire biological information of the user by extracting various kinds of data from the read test results. Note that a configuration in which the processing of generating text data through OCR from the captured image obtained by imaging the paper for the test results is performed by a device (the server 10, for example) other than the user terminal 20 may be employed.

Furthermore, in a case where exercise/sleep information has been selected on the screen illustrated in FIG. 8A, the control unit 21 displays an exercise/sleep information input screen as illustrated in FIG. 9B. The input screen illustrated in FIG. 9B includes an input section for inputting exercise content (exercise information) including the type of exercise and the amount of exercise or an exercise time and an input section for inputting sleep information including a sleep time, a clock time at which the user goes to bed, and a clock time at which the user gets up. A pull-down menu from which arbitrary one can be selected from predetermined types is provided in the input section for the type of exercise, and it is possible to input an arbitrary type of exercise by using the pull-down menu.

It is possible to input an arbitrary numerical value to the exercise amount or exercise time input section via the input unit 24. Also, the user terminal 20 can acquire measurement values regarding the exercise information that can be measured by the wearable device 30 from the wearable device 30, and the measurement values regarding the exercise information acquired from the wearable device 30 is input to the respective input sections on the input screen illustrated in FIG. 9B.

Also, an add button for adding an input section for exercise information is provided on the screen illustrated in FIG. 9B, and in a case where the add button is operated, an input section for new exercise content is displayed. Although it is possible to input arbitrary numerical value to each input section for sleep information via the input unit 24, a pull-down menu from which it is possible to select arbitrary one from predetermined numerical values may be provided. Also, measurement values regarding sleep information that the user terminal 20 has acquired from the wearable device 30 may be input to each input section for sleep information. The exercise/sleep information input screen is not limited to the configuration illustrated in FIG. 9B, and an input section in which an arbitrary comment can be input via the input unit 24 as exercise information and sleep information, for example, may be provided. Also, the exercise/sleep information input screen may be configured such that information (behavior information) regarding various behaviors in everyday life of the user is input in addition to the information regarding exercise and sleep.

The user inputs each piece of information that can be input to each input section on the input screen as illustrated in FIGS. 8B to 9B. The control unit 21 of the user terminal 20 receives each piece of information (user information) input by the user via the input unit 24 (S14) and displays each piece of received information in each corresponding input section. Note that the user terminal 20 may store previously input information in the storage unit 22. In this case, the control unit 21 displays information that has already been input in each corresponding input section when the input screen as illustrated in FIGS. 8B to 9B is displayed on the display unit 25. Each piece of information displayed in the input section can be modified via the input unit 24 on the input screen on which the information that has already been input is displayed, and the user can not only input information in each input section but also appropriately modify the information that has already been input. Also, in regard to information that can be acquired from measurement devices on the input screen as illustrated in FIGS. 8B to 9B, the control unit 21 acquires each piece of information from the measurement devices regularly or when the display unit 25 is caused to display the input screen and displays each piece of acquired information in each corresponding input section.

The input screen illustrated in FIGS. 8B to 9B includes a registration button for providing an instruction to execute processing of registering each piece of input information (user information) in the server 10 and a cancel button for providing an instruction to end (stop) the registration processing. The control unit 21 determines whether or not the instruction (registration instruction) to execute the user information registration processing has been received (S15) in accordance with whether or not the registration button has been operated via the input unit 24, and in a case where it is determined that the registration instruction has not been received (S15: NO), the processing in Step S14 is repeated. In a case where it is determined that the registration instruction has been received (S15: YES), the control unit 21 transmits each piece of information input via the input screen (user information) and the member ID of the user in an associated manner to the server 10 (S16) and provides an instruction to register the user information to the server 10. Specifically, in a case where profile information (attribute information, preference information, and the thought trend of the user) is input via the input screen illustrated in FIG. 8B, the profile information and the member ID are transmitted to the server 10. Also, the body information (biological information) and the member ID are transmitted to the server 10 in a case where body information is input via the input screen illustrated in FIG. 9A, and exercise and sleep information and the member ID are transmitted to the server 10 in a case where exercise and sleep information is input via the input screen illustrated in FIG. 9B. Note that the member ID of the user is set in the advice APP 22AP, for example.

The control unit 11 of the server 10 acquires the user information transmitted by the user terminal 20 and registers the acquired user information in the member information DB 12b (S17). Specifically, the control unit 11 acquires the member ID and the user information from the user terminal 20 and stores each piece of the acquired user information in the member information DB 12b in association with the acquired member ID. Note that in regard to the information that has already been stored in the member information DB 12b, each piece of information newly acquired from the user terminal 20 may be additionally stored or may be stored in an overwritten manner.

Also, each piece of information included in the user information may be stored in association with current date and time (update date and time). In a case where each piece of information acquired from the user terminal 20 is successively added and stored, the server 10 can acquire time-series user information from the user terminal 20 and can accumulate the time-series user information. Also, in a case where each piece of information is stored in an overwritten manner, the server 10 can hold the latest user information.

The control unit 11 notifies the user terminal 20 of an end of the registration of the user information after storing the user information acquired from the user terminal 20 in the member information DB 12b (S18). In a case where the notification of the end of the registration is provided from the server 10, the control unit 21 of the user terminal 20 displays a screen indicating the end of the registration of the user information on the display unit 25 (S19). Note that the control unit 21 may return the display to the initial screen of the input screen (registration screen) by causing the display unit 25 to display the screen illustrated in FIG. 8A instead of displaying the user information registration end screen. In a case where it is determined that the instruction to input any of user information has not been received on the screen illustrated in FIG. 8A in Step S12 (S12: NO), the control unit 21 skips the processing in Step S13 to S19.

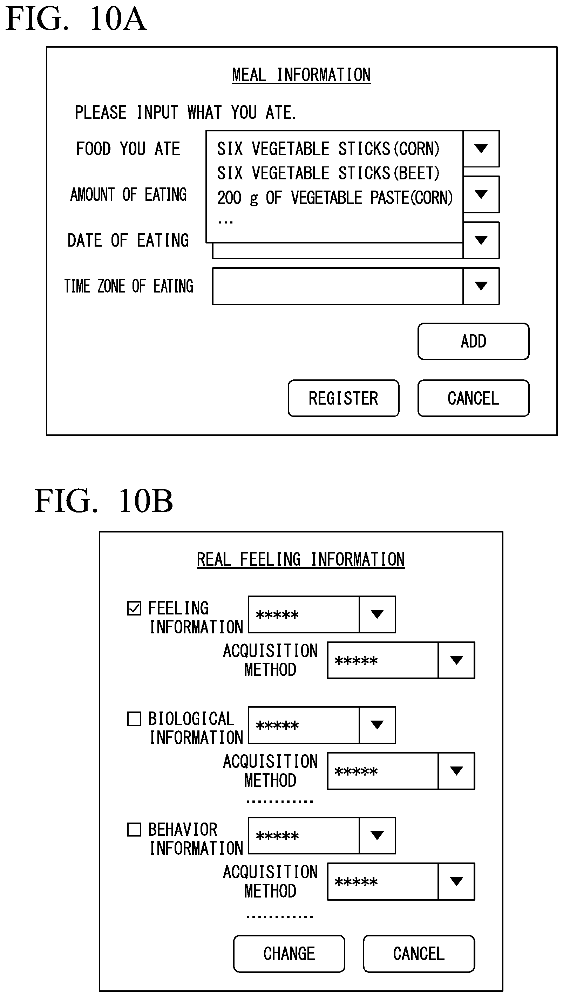

Next, the control unit 21 determines whether or not an instruction to input meal information (user information) has been received on the screen illustrated in FIG. 8A (S20). In a case where it is determined that the instruction to input meal information has been received (S20: YES), the control unit 21 displays the meal information input screen as illustrated in FIG. 10A on the display unit 25 (S21). The meal information input screen illustrated in FIG. 10A has an input section for inputting information (user intake information) regarding foods that the user has eaten (taken). Specifically, the meal information input screen has an input section for inputting each of the product names of products that the user has eaten, the amounts by which the user has eaten the foods (intake amounts), the dates on which the user has eaten the foods (dates), and time zones in which the user has eaten the foods. A pull-down menu from which arbitrary one of products that are sales targets can be selected is provided at each input section for the product name of the food that the user has eaten, and it is possible to input an arbitrary product name by using the pull-down menu.

For example, in a case where a list of food product names is stored in the storage unit 22, the control unit 21 can read the list of product names from the storage unit 22 and provide a pull-down menu displaying the read list of product names at the input section for the product name. For example, the control unit 21 causes the storage unit 22 to store the list of product names of the foods that are stored in a device for storing foods, such as a refrigerator, and are owned by the user. The control unit 21 updates the list of product names of the products stored in the storage unit 22 to information regarding foods that the user has not actually taken from among the foods owned by the user, by deleting the information regarding foods that the user has actually taken from among the list of the product names of the foods that the storage unit 22 is caused to store.

Also, in a case where the list of product names as described above is not stored in the storage unit 22, the control unit 21 may acquire the aforementioned list of product names from the server 10 and provides a pull-down menu displaying the acquired list of product names at the input section for the product name.

Additionally, a list of product names of foods that the user has purchased and has not yet eaten may be displayed in the pull-down menu provided at the input section for the product names as illustrated in FIG. 10A. In this case, if the user's purchase history and intake history of the foods are stored in the storage unit 22, the control unit 21 specifies foods that have not yet been consumed (not yet been eaten) on the basis of the purchase history and the intake history, generate the list of product names of the foods that have not yet been consumed, and display the list of product names in the pull-down menu. At this time, the control unit 21 specifies the remaining amounts of the foods that have not yet been consumed on the basis of the purchase history and the intake history and display the remaining amount of each food along with the list of product names of the foods that have not yet been consumed in the pull-down menu.

Also, in a case where the user's purchase history and intake history of the foods are not stored in the storage unit 22, the control unit 21 may acquire the purchase history and the intake history from the member information DB 12b of the server 10, generate a list of product names of the foods that have not yet been consumed on the basis of the acquired purchase history and intake history, and display the list of the product names in the pull-down menu. Note that the control unit 11 of the server 10 may generate the list of product names of the foods that have not yet been consumed and specify the remaining amount of each food on the basis of the purchase history and the intake history stored in the member information DB 12b.

In this case, it is only necessary for the control unit 21 to display the list of product names and the remaining amount of each food acquired from the server 10 in the pull-down menu. Note that the pull-down menu may be displayed or may not be displayed such that the names of products of the foods that the user has already eaten or the foods that has not been purchased cannot be selected. With such a configuration, the user can easily select the products that the user himself/herself has eaten, and the input operation is facilitated. Also, in the case where the remaining amount of each food is displayed as illustrated in FIG. 10A, the user can easily recognize the remaining amounts of foods as well.

A pull-down menu from which it is possible to select an arbitrary numerical value from among a plurality of numerical values is provided in the input section for the amount by which the user has eaten the food is provided, and it is possible to input an arbitrary numerical value by using the pull-down menu. Note that the pull-down menu is configured such that the number of grams (weight) can be selected in a case where a paste-like food is input as an eaten product, for example, and the pull-down menu is configured such that the number can be selected in a case where a stick-like food is input. Pull-down menus from which it is possible to select an arbitrary date and time zone from a plurality of dates and time zones are provided at the input sections for the date on which the food is eaten and for the time zone in which the food is eaten (intake timing), respectively are provided, and it is possible to input an arbitrary date and time zone by using the pull-down menu. The meal information input screen is not limited to the configuration illustrated in FIG. 10A, and each input section may be configured such that arbitrary information can be input via the input unit 24.

The user inputs each piece of information regarding the product names of the foods that the user himself/herself has eaten, the amount by which the user has eaten the foods, and the date and time (the date and the time zone) to the input section on the input screen as illustrated in FIG. 10A. The control unit 21 of the user terminal 20 receives each piece of information (meal information) input by the user via the input unit 24 (S22) and displays each piece of received information in each corresponding input section. The input screen illustrated in FIG. 10A includes a registration button for providing an instruction to execute processing of registering each piece of input information (meal information) in the server 10 and a cancel button for providing an instruction to end (stop) the registration processing. The control unit 21 determines whether or not the instruction (registration instruction) to execute the meal information registration processing has been received in accordance with whether or not the registration button has been operated via the input unit 24 (S23), and in a case where it is determined that the registration instruction has not been received (S23: NO), the processing in Step S22 is repeated. In a case where it is determined that the registration instruction has been received (S23: YES), the control unit 21 transmits each piece of information (the meal information and the user intake information) input via the input screen and the member ID of the user in an associated manner to the server 10 (S24) and provides an instruction to register meal information (user information) to the server 10.

The control unit 11 (acquisition unit) of the server 10 acquires meal information (user intake information) transmitted by the user terminal 20 and registers the acquired meal information in the member information DB 12b (S25). Specifically, the control unit 11 acquires the member **ID** and the meal information from the user terminal 20 and stores the acquired meal information in the intake history stored in the member information DB 12b in association with the acquired member ID. Here, the control unit 11 causes the intake history that has already been stored in the member information DB 12b to additionally store the meal information acquired from the user terminal 20. The control unit 11 notifies the user terminal 20 of an end of the registration of the meal information after storing the meal information acquired from the user terminal 20 in the member information DB 12b (S26). In a case where the notification to end the registration is provided from the server 10, the control unit 21 of the user terminal 20 displays a screen indicating the end of the registration of the meal information on the display unit 25 (S27). Note that the control unit 21 may return the display to the initial screen of the registration screen by causing the display unit 25 to display the screen illustrated in FIG. 8A instead of displaying the meal information registration end screen in this case as well. In a case where it is determined that an instruction to input meal information has not been received on the screen illustrated in FIG. 8A in Step S20 (S20: NO), the control unit 21 skips the processing in Steps S21 to S27.

Furthermore, the control unit 21 determines whether or not an instruction to input real feeling information has been received on the screen illustrated in FIG. 8A (S28). In a case where it is determined that the instruction to input real feeling information has been received (S28: YES), the control unit 21 displays an input screen of the real feeling information as illustrated in FIG. 10B on the display unit 25 (S29). The input screen of the real feeling information illustrated in FIG. 10B has an input section for inputting information of user's real feeling (user real feeling score information). Specifically, the input screen of the real feeling information has an input section for inputting each of user's feeling information, user's biological information, and user's behavior information.

A pull-down menu from which it is possible to select arbitrary one of user's feelings is provided at the input section for the user's feeling information, and it is possible to input an arbitrary feeling by using the pull-down menu. For example, in a case where a list of information specifying a feeling is stored in the storage unit 22, the control unit 21 can read the list of information specifying a feeling from the storage unit 22 and provide a pull-down menu displaying the list of information specifying the read feeling in the input section for the user's feeling information.

Also, in a case where the list of information specifying a feeling as described above is not stored in the storage unit 22, the control unit 21 may acquire the aforementioned list of information specifying a feeling from the server 10 and provide a pull-down menu displaying the acquired list of information specifying a feeling in the input section for the feeling information. Also, as illustrated in FIG. 10B, a list of methods by which the user's feeling has been acquired may be displayed in the pull-down menu provided in the input section for the user's feeling information. A pull-down menu that allows an arbitrary acquisition method to be selected from the plurality of acquisition methods is provided in the input section for the method of acquiring the user's feeling, and it is possible to input an arbitrary acquisition method by using the pull-down menu.

Also, the user's feeling information may be acquired from user biological information regarding the user's living body or may be acquired from user behavior information regarding a user's behavior. Moreover, the user's feeling information may be time-series data of user's feelings. In this case, the user's feeling information may include time-series data of the user's feelings either before or after or both before and after the user takes a food. Also, the user's feeling information may include at least one of user feeling information per unit time and information specifying emotional ups and downs. Here, the unit time may be one day or more, 1 week or more, 1 month or more, 3 months or more, 1 year or more, or three years or more, and the unit time may include two or more unit times in the same season in different years. Particularly, it is possible to provide, to the user, information regarding a food intake mode that gives an intermediate-to-long-term real feeling over a specific period of time rather than giving a transitory pleasure to the user by the user feeling information being an average value per unit time that is equal to or greater than 1 month, which is preferable. A food intake mode in which ups and downs of the feeling information per shorter unit time (1 hour, for example) is equal to or less than a specific level (that is, emotional ups and downs are small) and an average value of the user feeling information is large is preferably included. For example, as compared with a food by which feeling information is enhanced in a relatively short (1 hour, for example) unit time like a candy but the feeling information average value then drops in a relatively long (1 year or more, for example) unit time due to an increase in weight or the like, a food by which the feeling information in a relatively short (1 hour, for example) unit time is lower than that of the former example but the feeling information average value in a relatively long (1 year or more, for example) unit time the becomes higher due to a body condition adjustment effect or the like caused by an effect of dietary fiber like a vegetable with sweet taste can be selected as a preferable mode of enhancing the real feeling score information.

A pull-down menu from which any one item can be selected from the living body of the user is provided in the input section for the user's biological information, and it is possible to input an arbitrary item regarding the living body by using the pull-down menu. In a case where a list of information specifying the living body is stored in the storage unit 22, for example, the control unit 21 can read the list of information specifying the living body from the storage unit 22 and provide a pull-down menu displaying the read list of information specifying the living body in the input section for the biological information of the user. Also, in a case where the list of information specifying the living body as described above is not stored in the storage unit 22, the control unit 21 may acquire the aforementioned list of information specifying the living body from the server 10 and provide the pull-down menu displaying the acquired list of information specifying the living body in the input section for the biological information. Also, as illustrated in FIG. 10B, a list of methods of acquiring the living body of the user may be displayed in the pull-down menu provided in the input section for the biological information of the user. A pull-down menu from which an arbitrary acquisition method can be selected from among the plurality of acquisition methods is provided in the input section for the methods of acquiring the living body of the user, and it is possible to input an arbitrary acquisition method by using the pull-down menu. In addition, the user's biological information may be time-series data regarding the living body of the user.

A pull-down menu from which arbitrary one of user's behaviors can be selected is provided in the input section for the user's behavior information, and it is possible to input an arbitrary behavior by using the pull-down menu. In a case where a list of information specifying behaviors is stored in the storage unit 22, for example, the control unit 21 can read the list of information specifying behaviors from the storage unit 22 and provide the pull-down menu displaying the read list of information specifying behaviors in the input section for the user's behavior information.

Also, in a case where the list of information specifying behaviors as described above is not stored in the storage unit 22, the control unit 21 may acquire the aforementioned list of information specifying behaviors from the server 10 and provide a pull-down menu displaying the acquired list of information specifying behaviors in the input section for behavior information. Also, as illustrated in FIG. 10B, a list of methods of acquiring user's behaviors may be displayed in the pull-down menu provided in the input section for the user's behavior information. A pull-down menu from which it is possible to select an arbitrary acquisition method from the plurality of acquisition methods is provided in an input section for the method of acquiring user's behavior, and it is possible to input an arbitrary acquisition method by using the pull-down menu. The user's behavior information may include information specifying a behavior having a relatively low user real feeling score but highly required in terms of user's preference and/or daily habits, on the basis of information regarding a correlation between user intake information and user real feeling score information. The input screen of the real feeling information is not limited to the configuration illustrated in FIG. 10B, and each input section may be configured such that arbitrary information can be input thereinto via the input unit 24.

The user inputs, to each input section, one or more pieces of information from among feeling information, biological information, and behavior information on the input screen as illustrated in FIG. 10B. The control unit 21 of the user terminal 20 receives each piece of information (real feeling information) input by the user via the input unit 24 (S30) and displays each piece of received information in each corresponding input section. The input screen illustrated in FIG. 10B includes a registration button for providing an instruction to execute processing of registering input each piece of information (real feeling information) in the server 10 and a cancel button for providing an instruction to end (stop) the registration processing. The control unit 21 determines whether or not an instruction (registration instruction) to execute processing of registering meal information has been received on the basis of whether or not the register button has been operated via the input unit 24 (S31), and in a case where it is determined that the registration instruction has not been received (S31: NO), the control unit 21 repeats the processing in Step S30. In a case where it is determined that the registration instruction has been received (S31: YES), the control unit 21 transmits each piece of information (the feeling information, the biological information, and the behavior information) input via the input screen and the member ID of the user in an associated manner to the server 10 (S32) and provides an instruction to register meal information (user information) to the server 10.

The control unit 11 (acquisition unit) of the server 10 acquires the feeling information, the biological information, and the behavior information (real feeling information) transmitted by the user terminal 20 and registers the acquired feeling information, biological information, and behavior information in the member information DB 12b (S33). Specifically, the control unit 11 acquires the member ID and the feeling information from the user terminal 20 and stores the acquired feeling information in a feeling history stored in the member information DB 12b in an association with the acquired member ID. Here, the control unit 11 causes the feeling information acquired from the user terminal 20 to be additionally stored in the feeling history that has already been stored in the member information DB 12b.

The control unit 11 acquires the member ID and the biological information from the user terminal 20 and stores the acquired biological information in a biological history that is stored in the member information DB 12b in association with the acquired member ID. Here, the control unit 11 causes the biological information acquired from the user terminal 20 to be additionally stored in the biological history that has already been stored in the member information DB 12b. The control unit 11 acquires the member ID and the behavior information from the user terminal 20 and stores the acquired behavior information in the behavior history that is stored in the member information DB 12b in association with the acquired member ID. Here, the control unit 11 causes the behavior information acquired from the user terminal 20 to be additionally stored in the behavior history that has already been stored in the member information DB 12b.

The control unit 11 stores the feeling information, the biological information, and the behavior information acquired from the user terminal 20 in the member information DB 12b and then notifies the user terminal 20 of an end of the registration of the real feeling information (S34). The control unit 21 of the user terminal 20 displays a screen indicating an end of registration of the real feeling information on the display unit 25 in the case where the notification of the end of registration is provided from the server 10 (S35). Note that the control unit 21 may return the display to the initial screen of the registration screen by causing the display unit 25 to display the screen illustrated in FIG. 8A instead of displaying the real feeling information registration end screen. In a case where it is determined that an instruction to input real feeling information has not been received on the screen illustrated in FIG. 10B in Step S28 (S28: NO), the control unit 21 skips the processing in Steps S29 to S35.

Through the aforementioned processing, user information such as the attribute information, the profile information, the information regarding the body condition (biological information), the information regarding exercise, the information regarding sleep, the types, the amounts (intake amounts), and the intake timings of foods that the user has eaten, real feeling information and the like of the user is input and registered in the server 10 by using the user terminal 20 in the information processing system 100 according to the present embodiment. In the server 10, the control unit 11 may create predetermined information such as information to be utilized to develop products on the basis of the registered user intake information, user real feeling score information, and user identification information and output the created predetermined information. For example, the control unit 11 may receive inputs of types, amounts (intake amounts), and intake timings of foods related to the user's real feeling score. or nutrient components or effective components of the above foods, or effective materials containing the nutrient components or effective components from the relationship between relatively high user real feeling score information and the user intake information and register the input types, amount (intake amount), and intake timing of the foods, or the nutrient components or effective components of the foods, or the effective materials containing the nutrient components or the effective components. The control unit 11 may create predetermined information such as information to be utilized to develop products including the registered information and output the created predetermined information.

In another example, the control unit 11 may receive inputs of types, amounts (intake amounts), and intake timings of foods necessary for the user, or nutrient components or effective components of the above foods, or effective materials containing the nutrient components or the effective components from the relationship between relatively low user real feeling score information and user intake information and register the input types, amounts (intake amounts), and intake timings of the foods, or the nutrient components or the effective components of the foods, or the effective materials containing the nutrient components or the effective components. The control unit 11 may create predetermined information such as information to be utilized to develop products including the registered information and output the created predetermined information.

Note that the user information includes a vegetarian level of the user, information regarding preference or a principle in regard to foods (preference information), a thought trend (what is in mind) in everyday life, and the like. Also, the user information may include information (behavior information) regarding daily habits (behaviors as habits) in everyday life including exercise and sleep. The attribute information of the user may be time-series data of the attribute information of the user. The foods (the taken foods) that the user has eaten may include foods having relatively low user real feeling scores but highly required in terms of user preference and/or daily habits on the basis of the information regarding the correlation between the user intake information and the user real feeling score information.

Also, a comment regarding a change in body condition or emotion issued by the user eating the foods and information (shared information) regarding a food that the user desires to recommend to other users or cooking recipe and the like using the food may be included.

Furthermore, through the aforementioned processing, the information processing system 100 according to the present embodiment can provide a more appropriate advice (advice or the like in accordance with a score based n a current or future disease risk or various references, which will be described later) to the user by combining the aforementioned input information (real feeling information) in addition to the information regarding the intake amount and the intake timing of the foods that the user has taken in regard to foods. Also, in a case where a food contains an arbitrary nutrient component which will be described later in an effective amount or more, or in a case where a food contains a nutrient component and an effective component, it is possible to provide advice in regard to health functions that can be expected by the nutrient component or the effective component or both the nutrient component and the effective component, which is more preferable.

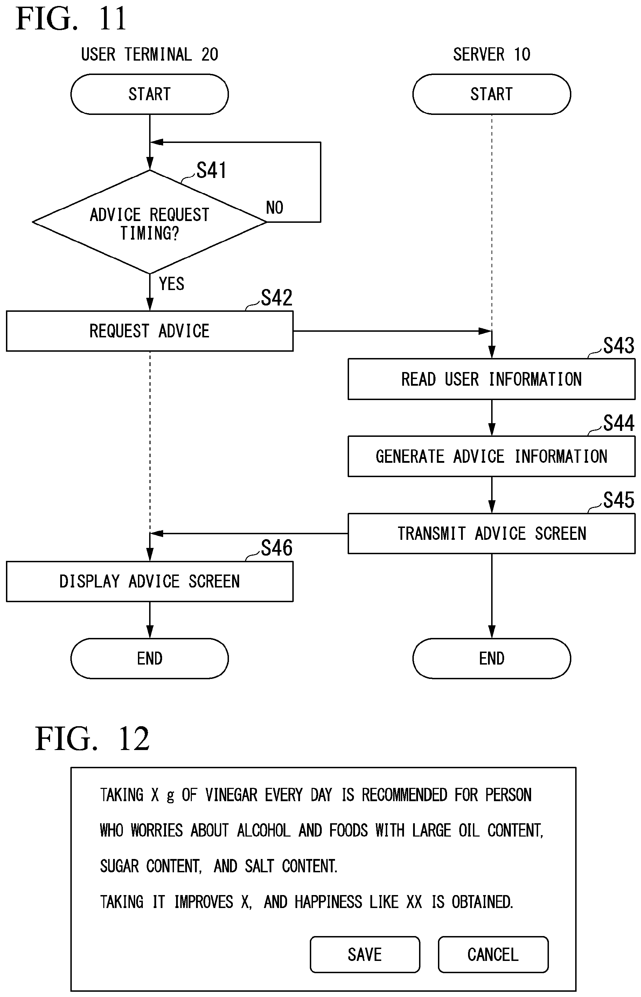

As described above, the server 10 successively register various kinds of information regarding the user and provides an appropriate advice to the user in accordance with the registered information. Next, processing performed by each device when the server 10 provides advice in accordance with user information of each user in the information processing system 100 will be described. FIG. 11 is a flowchart illustrating an example of advice providing processing procedure, and FIG. 12 is a schematic diagram illustrating a screen example of the user terminal 20. In FIG. 11, processing performed by the user terminal 20 is illustrated on the left side, and processing performed by the server 10 is illustrated on the right side, respectively. The following processing is executed by the control unit 21 in accordance with the control program 22P and the advice APP 22AP stored in the storage unit 22 of the user terminal 20 and executed by the control unit 11 in accordance with the control program 12P stored in the storage unit 12 of the server 10. Apart of the following processing may be realized by a dedicated hardware circuit.

In the information processing system 100, in a case where the user desires to receive provision of advice from the server 10, the user causes the user terminal 20 to activate the advice APP 22AP and requests for advice from the server 10 via the advice APP 22AP. Note that the user can cause the user terminal 20 to display a home screen (activation screen) by causing the user terminal 20 to activate the advice APP 22AP and require for advice via the home screen.

In a case where an instruction to activate the advice APP 22AP is received from the user via the input unit 24, the control unit 21 of the user terminal 20 causes the advice APP 22AP to be activated and displays the home screen on the display unit 25. Thereafter, in a case where an instruction to request for advice is received via the input unit 24, the control unit 21 requests for advice from the server 10. Note that the control unit 21 may request for advice from the server 10 without receiving a request instruction from the user when the advice APP 22AP is caused to be activated. In this case, it is only necessary for the user to perform an operation of activating the advice APP 22AP, and it is not necessary to perform an operation regarding an advice request instruction. Also, the control unit 21 may request for advice from the server 10 regularly or every time a preset clock time arrives after the advice APP 22AP is caused to be activated (that is, during the operation of the advice APP 22AP).

The control unit 21 of the user terminal 20 determines whether or not a timing at which the advice is to be requested from the server 10 has arrived in the case where the advice APP 22AP is activated (S41). For example, in a case where an advice request instruction is received via the home screen, the control unit 21 determines that the advice request timing has arrived. Also, the control unit 21 may determine that the advice request timing has arrived in a case where the processing of activating the advice APP 22AP is performed or may determine that the advice request timing has arrived in a case where a predetermined period of time has elapsed or in a case where a preset clock time has arrived. The control unit 21 waits while performing other processing in a case where it is determined that the advice request timing has not arrived (S41: NO).

In a case where it is determined that the advice request timing has arrived (S41: YES), the control unit 21 requests for advice from the server 10 (S42). Specifically, the control unit 21 transmits a member ID of the user and a request signal for requesting for advice to the server 10. In a case where the request for advice is received from the user terminal 20, the control unit 11 of the server 10 reads user information stored in the member information DB 12b in association with the member ID received from the user terminal 20 (S43). Then, the control unit 11 (information processing unit) generates appropriate advice information for the read user information on the basis of content stored in the advice DB 12c (S44). The control unit 11 generates advice information to be provided in consideration of at least a part of the user information stored in the member information DB 12b and a time zone (clock time) in which the request for advice has been issued.

For example, the control unit 11 obtains a correlation between the intake amount of a nutrient component that the user has taken and one or more of a feeling history, a biological history, and a behavior history on the basis of the intake history and the one or more from among the feeling history, the biological history, and the behavior history read from the member information DB 12b. In other words, one or more pieces of information from among user feeling information, user biological information, and user behavior information registered in advance and/or user real feeling score information derived from the information may be used, or user real feeling score information calculated every time may be used, as information obtained on the basis of the one or more from among the feeling history, the biological history, and the behavior history.

Specifically, the intake history of the user in a specific period of time and one with a large variation from among the feeling history, the biological history, and the behavior history are selected, and a point set in advance depending on the size of the variation is regarded as a real feeling score. Furthermore, another history with a high correlation with the history selected from a learning model stored in the storage unit can be selected, and a point predicted from the learning model from another history can be added to or accumulated on the selected score, and the result can be used as a real feeling score.

As a method of calculating the user real feeling score, the control unit 21 converts a keyword or numerical value data in each of the feeling information, the biological information, and the behavior information stored in the storage unit 22 into a numerical value of a positive point or a negative point, obtains arithmetic means of the converted numerical value, and derives a total value of resulted arithmetic means. Alternatively, the control unit 21 may obtain arithmetic means and derive a total value of the resulted arithmetic means by using a numerical value calculated by multiplying the numerical value of the positive point or the negative point by a correction coefficient specified for each user. Here, the values are not limited to the arithmetic means, and an arbitrary statistical value may be derived, and the derived statistical value may be used.

Alternatively, the control unit 21 may use data such as a peak maximum value, a minimum value, a chart area, or the like obtained from a biorhythm chart. Alternatively, the control unit 21 may represent a radar chart area value by plotting a numerical value and a point of each item on the radar chart. Also, a method of calculating a real feeling score by multiplying a numerical value selected from any of the acquired "user feeling information", "user biological information", and "user behavior information" by a correction coefficient may be employed, a neural network calculation method of calculating a real feeling score via a plurality of temporary scores obtained by multiplying the numerical value by a correction coefficient may be employed., or a deep learning method of calculating a real feeling score via multiple stages for calculating the temporary score may be employed.

In regard to the feeling information, the control unit 11 may use feeling information obtained by using a color chart and causing a user to select a color of the feeling from the color chart and converting the color selected by the user into a numerical value, or the control unit 11 may use feeling information obtained by converting the color selected by the user into a numerical value of a positive point or a negative point, obtaining an arithmetic means of the numerical value obtained through the conversion, and deriving a total value of the resulted arithmetic means. Here, the values are not limited to the arithmetic means, and an arbitrary statistical value may be derived, and the derived statistical value may be used. Alternatively, the control unit 21 may calculate a negative point, a positive point, and other numerical values of each of the user feeling information, the user biological information, and the user behavior information by numerical values obtained by positions represented by three-axis coordinates of an x axis, a y axis, and a z axis or represent the positions represented by the three-axis coordinates as vectors. For example, the control unit 11 may set coordinates to represent effects such as a "reference", a "model", a "target", an "ideal", an "expectation", an 'assumption", an "improvement", and the like and calculate the feeling information as an approximation rate between the coordinates of the user at the current time and the coordinates of the "reference" or the like. Alternatively, the control unit 11 may obtain a vector obtained from set coordinates and calculate the feeling information as an approximation rate between the vector of the user at the current time and the vector of the "reference" or the like.

The control unit 11 obtains a correlation between an intake history of the user and one or more pieces of information from among a feeling history, a biological history, and a behavior history and/or user real feeling score information derived from the information (the one or more pieces of information from among the feeling history, the biological history, and the behavior history). The control unit 11 may use data such as specific date and time, morning/evening/night, date, or the like or may use data obtained by summing up, averaging, or in a case of successive data, integrating numerical values of periods of time, such as specific periods of time, a specific number of days, every two days, weeks, months, years, for example, as data regarding the one or more information histories from among the feeling history, the biological history, and the behavior history. Alternatively, the control unit 11 may use numerical values calculated by multiplying the numerical values of periods of time, such as specific periods of time, a specific number of days, every two days, weeks, months, years, or the like by a correction coefficient specified for each user as the data regarding the one or more information histories from among the feeling history, the biological history, and the behavior history.

The control unit 21 may specify one or more of foods that the user has taken and/or nutrient components contained in the foods and/or the intake amounts thereof on the basis of the correlation between the intake history of the user and the one or more pieces of information from among the feeling history, the biological history, the behavior history, and the user real feeling score information derived therefrom (the feeling history, the biological history, and the behavior history). Here, an example of a change in feeling history is that anxiety about his/her health has decreased while a "happiness level" has increased in regard to the feeling history (1). An example of a change in biological history is that the user feels that the blood pressure that was slightly high has approached a normal value due to dropping of the blood pressure in regard to the biological history (2). An example of a change in behavior history is that a motivation of exercise for the behavior history has increased (3).

The control unit 21 determines a positive factor and a negative factor on the basis of a change in one or more pieces of information from among the feeling history, the biological history, the behavior history, and the user real feeling score information derived therefrom (the feeling history, the biological history, and the behavior history). For example, the control unit 21 determines each of (1) to (3) as a positive factor, the control unit 21 derives one or more pieces of information from among the feeling history, the biological history, and the user real feeling score information derived therefrom (the feeling history, the biological history, and the behavior history) determined as positive factors. The control unit 21 derives a causal relationship with one or more of the foods that the user has taken in one-day meals and/or nutrient components contained in the foods and/or the intake amounts thereof by performing analysis processing such as multivariate analysis or the like on the basis of the extracted information and the intake amounts of the nutrient components that the user has taken. The control unit 21 outputs advice information on the basis of one or more of the foods with causal relationships and/or the nutrient components contained in the foods and/or the intake amounts thereof on the basis of the derived causal relationship. For example, the control unit 21 outputs advice information on the basis of vinegar in a case where a causal relationship with the fact that the user has taken a larger amount of vinegar than usual by 10 mL in his/her one-day meals is derived through analysis processing such as multivariate analysis or the like on the basis of the extracted information and the intake amounts of the nutrient components that the user has taken.

Alternatively, the control unit 21 may specify at least one of the nutrient component that the user has taken with a change in user real feeling score information and the intake amounts of the nutrient component on the basis of the correlation between the intake history of the user and the user real feeling score information derived from one or more pieces of information from among the feeling history, the biological history, and the behavior history. Here, an example of the user real feeling score information is at least one of that anxiety about his/her health has decreased and the "happiness level" has increased in regard to the feeling history, that the user feels that the blood pressure that was slightly high has approached a normal value due to dropping of the blood pressure in regard to the biological history, and that the motivation of exercise has increased in regard to the behavior history.

Specifically, for the above determination of the positive factor and the negative factor, it is possible to use a user's intake history of a vinegar in a specific period of time, for example, (for 30 days or more, for example) and information generated by performing logistic regression analysis or an analysis using a determination tree model (for example, XGBoost, LightGBM, RandomForest) on questionnaire answer results regarding the feeling history, the biological history, and the behavior history, and construction can be achieved through machine learning or deep learning in the generation of the information. Particularly, it is possible to perform analysis with high precision by performing analysis using a determination tree model (for example, XGBoost, LightGBM, and RandomForest) on the same data and using an explanatory variable with high importance in both an explanatory variable obtained as a result of logistic regression analysis and the determination tree model regression analysis. Furthermore, in a case where the positive factor and the negative factor are determined from an intake history, a feeling history, a biological history, and a behavior history acquired from a specific user, it is possible to use an algorithm of a convolution neural network (CNN), a recurrent neural network (RNN), a long short-term memory (LSMT), random forest, support vector machine (SVM), a neural network, and the like described below.

Also, the control unit 21 can output advice information on the basis of attribute information, biological information, behavior information, or intake information (for example, one or more kinds from among foods and/or nutrient components contained in the foods and/or the intake amounts thereof) with causal relationships in the opposite direction with a plurality of pieces of feeling information from among derived causal relationships. For example, although a "running history" which is behavior information has a positive correlation with first feeling information "everyday vitality", the "running history" has a negative correlation with second feeling information "it is possible to concentrate" or "bright mood". In this case, in order to enhance both the first feeling information and the second feeling information, it is possible to provide a suggestion to increase a "vinegar intake frequency" which is meal intake information that enhances the second feeling history "it is possible to concentrate" while enhancing the behavior information "running history", or to provide a suggestion to carry out "exercise for about 10 minutes a day" to enhance the first feeling information "everyday vitality" while curbing the behavior information "running history". Additionally, it is also possible to provide predicted feeling information calculated from attribute information or the like before user's execution of the advice, as advice information, to the user.

The control unit 21 determines the positive factor and the negative factor on the basis of a change in user real feeling score information. For example, the control unit 21 determines, as a positive factor, each of the fact that the anxiety about his/her health has decreased and the "happiness level" has increased in regard to the feeling history (1), the fact that the user feels that the blood pressure that was slightly high has approached the normal value due to dropping of the blood pressure in regard to the biological history (2), and the fact that the motivation of exercise has increased in regard to the behavior history (3). The control unit 21 derives user real feeling score information from one or more pieces of information from among the feeling history, the biological history, and the behavior history determined as positive factors. The control unit 21 derives a causal relationship with at least one of the nutrient components that the user has taken from the one-day meal and the intake amounts of the nutrient components by performing analysis processing such as multivariate analysis or the like on the basis of the derived user real feeling score information and the intake amount of the nutrient component that the user has taken. The control unit 21 outputs advice information on the basis of at least one of the nutrient component with a causal relationship and the intake amount of the nutrient component on the basis of the derived causal relationship. For example, the control unit 21 outputs advice information on the basis of vinegar in a case where a causal relationship with the fact that the user has taken a larger amount of vinegar than usual by 10 mL in one-day meals is derived through analysis processing such as multivariant analysis or the like on the basis of the extracted information and the intake amount of the nutrient component that the user has taken.

As a specific example, a case where an intake history that the user took a larger amount of vinegar than usual by 10 mL in one-day meals has been obtained will be described. It is assumed that the user feels that the blood pressure that was slightly high has approached the normal value due to dropping of the blood pressure in the biological information, a positive factor has increased, the anxiety about his/her health has decreased, and the "happiness level" has increased due to dropping of the blood pressure in the feeling information. In other words, in a case where the effect of drinking vinegar is felt or the awareness of health has been enhanced, the former corresponds to a decrease in negative factor while the latter corresponds to an increase in positive factor. Also, a case where the motivation of exercise has been enhanced in the behavior information corresponds to an increase in positive factor.

In this case, the control unit 21 specifies at least one of the nutrient component that the user has taken, which has been changed in one or more pieces of information from among the feeling history, the biological history, and the behavior history and the intake amount of the nutrient component on the basis of the correlation between the intake history of the user and the one or more pieces of information from among the feeling history, the biological history, and the behavior history. The control unit 21 determines a positive factor and a negative factor on the basis of the change in the one or more pieces of information from among the feeling history, the biological history, and the behavior history. The control unit 21 extracts one or more pieces of information from among the feeling history, the biological history, and the behavior history determined as the positive factor. The control unit 21 derives a causal relationship with at least one of the nutrient components that the user has taken in the one-day meals and the intake amounts of the nutrient components through analysis processing such as multivariate analysis or the like on the basis of the extracted information and the intake amounts of the nutrient component that the user has taken.

Alternatively, the control unit 21 derives a correlation between an intake history of the user and user real feeling score information derived from one or more pieces of information from among a feeling history, a biological history, and a behavior history. For example, the control unit 21 converts a keyword or numerical data in each of feeling information, biological information, and behavior information stored in the storage unit 22 into a numerical value of a positive point or a negative point, obtaining arithmetic means of the converted numerical value, and derives a total value of the resulted arithmetic means. The control unit 21 calculates user real feeling score information on the basis of the derived total value. The control unit 21 acquires a correlation between the intake history of the user such as an intake of vinegar and the calculated user real feeling score information.

For example, it is possible to calculate a correlation coefficient between a target variable and each explanatory variable by obtaining a correlation coefficient calculated for each explanatory variable (user feeling information, user biological information, user behavior information) through multivariate analysis using an intermediate-to-long term feeling (real feeling) score over a specific period of time as a target variable. The control unit 21 categorizes the explanatory variables on the basis of the calculated correlation coefficients. For example, the control unit 21 categorizes correlation coefficients by regarding explanatory variables with correlation coefficients of equal to or greater than a specific value as positive factors and regarding explanatory variables with correlation coefficients of equal to or less than the specific value as negative factors. The control unit 11 may read content of advice registered in the advice DB 12c including information regarding the correlation on the basis of the result of the categorization of the correlation coefficients and generate advice information to be provided by using the read content of advice.

Also, the control unit 11 may create virtual user intake information for increasing a user real feeling score per unit time on the basis of the obtained information regarding the correlation and generate advice information including the created virtual user intake information. Also, the control unit 11 may create virtual user intake information for increasing a user real feeling score per unit time while including a food having a relatively low user real feeling score but highly required from the user preference and/or daily habits on the basis of the obtained information regarding the correlation and generate advice information including the created virtual user intake information, for example.

Also, the control unit 11 may create virtual user intake information for increasing a user real feeling score per unit time without changing behavior information on the basis of the obtained information regarding the correlation and generate advice information including the created virtual user intake information, for example. Also, the control unit 11 may create virtual user behavior information for increasing a user real feeling score per unit time on the basis of the obtained information regarding the correlation and generates advice information including the created virtual user behavior information, for example. Also, the control unit 11 may create information specifying a method for improving the user real feeling score without changing any one of or both preference or information related to daily habits on the basis of the obtained information regarding the correlation and generate advice information including the created information specifying the method of improving the user real feeling score.

Also, the control unit 11 may generate advice information by using content of advice registered in the advice DB 12c in association with ages, sexes, birthplaces, philosophy and beliefs, and the like of users or may generate advice information by using content of advice registered in the advice DB 12c in association with vegetarian levels, preference information, thought trends, and the like of the users. In this case, it is possible to generate advice information in consideration of attributes of the users, preferences, principles, ideas, and the like of the users in regard to materials and foods.

Also, the control unit 11 may generate advice information by using content of advice registered in the advice DB 12c in association with a body condition indicated by the biological information of the user, the amount of exercise indicated by exercise information, the amount of sleep indicated by sleep information, and the like. In this case, it is possible to generate advice information in consideration of the body condition, the amount of exercise, the amount of sleep, and the like of the user. Also, in a case where time-series information (time-series data) such as an intake history, biological information, exercise information, sleep information, and the like of the user is used, the control unit 11 may generate advice information in accordance with a body condition, the amount of exercise, the amount of sleep, and the like of the user specified from information at an arbitrary point or over an arbitrary period of time.

Moreover, the control unit 11 can also generate advice information in accordance with a food consumption trend of the user for the user who is continuously consuming a food on the basis of the food intake history of the user.

Also, the control unit 11 may freely select a population of users and acquire user intake information, user feeling information, user biological information, user behavior information, user attribute information (birthplaces, PDI values of the birthplaces, and the like) and the like of the users included in the selected population of the users. Also, the control unit 11 may change the selected users. The control unit 11 may acquire various kinds of user information of the users after receiving advice information in regard to the users who have already received the advice information. The control unit 11 may specify a meal and a behavior that are assumed to have high correlations with real feeling scores and output advice to recommend the specified meal and behavior. More specifically, it is possible to provide an appropriate advice to users with high relevance on the side of the intake information or on the side of the real feeling score information of the correlations or users who have been randomly picked up on the basis of the information regarding the correlation between the intake information and the real feeling score information saved in the advice DB 12c (for example, information regarding a correlation based on knowledge of professionals, information regarding a correlation obtained by a learning model based on the current or past data of assumed users who will use the advice, information regarding a correlation obtained by a learning model based on current or past data obtained from a user group arbitrarily including the assumed users). For example it is possible to output advice ("it is believed that intake of vinegar makes body movement better", "it is believed that vinegar poured into ramen noodles make body movement better", "it is believed that vinegar contained in sweet and sour pork makes body movement better") including information regarding a correlation between intake information and real feeling score information for a user (for example, "who takes a small/large amount of vinegar", "who prefers ramen noodles that is a cooked food for which vinegar can be used", or "who has taken sweet and sour pork which is a cooked food with high relevance with vinegar") who is determined to have a high relevance on the side of intake information on the basis of the correlation between arbitrary intake information (intake of "vinegar" which is a food, for example) and the real feeling score information (for example, feeling information that "body movement is good" has been collected from many of a certain group and the real feeling score is high), and it is possible to output advice ("it is believed that intake of vinegar makes body movement better") including the information regarding the correlation between the intake information and the real feeling score information for the user ("who has had poor body movement recently") who is determined to have a high relevance on the side of the real feeling score information.

Particularly, it is preferable to generate advice information by using "information regarding a correlation between intake information and real feeling score information" obtained by a learning model based on data obtained from a user group (which is preferably a group with the same attribute as that of the user, in particular) that does not include the assumed users in the past because it is possible to quickly provide advice to the user by using data on which analysis in regard to the correlation has already been done. Also, it is possible to generate advice information by using "information regarding a correlation between intake information and real feeling score information" obtained by a learning model based on current or past data obtained from the user group including the assumed users because the advice has a high self-fulfilling effect.

Furthermore, it is possible to provide a more effective advice by using user attribute information or the like at the time of determining provision conditions. For example, since a user in a nation with a relatively low PDI value places priority on review information, it is possible to cause the self-fulfilling effect of the present invention to be more strongly exhibited by outputting, to such a user, advice including the information regarding a correlation between intake information and real feeling score information such as "intake of vinegar makes body motion better in XXX (nation)". Also, a user in a relatively high PDI value places priority on information from professionals, it is possible to cause the self-fulfilling effect of the present invention to be more strongly exhibited by outputting advice including a correlation between intake information and real feeling score information from professionals such as "according to professionals, intake of vinegar makes body movement better" to such a user.

In this manner, the control unit 11 generates advice information including information regarding a correlation between intake information and the real feeling score information, for example, advice information regarding a behavior (daily habit) in everyday life of the user such as meal, exercise, sleep, and the like on the basis of the user information.

Also, the control unit 11 may generate advice information in accordance with environment information of the user for the user who is consuming a food on the basis of user environment information which is information specifying the environment of the user when the user takes the food. Here, the user environment information is temperature, humidity, and the like and may be time-series data. Advice information in one example may include advice that solves any one of or both anxiety and stress information. Also, advice information in an example may include any one of or both advice for improving daily habits and advice for solving any one of or both anxiety and stress information regarding a change in user's behavior. In this manner, the control unit 11 generates advice information regarding behaviors (daily habits) in everyday life of the user, such as meal, exercise, sleep, and the like on the basis of the user environment information.

Note that the foods in the present embodiment are mainly general foods and includes foods that are manufactured to include not only edible parts but also non-edible parts of materials. The "non-edible parts" of the materials in the present disclosure indicate parts that are typically not suitable for eating or drinking of the materials and parts that are discarded in an ordinary eating habit, and specifically discarded sites described in "Japanese Food Ingredients Chart 2015 (seventh revision)" (food ingredient chart defined by the Ministry of Health, Labor and Welfare), for example.

Also, materials that are used for the foods in the present embodiment may be any materials as long as they are foods that are used for eating and drinking of humans (the foods listed in "Japanese Food Ingredients Chart 2015 (seventh revision)") and are preferably plants, that is, edible plants, and it is possible to use beans, seeds, vegetables, grains, fruits, potatoes, mushrooms, algae, and the like among the categories described in "Japanese Food Ingredients Chart 2015 (seventh revision)" as plants. Note that the "edible parts" of the materials indicate parts obtained by removing discarded sites (non-edible parts) from the entire materials. The non-edible parts of the materials have poor feeding properties or combinations with other materials and are often discarded without being used for eating.

However, in a case where the foods in the present embodiment contain non-edible parts of materials, it is possible to reduce the amount of waste of the materials. Therefore, it is possible to reduce burdens of the material (non-edible part) of discarding processing on the global environment by eating the foods in the present embodiment and to contribute to protection of the global environment. Therefore, the control unit 11 may generate not only advice related to a meal and a nutrient component on the basis of a user's intake history but also advice information for contribution to protection of the global environment. In this case, the control unit 11 can generate advice information for contribution to protection of the global environment by using content of the advice registered in the advice DB 12d in association with each food and the amount of taken food. Furthermore, the foods in the present embodiment preferably contain both edible parts and non-edible parts of materials, further preferably contain both edible parts and non-edible parts derived from the same types of materials, and most preferably contain both edible parts and non-edible parts derived from the materials of the same types and same individuals.

As described above, advice information to be provided to the user is generated in accordance with at least a part of user information in the present embodiment. Note that in a case where a plurality of pieces of advice information are generated on the basis of content stored in the advice DB 12c, the control unit 11 may use all generated pieces of advice information as the advice information to be provided or may use randomly selected one piece of advice information as the advice information to be provided. Also, in a case where a plurality of pieces of advice information are generated, advice information with high priority may be used as the advice information to be provided, by setting priority for each advice stored in the advice DB 12c.

Note that the content of an arbitrary nutrient component in the food in the present embodiment is preferably equal to or greater than an effective amount (equal to or greater than a reference value of nutrient that can be displayed in an emphasized manner according to the food labeling law). Also, the food preferably contains one kind or two or more kinds of effective components. In the above description, the effective component is a component other than the nutrient components defined in Attached Table 9 of the food display standards (Cabinet Office Ordinance No. 10, 2015), for example, which are effective to maintain and improve health, exercise functions, beauty, and the like, and specific examples include polyphenol, carotenoid, chlorophyll, vitamin U, and the like. In regard to the content of the nutrient components, specifically, the content is preferably an amount in a level in which it is possible to display that replenishment can be performed, display that appropriate intake can be performed, display that the nutrient component has not been added, absolute display(high, included), absolute display (not included, low), relative display (display that the nutrient component has been enhanced or reduced), and non-addition emphasized display (sugars, sodium salts, and the like).

Further specifically, the content of dietary fiber (particularly, the content of insoluble dietary fiber) in the food is preferably equal to or greater than 3% by mass, is more preferably equal to or greater than 4% by mass, is more preferably equal to or greater than 5% by mass, is more preferably equal to or greater than 6% by mass, is more preferably equal to or greater than 7% by mass, is more preferably equal to or greater than 8% by mass, and is further preferably equal to or greater than 9% by mass, and the amount of dietary fiber (particularly, the amount of insoluble dietary fiber) contained per one serving size (corresponding to the amount by which the food is typically eaten once, and corresponding to one product in a case of a product with a size for one-time eating, for example) is equal to or greater than 1 g, is more preferably equal to or greater than 2 g, and is more preferably equal to or greater than 3 g. It is possible to collect the intake amount of the nutrient component contained in the food of the user in a specific period of time along with consumption date and time from the user by the food containing such a nutrient component, to know an effective intake timing and intake method of the nutrient component which cannot be known in the related art, and thus to propose more effective intake timing and intake method of the nutrient component than those in the related art to the user, which is preferable.

Also, the food in the present embodiment is preferably an effective material containing the above nutrient component or an effective component, is further preferably a food (a dietary fiber-containing food) that is manufactured by using a material containing dietary fiber (particularly, the food is preferably derived from a dietary fiber localized site and is further preferably derived from a non-edible part of the material), and is further preferably a food (insoluble dietary fiber-containing food) manufactured by using a material containing insoluble dietary fiber (particularly, the food is preferably derived from an insoluble dietary fiber localized site and is further preferably derived from a non-edible part of the material).

The insoluble dietary fiber localized site is a site where insoluble dietary fiber is localized in the entire material, and specifically means a site with a higher insoluble dietary fiber-containing proportion than an insoluble dietary fiber-containing proportion in the edible part. The insoluble dietary fiber localized site in the present disclosure represents a site where insoluble dietary fiber is localized in the entire material, specifically, a site with a higher insoluble dietary fiber-containing proportion than the edible part in the material, and represents a site with an insoluble dietary fiber containing proportion of more preferably equal to or greater than 1.1 times, further preferably equal to or greater than 1.2 times, further preferably equal to or greater than 1.3 times, further preferably equal to or greater than 1.4 times, further preferably equal to or greater than 1.5 times, further preferably equal to or greater than 1.6 times, further preferably equal to or greater than 1.7 times, further preferably equal to or greater than 1.8 times, further preferably equal to or greater than 1.9 times, and the most preferably equal to or greater than 2.0 times the insoluble dietary fiber-containing proportion of the edible part in a dried state.

Also, the insoluble dietary fiber-containing proportion in the insoluble dietary fiber localized site (particularly, non-edible part) is preferably greater than 10% by mass, is further preferably greater than 11% by mass, is further preferably greater than 12% by mass, is further preferably greater than 13% by mass, is further preferably greater than 14% by mass, is further preferably greater than 15% by mass, is further preferably greater than 16% by mass, is further preferably greater than 17% by mass, is further preferably greater than 18% by mass, is further preferably greater than 19% by mass, and is further preferably greater than 20% by mass in terms of dried mass. Also, the same as above also applies to the dietary fiber localized site. Note that "int terms of dried mass" in the present disclosure indicates a mass conversion value when the water content is 0% by mass. Note that the measurement of water content in a sample can be measured in accordance with Japanese Food Ingredients Chart 2015 (seventh revision). Although the dietary fiber localized site or the insoluble dietary fiber localized site in the present disclosure may be a part of an "edible part" (for example, seed skin parts of vegetables, grains, beans, or fruits, particularly, seed skin parts of beans) or a "non-edible part" of the aforementioned food, the insoluble dietary fiber localized site is preferably a "non-edible part".

Also, the content of the dietary fiber localized site or an insoluble dietary fiber localized site (particularly, a non-edible part) with respect to the total mass of the entire food in the present disclosure is preferably equal to or greater than 1% by mass, is more preferably equal to or greater than 3% by mass, and is further preferably equal to or greater than 5% by mass. On the other hand, although an upper limit is typically not limited, the upper limit is preferably equal to or less than 70% by mass, is more preferably equal to or less than 60% by mass, and is more preferably equal to or less than 50% by mass. Also, the content of the dietary fiber localized site or the insoluble dietary fiber localized site (particularly, a non-edible part) with respect to the total mass of the entire food in the present disclosure is preferably equal to or greater than 3% by mass, is more preferably equal to or greater than 5% by mass, and is further preferably equal to or greater than 9% by mass.

On the other hand, although the upper limit is typically not limited, the upper limit may be preferably equal to or less than 70% by mass, may be more preferably equal to or less than 60% by mass, and may be further preferably equal to or less than 50% by mass. Since the non-edible part of a material typically contains a large amount of dietary fiber or insoluble dietary fiber, it is possible to provide a food from which a nutrient component of the material can be taken without any loss, by manufacturing a food including not only an edible part of the material but also the non-edible part like the food as in the present embodiment, which is more preferable. Therefore, the food in the present embodiment is a food that enables efficient intake of the nutrient component such as dietary fiber and enables reduction of the amount of waste.

The control unit 11 of the server 10 may generate advice information in accordance with user information of each user by using a learned model that has been caused to perform learning through machine learning or deep learning. In this case, it is possible to use a learned model that has been caused to perform learning to use at least a part of the user information stored in the member information DB 12b, a time zone (clock time) in which the advice has been requested, and the like as inputs, to compute optimal advice information on the basis of the input information and output the computed result (advice information). Such a learned model can be constructed through a convolution neural network (CNN), a recurrent neural network (RNN), a long short-term memory (LSTM), or the like, for example.

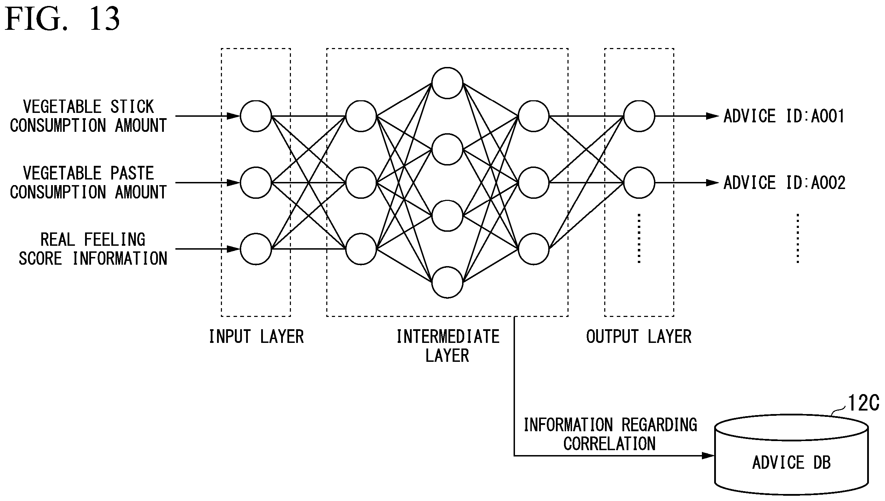

FIG. 13 is a schematic view illustrating a configuration example of the learning model. The learning model in one example illustrated in FIG. 13 is a learning model that uses food intake information and real feeling score information including one or more from among feeling information, biological information, and behavior information from among user information as inputs and that has learned to specify advice to be provided in a case where the food intake information and the real feeling score information are input. The learning model illustrated in FIG. 13 is configured of an input layer, an intermediate layer, and an output layer, the input layer includes three input nodes, and vegetable stick consumption amount, vegetable paste consumption amount, and user's real feeling score information are input as food intake information to the respective input nodes. Note that although the configuration in which the real feeling score information is input to one of the input nodes is illustrated as an example in FIG. 13, input nodes to which the respective pieces of information are input are provided in a case where a configuration in which a plurality of types of information such as feeling information, biological information, behavior information, and the like are to be input is employed. Also, each piece of information (each numerical value) to be input to the input nodes may be information per day or may be time-series information over a predetermined period of time, such as for 1 week. For example, a configuration in which a food intake history over one week, a change in real feeling score information for 1 week, or the like is input may be employed, and in this case, it is possible to specify advice in consideration of various histories and changes in the predetermined period of time.

Each piece of data input to the input node is input to the intermediate layer. The intermediate layer includes a plurality of (three in FIG. 13) fully connected layers, and a node in each layer calculates an output value based on the input data by using a weight coefficient and a function between the layers and inputs the calculated output value to a node of the following layer. The intermediate layer finally gives each output value to each output node of the output layer by successively inputting the output value of the node of each layer to the node of the following layer. The output layers include a plurality of output nodes, and each output node outputs a determination proportion to each advice stored in the advice DB 12c. For example, a first output node outputs a determination proportion for advice with an advice ID of A001 stored in the advice DB 12c, and a second output node outputs a determination proportion for advice with an advice ID of A002. The determination proportion output by each output node indicates probability at which the advice with the advice ID associated with each output node is appropriate for the data input to the input layer. The output value of each output node of the output layer is a value from 0 to 1.0, for example, and the sum of the determination proportions output from all the output nodes is 1.0. The number of input nodes in the input layer, the layer numbers of the intermediate layers, and the number of output nodes in the output layer are not limited to those in the example illustrated in FIG. 13. The learning model is not limited to a neural network (deep leaning) configured to include multiple intermediate layers as illustrated in FIG. 13, and a learning model that is constructed by various machine learning algorithms may be used. Furthermore, the learning model may be based on current or past data of assumed users who use advice, or further, the learning model may be based on data obtained from a user group arbitrarily including the current or past assumed users, or the model may be a combination thereof. Particularly, it is preferable to generate advice information by using "information regarding a correlation between intake information and real feeling score information" obtained by a learning model based on data obtained from a user group that does not include the past assumed data (which is preferably a group with the same attribute as that of the users) since it is possible to quickly provide advice to a user by using data on which analysis regarding the correlation has already been done. Also, it is preferable to generate advice information by using "information regarding a correlation between intake information and real feeling score information" obtained by a learning model based on data obtained from a learning model based on data obtained at present or in the past from a user group including the user (which is preferably a group with the same attribute as that of the user, in particular) because the self-fulfilling effect of the present invention is enhanced.

The control unit 11 may acquire output values of the intermediate layers (information regarding a correlation between user intake information and user real feeling score information or advice information including the information regarding the correlation), include the acquired output values in the advice information, and further save the acquired output values in the advice DB 12c.

Note that in a case where advice information in accordance with user information such as an age, a sex, a birthplace, philosophy and beliefs, and the like of the user is generated, it is only necessary to use the information regarding the age, the sex, the birthplace, and the philosophy and beliefs of the user as inputs and to use a learning model that has finished learning to specify advice to be specified in a case where such information is input. Also, in a case where advice information in accordance with user information such as a vegetarian degree, preference information, a thought trend, and the like of the user is generated, it is only necessary to use the information regarding a vegetarian level, preference information, and the thought trend of the user as inputs and to use a learning model that has finished learning to specify advice to be provided in a case where such information is input.

Furthermore, in a case where advice information in accordance with user information such as exercise information, sleep information, and the like of the user is generated, it is only necessary to use the information such as the exercise information and the sleep information of the user as inputs and to use a learning model that has finished learning to specify advice to be provided in a case where such information is input. It is possible to specify an optimal advice for arbitrary user information by using user information to be considered when the advice information is specified as input and causing the model to learn to specify content of advice suitable for the input user information in this manner. It is possible to generate advice in consideration of user's body condition (physical condition) and the like by specifying advice by using such a learning model. Also, in a case where learning models are used, different learning models may be used for each timing at which the advice is provided. For example, advice in accordance with the user information may be specified by using a learning model that is different for each season, a learning model that is different for each time zone, or the like. In this case, it is possible to generate an optimal advice that also takes a timing (a season, a time zone, or the like) at which the advice is to be provided into consideration.

The learning model as described above performs learning by using training data including the user information to be input and the advice ID of the advice corresponding to the user information as one set. The learning model illustrated in FIG. 13 performs learning by using training data including food intake information, real feeling score information, intake information, and an advice ID corresponding to the real feeling score information as one set. The advice used for the training data can be advice recommended by a professional such as a doctor for the corresponding user information, advice from which the user related to the user information has felt an effect, or the like. The learning model performs learning such that an output value of 1.0 is output from an output node corresponding to the advice **ID** included in the training data and an output value of 0.0 is output from the other output nodes in a case where user information (information to be input) included in the training data is input to each input node.

Note that the learning model performs learning to optimize a weight coefficient and a function to couple nodes of each of the intermediate layers. The learning processing of the learning model may be performed by the server 10 or may be performed by a different device, or learning model for which learning processing has been completed in advance may be used. In a case where the learning processing of the learning model is performed by the different device, the control unit 11 uses the learning model obtained by the different device performing learning processing to perform the aforementioned processing in the server 10. Also, the different device may acquire output values (information regarding a correlation between the user intake information and the user real feeling score information) of the intermediate layers of the learning model. In the case where the different device acquires the output values of the intermediate layers of the learning model, the control unit 11 uses the output values of the intermediate layers acquired by the different device to perform the aforementioned processing in the server 10.

In a case where the advice information in accordance with the user information is generated, the control unit 11 (output unit) transmits (outputs) an advice screen displaying the advice information to the user terminal 20 as illustrated in FIG. 12 (S45). In a case where the advice screen is received (acquired) from the server 10, the control unit 21 of the user terminal 20 displays the received advice screen on the display unit 25 (S46). The advice screen illustrated in FIG. 12 displays an advice message related to a meal, and the advice message illustrated in FIG. 12 is content generated on the basis of a correlation between content of the meal of the user (intake information) and the user real feeling score information, for example.

Note that the advice message illustrated in FIG. 12 may be generated in consideration of information regarding a user's blood glucose level (biological information) as well in addition to the correlation between the user's meal content and the user real feeling score information. The advice screen includes a save button to provide an instruction to save the displayed advice information and a cancel button to provide an instruction to end the display without saving it. In a case where the save button is operated via the input unit 24 on the advice screen, the control unit 21 stores the displayed advice information in the storage unit 22 in association with the date and time information at this point, for example. The user terminal 20 can thus accumulate the advice provided from the server 10 in association with the date and time of the provision.

Also, the control unit 21 may specify a user real feeling score on the basis of sensor information that a sensor included in a wearable terminal or the like has acquired by sensing the user, by the wearable terminal or the like that the user is wearing by using the learned model that has been caused to learn various kinds of sensor information in advance through machine learning or deep learning. In this case, it is only necessary to use a learning model that is caused to perform learning to use the sensor information as an input and output a user real feeling score including one or more pieces of information from among user feeling information, user biological information, and user behavior information for the input sensor information. Also, a learning model that has been caused to perform learning to use the sensor information and the user intake information as inputs and output the information regarding the correlation between the user intake information and the user real feeling score information for the input sensor information and user intake information may be used. Such a learned model can be constructed through CNN, for example.

Through the aforementioned processing, the server 10 can generate and provide an appropriate advice to be provided to the user on the basis of user information registered in the member information DB 12b in the information processing system 100 according to the present embodiment. Note that the advice provided by the server 10 may be content generated on the basis of at least a part of the user information registered in the member information DB 12b and may be content that is different in accordance with the advice provision timing (a clock time or a time zone). In this manner, the server 10 can provide various kinds of advice related to daily habits in user's behaviors including meals, exercise, and sleep and advice related to contribution to protection of the global environment on the basis of a correlation between the information regarding the user, such as user's attribute information, profile information, body information, exercise information, sleep information, information regarding eaten foods, and the like and the user real feeling score information. Note that it is possible to generate an appropriate advice to be provided to the user in accordance with various kinds of information related to the user by appropriately setting advice provision conditions in accordance with the content of the advice.

In the information processing system 100 according to the present embodiment, the user terminal 20 can also be configured to generate advice information in accordance with user information. For example, in a case where the user terminal 20 stores the user information and the advice DB 12c in the storage unit 22, the control unit 21 can specify content of the advice in accordance with the user information on the basis of the content stored in the advice DB 12c. Also, the control unit 21 can specify content of the advice in accordance with the user information by using the learning model even in a case where the user terminal 20 stores the learning model in the storage unit 22 in addition to the user information and the advice DB 12c. For example, in a case where high (or low) real feeling score information is exhibited in a certain group on the basis of information related to a correlation between the intake information and the real feeling score information stored in the advice DB 12c and/or information related to a correlation obtained by analyzing the intake information and the real feeling score information at present or in the past in a group arbitrarily including an assumed user who uses the advice, it is possible to specify one or more pieces of intake information (intake forms of types, amounts, intake timings, seasons, eating combinations, cooking methods, contained components, and the like of foods) as factors of the real feeling score information and to generate or specify and provide advice including the information related to the correlation to a user for which effectiveness or relevance is presumed from the correlation, such as a user with similar intake information or a user for which an improvement in the real feeling score information is required.

In the information processing system 100 according to the present embodiment, the server 10 is not limited to the configuration in which the server 10 generates and provides advice to be provided to the user in response to an advice request from the user terminal 20. For example, the server 10 may generate and provide the advice to be provided to the user periodically or in a case where a preset clock time arrives. In the case of such a configuration, the control unit 11 of the server 10, for example, generates advice information to be provided to each user and stores the advice information in an association with the member ID of each user in the storage unit 12 on the basis of the user information of each user registered in the member information DB 12b in a case where a predetermined time has elapsed or in a case where a setting clock time has arrived. Note that the control unit 11 may store the date and time when the advice information has been generated in the storage unit 12 in addition to the member ID and the advice information.

On the other hand, the user terminal 20 may be configured to access the server 10 when the advice APP 22AP is activated, acquire advice information, and display the acquired advice information on a home screen (activation screen). In the case where the advice information is displayed on the home screen, the user can check the advice on the home screen merely by causing the user terminal 20 to activate the advice APP 22AP. Note that the server 10 may provide a push notification of the generated advice information to the user terminal 20 instead of storing the advice information in the storage unit 12 in a case where the advice information to be provided to each user is generated. In this case, the user terminal 20 can display that the new advice information has been received on the display unit 25 and can thus notify the user of the fact that there is new advice information.

In the information processing system 100 according to the present embodiment, the user terminal 20 may be configured to receive user's evaluation on the content of the advice acquired from the server 10. With such a configuration, it is possible to feed back the user's evaluation to the content of the advice and to prepare advice that is effective for the user by updating the content of the advice with low evaluation. For example, an evaluation button (like button) for inputting that the display advice has been useful may be provided on the screen illustrated in FIG. 12, and advice with content to be updated may be picked up in accordance with content of evaluation through the evaluation button. Also, evaluation for the advice may be received by five-level evaluation, for example, and content of advice with low evaluation may be updated. Note that content of the evaluation that the user has conducted on the advice may be transmitted to the server 10 and may be stored in the member information DB 12b.

In the information processing system 100 according to the present embodiment, the advice to be provided to the user of the user terminal 20 is not limited to the configuration in which the advice is generated by the server 10, and the advice may be generated by the user terminal 20 and may be displayed on the display unit 25, for example. In this case, it is possible to employ a configuration in which the user terminal 20 stores various kinds of user information input via the input unit 24 and the advice DB 12d in the storage unit 22 and the control unit 21 (information processing unit) generates advice information in accordance with at least a part of the user information stored in the storage unit 22 on the basis of the content stored in the advice DB 12d.

### (Second embodiment)

In an information processing system 100 according to the present embodiment, a user terminal 20 performs not only processing of receiving intake information of foods that a user has eaten by an input from an input unit 24 but also processing of receiving intake information by imaging the foods that the user has eaten by a camera 27. Also, in a case where the server 10 acquires the captured image of the foods that the user has eaten form the user terminal 20, the server 10 performs processing of specifying information regarding the foods that the user has eaten or information of nutrient components taken through the foods that the user has eaten by analyzing the captured image. Note that a configuration in which the processing of specifying the information regarding the foods that the user has eaten or the information regarding the taken nutrient components form the captured image is performed by the user terminal 20 may be employed instead of the configuration in which the server 10 performs the processing. In this case, the user terminal 20 specifies the information of the foods that the user has eaten or the information of the taken nutrient components by analyzing the image captured by the camera 27 and transmits the specified intake information to the server 10.

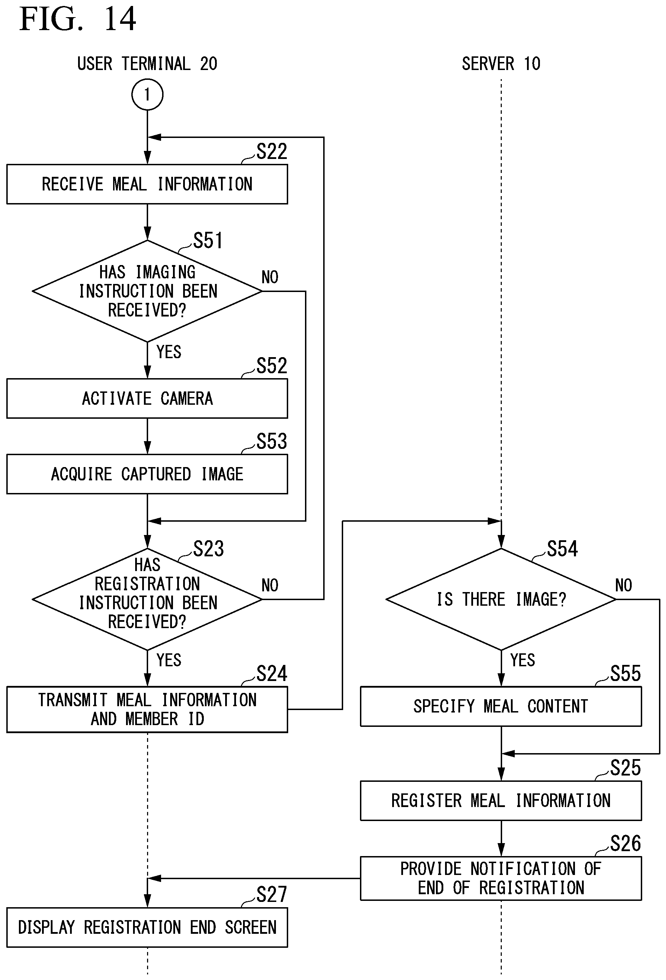

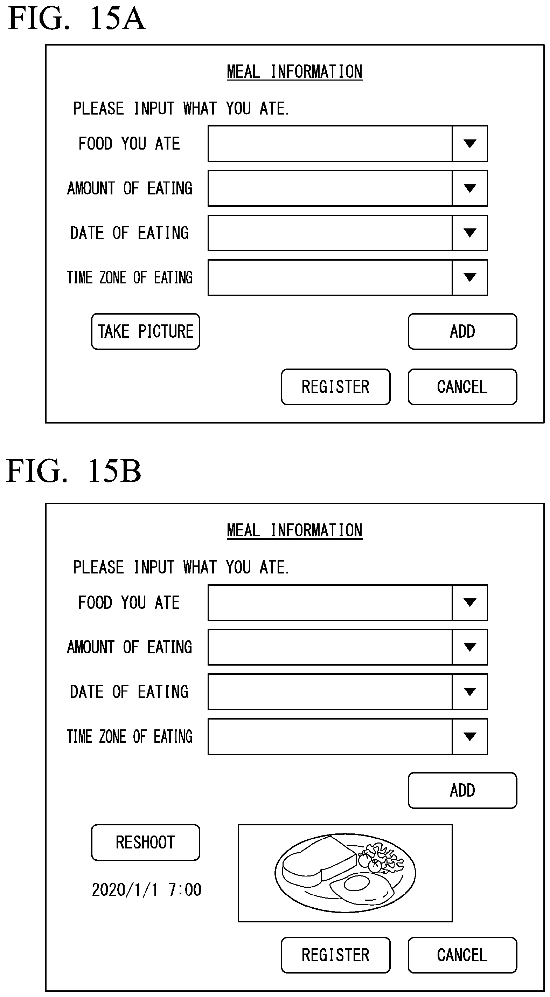

FIG. 14 is a flowchart illustrating an example of user information registration processing procedure according to the second embodiment, and FIGS. 15A and 15B are schematic view illustrating screen examples of the user terminal 20. The processing illustrated in FIG. 14 is obtained by adding Steps S51 to S53 between Steps S22 and S23 and adding Steps S54 and S55 before Step S25 in the processing illustrated in FIGS. 6 and 7. Description of the same steps as those in FIGS. 6 and 7 will be omitted. Also, illustration of each step in FIG. 6 and Steps S28 to S35 in FIG. 7 is omitted in FIG. 14.

In the information processing system 100 according to the present embodiment, the user terminal 20 and the server 10 performs processing similar to those in Steps S11 to S22 in FIGS. 6 and 7. In this manner, in a case where user information (profile information, body information, exercise information, and sleep information) input via the input screens in FIGS. 8B to 9B is received, the user terminal 20 transmits the received information to the server 10, and the server 10 registers the user information received from the user terminal 20 in the member information DB 12b.

The user terminal 20 in the present embodiment displays a meal information input screen as illustrated in FIG. 15A on the display unit 25 in Step S21. The meal information input screen illustrated in FIG. 15A has a configuration that is similar to that of the input screen illustrated in FIG. 10A and further has a photo imaging button for imaging meals and foods that the user has eaten with a camera 27. Note that the input screen illustrated in FIG. 15A may have an input section for inputting, as text data, for example, content of meals that the user has eaten or content of nutrient components that the user has taken through the eaten meals. In this case, the control unit 21 can receive the content of meals that the user has eaten or the content of the taken nutrient components via the input section.

The user inputs, to each input section, each piece of information such as foods that the user himself/herself has eaten, the dates on which the user has eaten them, and time zones in which the user has eaten them on the input screen illustrated in FIG. 15A. In this manner, the control unit 21 of the user terminal 20 receives food intake information (meal information) input by the user (S22). Note that the control unit 21 displays each piece of received information in each corresponding input section. Also, in a case where the user desires to image general foods (meals) that the user himself/herself has eaten with the camera 27, the user performs an operation of selecting the photo imaging button via the input unit 24. The control unit 21 determines whether an instruction to image the meal that the user has eaten with the camera 27 (imaging instruction) has been received in accordance with whether the photo imaging button has been operated via the input unit 24 (S51). In a case where it is determined that the imaging instruction has not been received (S51: NO), the control unit 21 moves on to the processing in Step S23. In a case where it is determined that the imaging instruction has been received (S51: YES), the control unit 21 activates the camera 27 (S52), performs imaging in response to the imaging instruction via the input unit 24, and acquires a captured image (S53).

The control unit 21 displays the captured image on the meal information input screen as illustrated in FIG. 15B, for example, in a case where the captured image of the meal has been acquired. A "reshoot" button for retrying imaging of the meal is displayed on the input screen illustrated in FIG. 15B. In a case where the "reshoot" button is imaged, the control unit 21 activates the camera 27 again, acquires a captured image again in response to the imaging instruction via the input unit 24, and displays the acquired captured image on the meal information input screen. In a case where a registration button has been operated on the input screen illustrated in FIG. 15B, that is, in a case where the control unit 21 has received a registration instruction (S23: YES), the control unit 21 transmits the meal information (the intake information and the captured image input via the input section) input via the input screen and the member ID of the user in an associated manner to the server 10 (S24).

In a case where the meal information is acquired from the user terminal 20, the control unit 11 of the server 10 in the present embodiment determines whether or not the captured image is included therein (S54). In a case where it is determined that the captured image is included (S54: YES), the control unit 11 specifies content of the meal that the user has eaten by analyzing the captured image (S55). The control unit 11 specifies foods, materials, and types of cooked foods that appear in the captured image through template matching, for example. In this case, templates indicating image feature amounts extracted from captured images capturing foods, materials, and cooked foods are stored in advance in the storage unit 12, and in a case where a region that coincides with some template is detected in a captured image, the control unit 11 specifies that the detected region is a region of the food, the material, or the cooked food corresponding to the template. The control unit 11 specifies all foods, materials, and cooked foods included in the captured image. Note that the control unit 11 may be configured to specify the types of nutrient components taken by eating the foods, materials, and cooked foods included in the captured image and intake amounts thereof instead of specifying the foods, material, and cooked foods included in the captured image.

Also, the control unit 11 may specify the foods, the materials, and the types of cooked foods in the captured image by using a learned model that have been caused to learn various foods, materials, cooked foods, and the like in advance through machine learning or deep learning. In this case, it is only necessary to use the captured image as an input and to use the learning model that has been caused to learn to output the foods, the materials, or the types of cooked foods and amounts thereof in the input captured image. Note that the learning model may be a model that has been caused to learn to output the types of nutrient components taken by eating the foods, the materials, and the cooked foods appearing in the captured image and the intake amounts in a case where the captured image is input. Such a learned model can be constructed through CNN, for example.

In a case where it is determined that no captured image is included in the meal information acquired from the user terminal 20 (S54: NO), the control unit 11 skips the processing in Step S55. Thereafter, the control unit 11 registers, in the member information DB 12b, the meal information acquired from the user terminal 20 and meal information specified from the captured image (S25). In this manner, not only the intake information input via the input section on the input screen of the user terminal 20 but also the content of the meal (intake information) captured by the camera 27 of the user terminal 20 are registered in the member information DB 12b. Thereafter, the control unit 11 of the server 10 and the control unit 21 of the user terminal 20 execute processing in and after Step S26.

Through the aforementioned processing, the user terminal 20 can transmit the image capturing the meal that the user has eaten to the server 10, and the server 10 can acquire content of the meal (foods, materials, cooked foods) appearing in the captured image received from the user terminal 20. Therefore, user's attribute information, profile information, information regarding a body condition of the user (biological information), information regarding exercise, information regarding sleep, information regarding types of foods that the user has eaten, intake amounts, and intake timings, and the like (user information) are input by using the user terminal 20 and can be registered in the server 10 in the present embodiment as well. Also, in a case where the user images the meal immediately before eating, it is possible to use the imaging date and time as the meal intake date and time and to thereby automatically acquire the meal intake timing (intake date and time) from the imaging date and time.

The user terminal 20 and the server 10 in the present embodiment can perform processing that is similar to the processing illustrated in FIG. 11. Therefore, advice information in accordance with the user information registered in the server 10 is provided to the user terminal 20 by the user terminal 20 and the server 10 performs the similar processing.

According to the present embodiment, effects that are similar to those of the aforementioned first embodiment are obtained. Also, according to the present embodiment, the user can input information regarding meals that the user himself/herself has eaten not only by inputting information regarding the meals (general foods) that the user himself/herself has eaten via the input unit 24 but also capturing images by the camera 27 in the user terminal 20. Also, according to the present embodiment, it is possible to provide advice in accordance with intake information of the general foods that the user has eaten. It is possible to appropriately apply the modification examples described in the aforementioned first embodiment to the information processing system 100 according to the present embodiment as well.

### (Third embodiment)

An information processing system that proposes intake of a food for a user on the basis of a target intake amount of a user for a predetermined nutrient component or a target amount of contribution of the user to a global environment will be described. Although a food to be proposed for the user is specified on the basis of a target intake amount or a target amount of contribution in a predetermined period of time, such as for 1 month, for example, in the present embodiment, the predetermined period of time is not limited to 1 month. In a case where the target value in the present invention is a target that is difficult to be achieved, it is preferable to employ a food that is suitable to achieve the target and a combination thereof and to set a plurality of intermediate targets in order to enable the target of the user to be achieved in the present invention. Particularly, there is a problem that although users get bored of foods and find difficult in continuing to eat them if variations are not secured due to the characteristic that eating is intermittently performed every day, known foods (supplements, for example) that highly contribute to a target that is difficult to be achieved generally have poor taste variations and menus using the foods tend to be similar.

However, the present invention can provide an achievement method that can employ foods that are highly related to foods in which consumers are interested and that highly contribute to their targets, that the consumers are unlikely to be bored of, and that is sustainable. Particularly, it is possible to propose a sustainable achievement method while securing variety by outputting the achievement method including cooking methods and intake methods by mainly employing foods that are used as staple foods, such as noodles, breads, imitation rice, and the like and that highly contribute to the targets, specifically staple foods which are bean processed food, which is more preferable. Since the information processing system according to the present embodiment can be realized by a device that is similar to that of the information processing system 100 according to the first embodiment, description regarding the configuration will be omitted. Note that the storage unit 12 of the server 10 in the present embodiment stores a target amount DB 12d in addition to the DBs 12a to 12d illustrated in FIGS. 3A to 5.

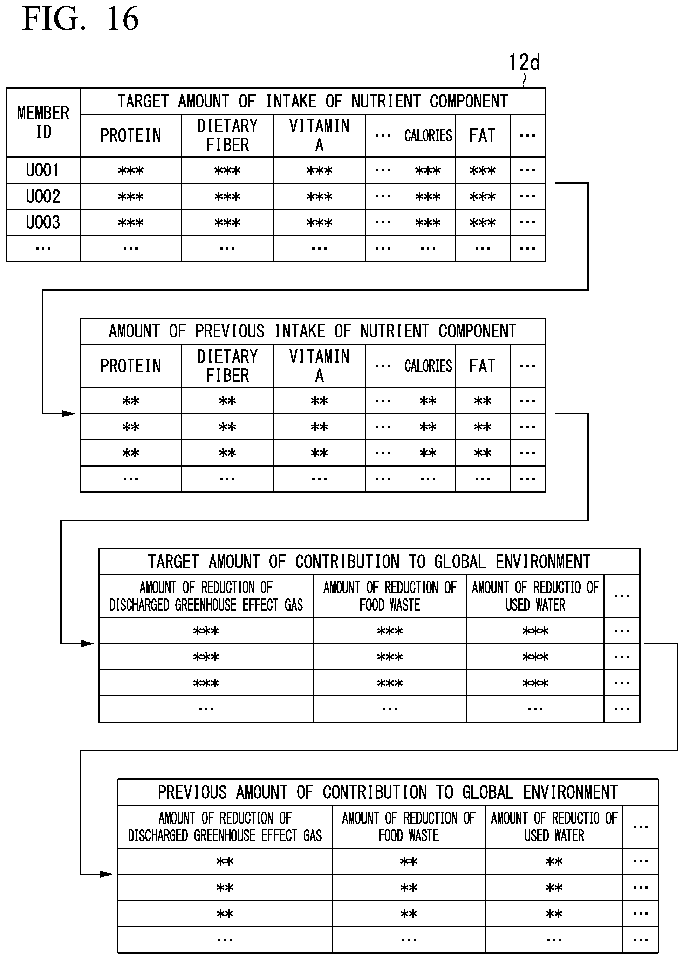

FIG. 16 is a schematic view illustrating a configuration example of the target amount DB 12d. The target amount DB 12d stores a target intake amount of a predetermined nutrient component for a predetermined period of time and an amount by which the user has already taken it and the target amount of contribution of the user to the global environment for a predetermined period of time and the previous contribution amount. The target amount DB 12d illustrated in FIG. 16 includes a member ID column, a target intake amount and a previous intake amount column of a nutrient component, a target global environment contribution amount column and a previous contribution amount column, and the like and stores the target amounts (the target intake amount and the target amount of contribution) that each user has set and the amounts of achievement with respect to the target amounts (the previous intake amount and the previous contribution amount) in association with the member ID.

The member ID column stores the member IDs of the users who have been registered as members. The target nutrient component intake amount column stores the target intake amount and the target intake calories that the user has set for a predetermined nutrient component. As the predetermined nutrient component, it is possible to use, for example, a nutrient component, the shortage of which affects maintaining and promoting of health and a nutrient component, excessive intake of which affects maintaining and promoting of health. As examples of the "nutrient component, shortage of which affects maintaining and promoting of health" in the present embodiment (or the "nutrient component, excessive intake of which affects maintaining and promoting of health"), it is possible to list the nutrient components corresponding to "display indicating that the nutrient component can be replenished (emphasizing that the amount of the nutrient component is large)" (or "display indicating that the nutrient component can be appropriately taken (emphasizing that the amount or the calories of the nutrient component is small)" from among the nutrient components defined as "the nutrient components and the like, shortage and excessive intake of which affect maintaining and promoting of Japanese health" in the nutrient component display handbook (issued by Food Safety Division, Bureau of Public Health and Medical Care, Tokyo Metropolitan Government Bureau of Social Welfare in August, 2019).

Specifically, "the nutrient components, the shortage of which affects maintaining and promoting of health" include protein, dietary fiber, zinc, potassium, calcium, iron, copper, magnesium, niacin, a pantothenic acid, biotin, vitamin A, vitamin B1, vitamin B2, vitamin B6, vitamin B12, vitamin C, vitamin D, vitamin E, vitamin K, a folic acid, and the like.

Also, "the nutrient components, the excessive of which affects maintaining and promoting of healthy" include calories, fat, a saturated fatty acid, a trans fatty acid, cholesterol, sugars, sodium, and the like. Therefore, the target nutrient component intake amount column stores the target intake amount set by the user for at least one of the nutrient components as described above, and the nutrient component previous intake amount column stores the amount of component that the user has already taken for each of the aforementioned nutrient components.

The target global environment contribution amount column stores the target amount of contribution that the user has set for contribution to the global environment. The global environment in the present invention refers to all external matters such as home, a society, nature, and the like surrounding people and creatures on the Earth, is an environment concept of a broad meaning including not only natural environments such as an ocean environment and atmospheric environment but also a social environment, and has a similar meaning in the present embodiment as well. More specifically, representative examples of targets for the global environment in the present invention include any one or a plurality items from among 17 goals, 169 targets, and 232 indexes in sustainable development goals (SDGs) which are international goals to realize a sustainable and better world by 2030 described in "2030 Agenda for Sustainable Development" adopted in the UN summit in September 2015. More specifically, items such as the amount of reduction of the amount of discharged greenhouse effect gas (the amount of reduction of carbon dioxide, in particular, or further, the amount of reduction of carbon footprint), the amount of reduction of the amount of discarded industrial waste (an improvement in a zero emission rate, a recycle rate, or a recycle rate), the amount of reduction of food waste (the amount of reduction of waste, in particular, and further, the amount of reduction of the amount of discarded food non-edible parts), effective utilization of water resources (the amount of reduction of the amount of used water or the amount of reduction of amount of discharged water), and the like are listed, these targets can be used as target items of contribution to the global environment in the present embodiment as well, and it is possible to employ the target value of the item in regard to the target numerical value for each target item.

Therefore, a target contribution amount set by the user for at least one of the aforementioned items is stored in the target global environment contribution amount column, and the amount of contribution (the amount of reduction) by which the user has already contributed to each of the aforementioned items is stored in the global environment previous contribution amount column. As for the member ID to be stored in the target amount DB 12d, a registered member ID is stored by the control unit 11 in a case where a new member is registered in the member information DB 12b. The target amount (the target intake amount and the target amount of contribution) to be stored in the target amount DB 12d is stored in the control unit 11 in a case where the control unit 11 acquires each target amount via the communication unit 13. As for the amount of achievement (the amount of previous intake and the amount of previous contribution) to be stored in the target amount DB 12d, in a case where the control unit 11 acquires information regarding a food (general food) that the user has taken via the communication unit 13, the control unit 11 specifies and stores the amount of achievement by the food that the user has taken. The details stored in the target amount DB 12d is not limited to the example illustrated in FIG. 16, and items related to the nutrient component and the contribution to the global environment to be stored in the target amount DB 12d are not limited to the aforementioned examples.

In the information processing system 100 according to the present embodiment, the user terminal 20 and the server 10 perform user information registration processing that is similar to the processing illustrated in FIGS. 6 and 7. In this manner, the user terminal 20 can receive user information such as attribute information, profile information, biological information, exercise information, sleep information, intake information (meal information), real feeling information, and the like of the user, and the server 10 can register the user information input via the user terminal 20 in the member information DB 12b. Note that in the present embodiment, the user terminal 20 performs processing of receiving a user's target intake amount of the nutrient component and the user's target amount of contribution to the global environment in addition to the aforementioned user information and registering them in the server 10 (target amount DB 12d). Here, the user's target intake amount of the nutrient component is information (shared information) that is shared by other users. Therefore, in the present embodiment, the user information input screen (registration screen) is slightly different from the configuration in the first embodiment illustrated in FIG. 8A, and the user information registration processing is slightly different from the processing illustrated in FIGS. 6 and 7.

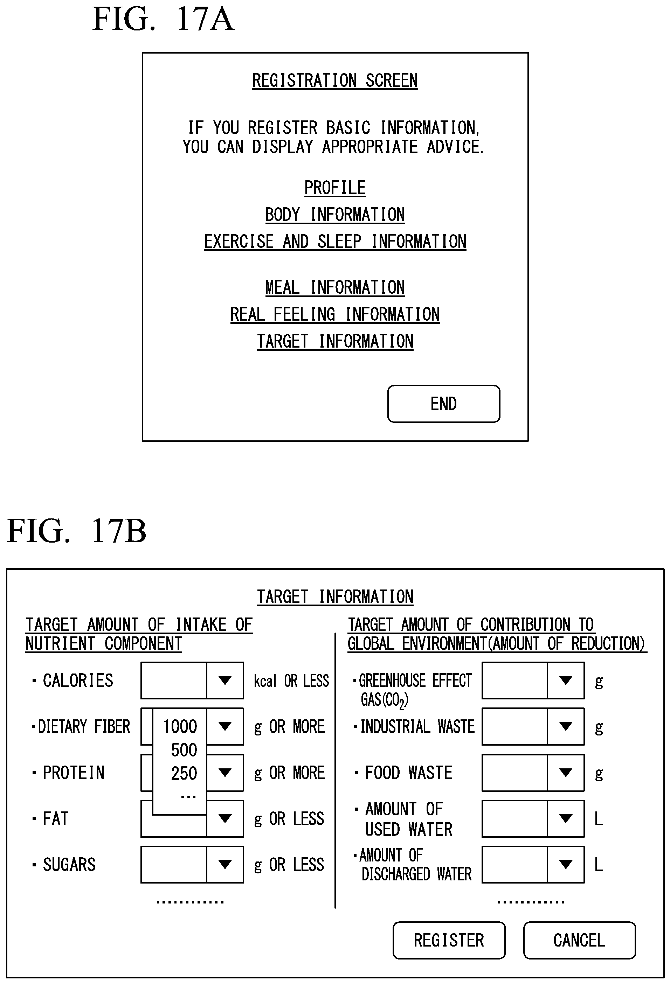

FIG. 17A is a schematic view illustrating a registration screen example in the third embodiment, and FIG. 17B is a schematic view illustrating a target information input screen example. The registration screen in the present embodiment is configured such that user's target information can be selected as user information to be input (registered) in addition to the configuration illustrated in FIG. 8A. In a case where target information is selected on the screen illustrated in FIG. 17A, the control unit 21 of the user terminal 20 displays the target information input screen as illustrated in FIG. 17B on the display unit 25. The target information input screen illustrated in FIG. 17B has an input section for inputting a preset target intake amount for a nutrient component and an input section for inputting a preset target amount of contribution to the global environment. In each of the input section for the target intake amount and the input section for the target amount of contribution, a pull-down menu that allows any of numerical values (amounts) set for nutrient component or an item of contribution to the global environment in advance to be selected is provided. Note that each selectable numerical value may be determined from eating habits, daily habits, or the like of the user, for example. Alternatively, it is also possible to select a food and to make determination from nutrient components contained in the food.

Also, the input section for the target intake amount and the input section for the target amount of contribution may be configured such that arbitrary numerical values can be input. Note that registered content may be displayed on the target information input screen for target amounts that the user has already registered. In the case of such a configuration, the user can check the target amount that has already been registered (or can check a registration history in a time-series manner) and can add a change in a case where the user desires to add a change. Alternatively, it is possible to select a setting period of time in which the target amount is to be achieved and to select a plurality of target amounts and setting periods of time. Specifically, it is possible to set a target intake amount per day, and to perform setting for breakfast, lunch, dinner, snack, and the like, or to perform setting for each time zone. Alternatively, it is possible to set a target intake amount per week for each day, and for the setting for each day, it is also possible to perform repeated setting of the target intake amount per day. Moreover, it is also possible to perform individual setting such as setting for special days in conjunction with a user's schedule table or the like.

On the target information input screen illustrated in FIG. 17B, "or more" or "or less" is displayed in association with the input section for the target intake amount of the nutrient component. Alternatively, it is also possible to set a target intake amount achievement period of time, to set a plurality of above setting periods of time, and to select it in a stepwise manner, for example. Specifically, "or less" is displayed for calories, fats. and sugars, and "or more" is displayed for dietary fiber and protein. In this manner, for the nutrient component, shortage of which affects maintaining and promoting of health, such as dietary fiber or protein, a lower limit value is input to the input section, and a value that is equal to or greater than the input value is received as a target intake amount. On the other hand, for a nutrient component, excessive intake of which affects maintaining and promoting health, such as calories, fats, or sugars, an upper value is input to the input section, and a value that is equal to or less than the input value is received as the target intake amount. Note that "or more" or "or less" for each nutrient component may be able to be changed. Also, for the item of contribution to the global environment, a lower limit is input to the input section, and a value that is equal to or greater than the input value is received as the target amount of contribution.

The user selects a nutrient component or an item of contribution to the global environment for which the user himself/herself desires to set a target and inputs a target amount to the corresponding input section on the input screen illustrated in FIG. 17B. In a case where a registration button is operated on the target information input screen, the control unit 21 of the user terminal 20 transmits the target information (the target intake amount of the nutrient component or the target amount of contribution for the item of contribution to the global environment) input via the input screen and the member ID of the user in an associated manner to the server 10 and provides an instruction to register the target information to the server 10. The control unit 11 (the target intake amount acquisition unit, the target contribution amount acquisition unit) of the server 10 acquires the target information from the user terminal 20 and stores the acquired target information in association with the member ID in the target amount DB 12d (the target intake amount storage unit, the target contribution amount storage unit). In this manner, the target intake amount for the nutrient component or the target amount of contribution for the item of contribution to the global environment input by the user via the user terminal 20 is registered in the server 10 (target amount DB 12d).

Also, in the present embodiment, the server 10 performs processing of specifying the amount of intake of each nutrient component (the amount by which the nutrient component has already been taken) and the amount of contribution to the global environment (the amount by which contribution has already been made) due to the food taken by the user and registering the specified intake amount and contribution amount in the target amount DB 12d in a case where intake information (meal information is received from the user terminal 20. Specifically, after the processing of Step S25 in FIG. 7, the control unit 11 of the server 10 specifies the amount of nutrient component that the user has taken by a meal and the amount of contribution to the global environment (amount of achievement) on the basis of information regarding the meal acquired from the user terminal 20. The amount of nutrient component and the amount of contribution to the global environment (the amount of achievement) that can be achieved in a case where a food (general food) is eaten, for example, are registered in advance in the storage unit 12 or a different server. Then, the control unit 11 extracts foods contained in the meal content of the user and acquires the amount of nutrient component and the amount of contribution to the global environment corresponding to each of the foods from the storage unit 12 or the different server. The control unit 11 stores the acquired amount of achievement in association with the member ID in the target amount DB 12d. Note that the control unit 11 adds the acquired amount of achievement to the amount that has already been taken and the amount by which contribution has been made, which are stored in the target amount DB 12f, and stores the result in the target amount DB 12f. It is thus possible to sequentially update and store the nutrient component amount and the amount of contribution to the global environment achieved by the user in a predetermined period of time (for one month, for example).

As described above, the target intake amount for the nutrient component and the target amount of contribution to the global environment are set in the server 10 for each user in the present embodiment. Also, the amount of taken nutrient component and the amount of contribution to the global environment achieved by the user through a meal are accumulated in the server 10 every time the intake information (meal information) of the user is transmitted from the user terminal 20 to the server 10. In the aforementioned configuration, in a case where the user selects a food displayed on a website while viewing the website provided by the server 10, for example, the server 10 according to the present embodiment performs processing of proposing (giving advice for) a food that is more suitable for the user on the basis of the selected food and the target amount set by the user.

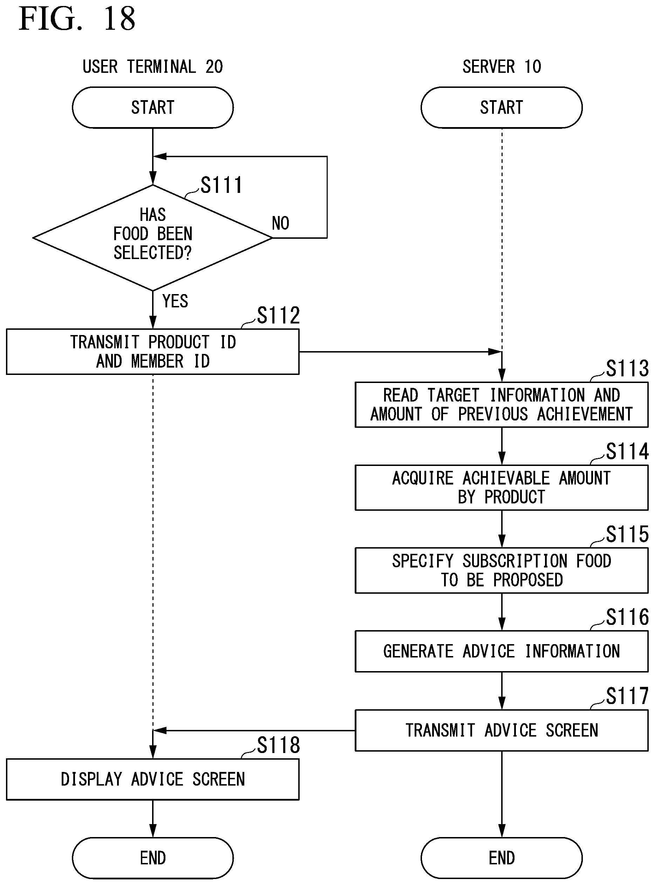

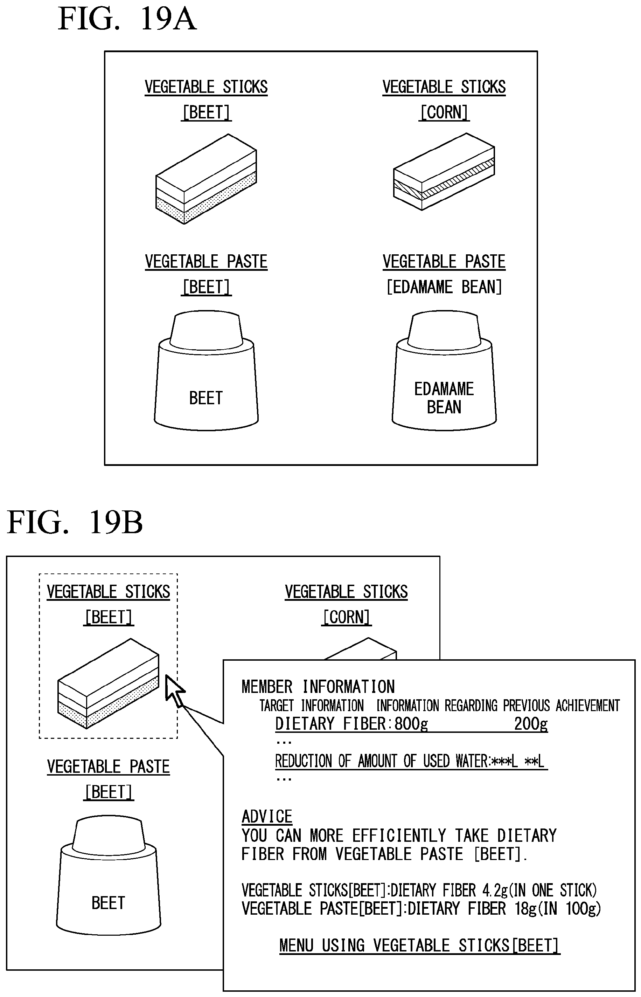

FIG. 18 is a flowchart illustrating an example of advice providing processing procedure according to the third embodiment, and FIGS. 19A and 19B are schematic views illustrating screen examples of the user terminal 20. In FIG. 18, processing performed by the user terminal 20 is illustrated on the left side, and processing performed by the server 10 is illustrated on the right side. The following processing is executed by the control unit 21 in accordance with the control program 22P and the advice APP 22AP stored in the storage unit 22 of the user terminal 20 and is executed by the control unit 11 in accordance with the control program 12P stored in the storage unit 12 of the server 10. A part of the following processing may be realized by a dedicated hardware circuit.

In the information processing system 100 according to the present embodiment, the user selects, through an input unit 24, a food that the user is interested in from among foods displayed on a website while viewing the website released by the server 10 via the network N, for example, by using the user terminal 20. As illustrated in FIG. 19A, for example, the user selects an arbitrary food in the website on which a plurality of kinds of foods are displayed. The control unit 21 of the user terminal 20 determines whether or not any of the foods has been selected via the website that is being displayed (S111), and in a case where no food has been selected (S111: NO), the control unit 21 waits while performing different processing. In a case where it is determined that any of the foods has been selected (S111: YES), the control unit 21 transmits the product ID of the selected food and the member ID of the user to the server 10 (S112). Note that the product ID is stored in the website, for example, and the member ID is set in the advice APP 22AP, for example.

The control unit 11 of the server 10 reads the target information (the target intake amount of the nutrient component and the target amount of contribution to the global environment) stored in the target amount DB 12f in association with the member ID and the amount of previous achievement (the amount by which the nutrient component has already been taken and the amount by which the global environment has been contributed to) in a case where the control unit 11 receives a product ID and a member ID from the user terminal 20 (S113). Note that it is only necessary for the control unit 11 to read the target amount and the amount of previous achievement for the nutrient component and the item of contribution to the global environment, for which the target amount (the target intake amount or the target amount of contribution) has been registered. Next, the control unit 11 acquires the amount of nutrient component and the amount of contribution to the global environment (the amount of possible achievement) that can be achieved by eating the food selected by the user (S114). The amount of nutrient component and the amount of contribution to the global environment that can be achieved by eating each food are stored in advance in the product information DB 12a, for example, and the control unit 11 reads the amount of possible achievement corresponding to the product ID from the product information DB 12a. Note that the amount of possible achievement corresponding to each food may be stored in a different server, and in this case, the control unit 11 may acquire the amount of possible achievement corresponding to each food from the different server.

Next, the control unit 11 specifies a food to be proposed for the user on the basis of the target information and the amount of previous achievement read in Step S113 and the amount of possible achievement acquired in Step S114 (S115). For example, the control unit 11 (the achieved intake amount acquisition unit, the achieved contribution amount acquisition unit) acquires the amount of nutrient component and the amount of contribution to the global environment (possible achievement amount) that can be achieved by eating for each of foods as sales targets. The control unit 11 acquires the amount of possible achievement corresponding to each food from the product information DB 12a or another server. Then, the control unit 11 calculates a remaining target intake amount by subtracting the amount by which the user has already taken the nutrient component from the user's target intake amount for the nutrient component and specifies a food to be taken to achieve (realize) the remaining target intake amount.

In regard to a nutrient component for which a value that is equal to or greater than a predetermined amount has been set as a target intake amount, for example, the control unit 11 extracts a food containing a larger amount of achievable nutrient component than the food selected by the user from among foods as sales targets and specifies a food to be proposed. Here, in a case where the remaining target intake amount is greater than the amount of nutrient component that can be achieved by the food selected by the user, the food to be proposed may be specified. Also, for the nutrient component for which a value that is equal to or less than a predetermined amount has been set as a target intake amount, the control unit 11 extracts a food that contains a smaller amount of achievable nutrient component than the food selected by the user from among the foods as sales targets and specifies the food as a food to be proposed. Here, in a case where the remaining target intake amount is smaller than the amount of nutrient component that can be achieved by the food selected by the user, the food to be proposed may be specified.

Similarly, the control unit 11 calculates a remaining target amount of contribution by subtracting the amount by which the user has already contributed from the user's target amount of contribution to the global environment and specifies a food to be taken to achieve (realize) the remaining target amount of contribution. For example, the control unit 11 extracts a food with a larger amount of achievable contribution than that of the food selected by the user from among the foods as sales targets and specifies a food to be proposed. In a case where the remaining target intake amount is larger than the amount of contribution that can be achieved by the food selected by the user, the food to be proposed may be specified in this case as well. Note that in a case where a target amount has been set for an item of contribution to a plurality of nutrient components or a plurality of global environments, the control unit 11 specifies a food by which it is possible to further approach each target amount as a food to be proposed. Also, the control unit 11 may specify a food suitable for a taste of the user as a food to be proposed in consideration of the preference information (a taste of meals) of the user as well.

Also, in a case where a food in the present embodiment contains "a nutrient component, shortage of which affects maintaining and promoting of health" in content in a level in which it is possible to display "high", "contained", or "enhanced" in an emphasized manner and/or in a case where the food contains "a nutrient component, excessive intake of which affects maintaining and promoting of health" in content in a level in which it is possible to display "low", "not contained", or "reduced" in an emphasized manner, it is possible to specify a food from which an appropriate amount of nutrient component with high user acceptability can be taken. In a case where a high target intake amount has been set for protein and/or dietary fiber and a low target intake amount has been set for calories, it is preferable to specify foods that contain protein and/or dietary fiber in content in a level in which it is possible to display "high", "contained", or "enhanced" in an emphasized manner (more specifically, a food that contains 8% by mass or more dietary fiber is preferable, a food with a dietary fiber content proportion of 0.15 or more with respect to sugars is further preferable, and a food containing a bean processed product as a main ingredient (noodles, breads, or imitation rice used as a staple food, in particular) is preferable) and to specify foods that contain calories in content in a level in which it is possible to display "low", "not contained", or "reduced" in an emphasized manner.

The control unit 11 (generation unit) generates advice information as illustrated in FIG. 19B on the basis of the foods specified in Step S115 (S116). FIG. 19B illustrates an example of advice screen displayed in a case where vegetable sticks [beet] have been selected on the screen illustrated in FIG. 19A. On the advice screen illustrated in FIG. 19B, a target intake amount of dietary fiber and a target reduction amount for an amount of used water as target information that the user has set and an amount of taken dietary fiber and a reduction amount of an amount of used water as information regarding details that have already been achieved are displayed. Also, a message to propose vegetable paste [beet] from which it is possible to more efficiently take dietary fiber than the selected vegetable sticks [beet] is displayed, and information of comparing the amount of dietary fiber from the vegetable sticks [beet] selected by the user and the amount of dietary fiber from the proposed vegetable paste [beet] is displayed, on the screen illustrated in FIG. 19B. Note that in a case where the amount of contribution to the global environment of the proposed food is larger than that of the food selected by the user, a message to propose another food with a larger amount of contribution to the global environment than the selected food is displayed on the screen illustrated in FIG. 19B. At that time, information of comparing the amount of contribution to the global environment of the food selected by the user with the amount of contribution to the global environment of the proposed food may be displayed. Also, a link to display a menu (an intake method and a cooking method) using the proposed food is set on the screen illustrated in FIG. 19B, and the menu using the proposed food is provided via the link. Note that a menu using the proposed food may be displayed on the screen illustrated in FIG. 19B.

The control unit 11 transmits advice screen on which generated advice information is displayed to the user terminal 20 (S117). The control unit 21 of the user terminal 20 receives the advice screen from the server 10 and displays the advice screen as illustrated in FIG. 19B on the display unit 25 (S118). As illustrated in FIG. 19B, the user terminal 20 displays the advice screen received from the server 10 in a superimposed manner on the display screen illustrated in FIG. 19A although the invention is not limited to such a configuration. In a case where the user selects an arbitrary food while viewing a website by using the user terminal 20 through the aforementioned processing, the server 10 can specify a food by which the target information of the user can be further achieved and provide advice to the user. In this manner, the user can receive provision of the advice to achieve the target that the user himself/herself has set.

In the present embodiment, same effects as those in each of the aforementioned embodiments are obtained. Also, in the present embodiment, it is possible to receive advice to achieve target information of the user in a case where the user selects an arbitrary food via a viewing image. At that time, it is possible to provide an amount by which the user target can be achieved for the food selected by the user and a proposed food. Therefore, the user can take an achievable level of his/her target into consideration when the user considers purchase and intake of a food. Furthermore, the user can check the achievable level in an achievement plan in an arbitrary period of time that the user sets and receive and consider advice for realizing the achievement plan. The configuration of the present embodiment can also be applied to the information processing system 100 in the second embodiment as well, and similar effects can be obtained even in the case where the configuration is applied to the information processing system 100 in the second embodiment. Also, it is possible to apply modifications appropriately described in each of the aforementioned embodiments to the information processing system 100 in the present embodiment.

The present embodiment is not limited to the configuration in which the user himself/herself sets target information. For example, target intake amount for each nutrient component and a target amount of contribution for each item of contribution to the global environment may be registered in advance in the storage unit 12 or another server in accordance with information regarding an age, a sex, a birthplace (a nation or a region), a religion, a vegetarian level, a taste trend regarding meals (preference information), and the like. In this case, the control unit 11 of the server 10 may acquire the target amounts in accordance with the aforementioned information of the user from the storage unit 12 or another server and set the target amounts in the target information of the user. Also, the control unit 11 may acquire and set the target information in accordance with the user from a web server that releases such information.

For example, it is possible to use, as a target intake amount for dietary fiber, an intake amount (median) described in "Table 2 Values (g/day) referred to for calculating a target amount of dietary fiber" in "1-4. Carbohydrate, 4. Dietary fiber" in "II Item-by-item discussion, 1. Energy and nutrients" in "Dietary Reference Intakes for Japanese

(2020)". Also, target amounts in accordance with the birthplace or residence of the user may be acquired from reference intakes of each nutrient component released in each nation and may be used as the target information. In a case of such a configuration, it is possible to automatically set the target amounts in accordance with the situation of the user.

Also, it is possible to automatically set targets amount in accordance with users eating habits or daily habits, such as a case where the user is going on diet. Specifically, the reference intakes in accordance with the birth nation or residence associated with the user ID are selected by the system, and intake amounts described in the reference intakes can be used. For example, it is possible to use intake amounts described in "Dietary Fiber, g" corresponding to the age and the sex of the user from the list in "Table A7-1, Daily Nutritional Goals for Age-Sex Groups Based on Dietary Reference Intakes & Dietary Guidelines Recommendations" in "Appendix 7, Nutritional Goals for Age-Sex Groups Based on Dietary Reference Intakes & Dietary Guidelines Recommendations" in "2015-2020 Dietary Guidelines for Americans" as target intake amounts for dietary fiber in the U.S.

Alternatively, if the birthplace or residence of the user is U.K., it is possible to select and use intake amounts described in "Age group" in accordance with the age of the user from the list in "Dietary Fibre" in "Nutrition Requirements _Revised August 2019" as the target intake amounts for dietary fiber. The reference intakes for each nutrient component released in each nation are not limited to the above description, and it is possible to acquire them from data stored in the storage unit or information from websites of an administrative agency or a research institute in each nation released through the network N.

Also, when target information acquired from another server or the like on the basis of information regarding the user is provided to the user terminal 20 by the server 10 as described above, and the user terminal 20 displays an input screen of the target information illustrated in FIG. 17B as described above, target information (each target amount) provided from the server 10 may be displayed. In this case, the user can recognize typical target information in his/her situation (an age, a sex, a birthplace, a religion, and the like) and appropriately set his/her target information. Therefore, the user can set target information within an achievable range.

In the information processing system 100 in each of the aforementioned embodiments, the control unit 11 of the server 10 may take foods that the user has not purchased or taken from among foods into consideration in addition to the user information stored in the member information DB 12b when the advice information to be provided to the user is generated. In this manner, it is possible to generate advice information for recommending purchase of foods that the user has not purchased or taken, for example. Note that the information regarding foods that the user has not purchased or taken may be input to and registered in the server 10 via the user terminal 20 by the user. Also, the server 10 may specify foods other than foods that the user has already purchased from among foods as sales targets and regard the specified foods as foods that the user has not purchased or taken.

In the information processing system 100 in each of the aforementioned embodiments, a providing destination to which the server 10 provides advice is not limited to the user terminal 20 and may be a terminal (user terminal 20) of a guardian, a family member, a friend, a care giver, or the like of the user, for example. In this case, it is possible to provide advice information to a guardian or the like of the user in a case where the server 10 generates advice information for the user by registering the terminal that is the providing destination of the advice in the server 10 in advance. Therefore, it is possible to share various kinds of information regarding meals, exercise, sleep, and the like of the user between the user and the guardian or the like and to expect that this may leads to an improvement in daily habits of the user, by providing the advice information to someone other than the user himself/herself.

The information processing system 100 in each of the aforementioned embodiments may be configured such that the processing realized by the user terminal 20 executing the advice APP 22AP is divided into a plurality of application programs. For example, a configuration in which processing of the user terminal 20 acquiring information (user information) regarding users via the input unit 24 and the like and causing the server 10 to accumulate the information and the processing of acquiring the advice in accordance with the user information accumulated in the server 10 from the server 10 are realized by different application programs may be employed. In this case, the control unit 21 of the user terminal 20 performs processing of receiving user information such as attribute information, profile information, biological information, exercise information, sleep information, types of foods that the user has eaten, amounts of intake and timings of intake (user intake information), information regarding real feelings, and the like of the user via the input unit 24 and transmitting the user information to the server 10 by executing the program for accumulating the user information.

Also, the control unit 21 performs processing of transmitting a member ID (identification information) of the user to the server 10, requesting advice, acquiring advice in accordance with the user information of the user from the server 10, and displaying the advice on the display unit 25 by executing the program for acquiring the advice. Even in a case where such a configuration is employed, it is possible to perform processing similar to that in each of the aforementioned embodiments, and similar effects can be obtained. Note that the user information accumulated in the server 10 may be input via an application program stored in the user terminal 20 or may be input via a predetermined website released via the network N. Also, in a case where the user information is stored in a different storage device, the server 10 may be configured to acquire the user information from the different device.

Also, the processing performed by the server 10 may be distributed to a plurality of servers in the information processing system 100 in each of the aforementioned embodiments. For example, the processing of accumulating user information that the server 10 acquires from each user terminal 20 and the processing of providing advice in accordance with the user information accumulated by the server 10 to each user terminal 20 may be performed by different servers. In this case, the accumulation server that accumulates the user information performs processing of acquiring various kinds of information (user information) related to the users input by using the user terminals 20 and accumulates the information in the member information DB 12b as illustrated in FIG. 4.

On the other hand, the providing server that provides advice performs processing of acquiring user information of a user corresponding to a user terminal 20 from the accumulation server in response to a request from the user terminal 20, generating advice in accordance with the acquired user information on the basis of details stored in the advice DB 12d as illustrated in FIG. 5, for example, and outputting the advice to the user terminal 20. At this time, the providing server acquires a member ID (identification information) of the user from the user terminal 20, for example, and receives the request of the advice, acquires the user information corresponding to the received member ID from the accumulation server, and generates the advice in accordance with the acquired user information. The user terminal 20 that has acquired the thus generated advice can provide the advice in accordance with the user information to the user of the user terminal 20 by displaying the acquired advice on the display unit 25. For example, a configuration in which a sales company that sells foods accumulates user information in an accumulation server and a providing company that provides advice provides advice suitable for each user by using the accumulated user information can be employed.

In the aforementioned first to third embodiments, the user intake information is one example of the intake information, the user feeling information is one example of the feeling information, the user biological information is one example of the biological information, the user behavior information is one example of the behavior information, the user real feeling score information is one example of the real feeling score information, the user attribute information is one example of the behavior information, the user preference is one example of the preference, the user environment information is one example of the environment information, and the virtual user behavior information is one example of the virtual behavior information.

Although the embodiments have been described above, these embodiments have been proposed as examples and are not intended to limit the scope of the invention. These embodiments can be implemented in other various forms, and various omissions, replacements, modifications, and combinations can be performed without departing from the gist of the invention.

Note that the server 10, the user terminal 20, and the wearable device 30 included in the aforementioned information processing system 100 may be realized by computers. In that case, a program that realizes functions of each functional block is recorded in a computer-readable recording medium. The program recorded in the recording medium may be realized by causing a computer system to read it and by a CPU executing it. The "computer system" described here is assumed to include hardware such as an operating system (OS), peripheral devices, and the like.

Also, the "computer-readable recording medium" refers to a portable medium such as a flexible disk, a magneto-optical disk, a ROM, or a CD-ROM. Moreover, the "computer-readable recording medium" includes a storage device such as a hard disk incorporated in the computer system.

Furthermore, the "computer-readable recording medium" may include a recording medium that dynamically holds the program for a short period of time. The recording medium that dynamically holds the program for a short period of time is, for example, a communication line in a case where the program is transmitted via a network such as the Internet or a communication network line such as a telephone network line.

Additionally, the "computer-readable recording medium" may include a recording medium that holds the program for a specific period of time, such as a volatile memory inside a computer system that servers as a server or a client. Also, the above program may be for realizing some of the aforementioned functions. Also, the above program may be able to realize the aforementioned functions by being combined with a program that has already been recorded in the computer system. Also, the above program may be realized by a programmable logic device. The programmable logic device is, for example, a field programmable gate array (FPGA).

The embodiments disclosed herein are illustrative examples in any sense and should not be considered as limitations. The scope of the present invention is indicated by the claims rather than the above sense, and all modifications within the meanings and the scope of the claims are intended to be included therein.

### [Reference Signs List]

100 Information processing system
10 Server
11 Control unit
12 Storage unit
13 Communication unit
20 User terminal
21 Control unit
22 Storage unit
23 Communication unit
25 Display unit
27 Camera
30 Wearable device
12a Product information DB
12b Member information DB
12c Advice DB

## Claims

1. A computer program product (12P) comprising instructions which, when the program is executed by a computer (10), cause the computer to:
acquire, from a terminal (20) of a user, intake information regarding a food that the user has taken:
specify an amount of achievement by which a nutrient component has already been taken based on the intake information;
acquire, from the terminal of the user, real feeling score information, which represents a score derived from one or more pieces of score information from among items of feeling information, biological information, and behavior information of the user; store, to a storage unit (12), the acquired intake information, the amount of achievement, the real feeling score information, and identification information of the user;
acquire, from the terminal of the user, information shared with another user in regard to the food, the shared information including information specifying a target intake amount of the user for the nutrient component;
store, to the storage unit, the target intake amount;
read, from the storage unit, the target intake amount of the nutrient component and the amount of achievement;
acquire an amount of the nutrient component of possible achievement that can be achieved by eating the food;
specify a proposed food based on the target intake information, the amount of achievement, and the amount of the nutrient component of possible achievement;
generate, based on the proposed food, the advice information including information regarding a correlation between the intake information and the real feeling score information, and information regarding an intake method and a cooking method using the proposed food for realizing the target intake amount for the nutrient component; and
output, to the terminal of the user, the advice information.

2. The computer program product according to claim 1, wherein the information regarding the correlation between the intake information and the real feeling score information is information based on data obtained in a nation with a PDI value within ±20 with respect to a PDI value of a nation to which the user belongs.

3. The computer program product according to claim 1 or 2, wherein the food that the user has taken includes a food leading to relatively low real feeling scores but highly required in terms of a preference and/or daily habits, on the basis of the information regarding the correlation between the intake information and the real feeling score information.

4. The computer program product according to any one of claims 1 to 3, wherein the real feeling score information includes time-series data of the feeling information.

5. The computer program product according to claim 4, wherein the time-series data includes data before and after the user takes a food.

6. The computer program product according to any one of claims 1 to 5, wherein the advice information includes virtual intake information for raising a real feeling score per unit time on the basis of the information regarding the correlation between the intake information and the real feeling score information.

7. The computer program product according to claim 6, wherein the advice information includes virtual intake information for raising a real feeling score per unit time including a food leading to a relatively low real feeling score but highly required in terms of a preference and/or daily habits, on the basis of the information regarding the correlation between the intake information and the real feeling score information.

8. The computer program product according to claim 6 or 7, wherein the advice information includes virtual intake information for raising a real feeling score per unit time without any change in behavior information, on the basis of the information regarding the correlation between the intake information and the real feeling score information.

9. The computer program product according to claim 8, wherein the advice information includes virtual behavior information for raising a real feeling score per unit time.

10. The computer program product according to any one of claims 1 to 9, wherein the advice information includes either or both of advice for improving daily habits and advice for solving either or both of information regarding anxiety and information regarding a stress due to a change in behavior of the user.

11. The computer program product according to any one of claims 1 to 10,
wherein the program causes the computer to
output advice information at an arbitrary timing and then acquire one or more pieces of information from among feeling information regarding a feeling of a user who has followed the advice information or a user who has not followed the advice information, biological information, and behavior information and/or real feeling score information derived from such information,
output, to the information processing unit, the acquired intake information, one or more pieces from among the feeling information, the biological information, and the behavior information and/or the real feeling score information derived from such information and the identification information of the user, and
acquire, from the information processing unit, properness verification information for information regarding a correlation when the advice information is output.

12. The computer program product according to claim 11, wherein the program causes the computer to newly perform information processing by using the one or more pieces of information from among the feeling information regarding the feeling of the user who has followed the advice information and the user who has not followed the advice information, the biological information, and the behavior information and/or the real feeling score information derived from such information, on the basis of the acquired properness verification information.

13. The computer program product according to claim 12, wherein the program causes the computer to newly acquire, from the information processing unit, advice information including information regarding a correlation between the intake information and the real feeling score information.

14. An information processing method that is executed by a computer (10), comprising:
acquiring, from a terminal (20) of a user, intake information regarding a food that a user has taken;
specifying an amount of achievement by which a nutrient component has already been taken based on the intake information;
acquiring, from the terminal of the user, real feeling score information, which represents a score derived from one or more pieces of score information from among items of feeling information regarding a feeling of the user, biological information, and behavior information of the user;
storing, to a storage unit (12), the acquired intake information, the amount of achievement, the real feeling score information, and identification information of the user;
acquiring, from the terminal of the user, information shared with another user in regard to the food, the shared information including information specifying a target intake amount of the user for the nutrient component;
storing, to the storage unit, the target intake amount;
reading, from the storage unit, the target intake amount of the nutrient component and the amount of achievement;
acquiring an amount of the nutrient component of possible achievement that can be achieved by eating the food;
specifying a proposed food based on the target intake information, the amount of achievement, and the amount of the nutrient component of possible achievement;
generating, based on the proposed food, the advice information including information regarding a correlation between the intake information and the real feeling score information, and information regarding an intake method and a cooking method using the proposed food for realizing the target intake amount for the nutrient component; and
outputting, to the terminal of the user, the advice information.

15. An information processing device (10) comprising:
a registration unit (12) that registers a user;
an acquisition unit (13) that acquires, from a terminal (20) of the registered user, intake information regarding a food that the user has taken, and real feeling score information, which represents a score derived from one or more pieces of score information from among items of intake information regarding a food that the user has taken, feeling information regarding a feeling of the user, biological information, and behavior information;
a storage unit (12) that stores, in an associated manner, the acquired intake information, the real feeling score information, and identification information of the user;
an output unit (13) that outputs, to the terminal of the user, advice information; and
a control unit (11) configured to specify an amount of achievement by which a nutrient component has already been taken based on the intake information; wherein
the acquisition unit is configured to receive acquired information shared with another user in regard to the food, the shared information including information specifying a target intake amount of the user for the nutrient component, the storage unit is configured to store the target intake amount and the amount of achievement, and
the control unit is configured to:
read, from the storage unit, the target intake amount of the nutrient component and the amount of achievement,
acquire an amount of the nutrient component of possible achievement that can be achieved by eating the food,
specify a proposed food based on the target intake information, the amount of achievement, and the amount of the nutrient component of possible achievement, and
generate, based on the proposed food, the advice information including information regarding a correlation between the intake information and the real feeling score information, and information regarding an intake method and a cooking method using the proposed food for realizing the target intake amount for the nutrient component.

## Patentansprüche

1. Computerprogrammprodukt (12P), umfassend Anweisungen, die, wenn das Programm durch einen Computer (10) ausgeführt wird, den Computer zu Folgendem veranlassen:
Erfassen von Verzehrinformationen in Bezug auf ein Lebensmittel, das der Benutzer aufgenommen hat, von einem Endgerät (20) eines Benutzers;
Spezifizieren einer Erreichungsmenge, die von der Nährstoffkomponente basierend auf den Verzehrinformationen bereits aufgenommen wurde;
Erfassen, von dem Endgerät des Benutzers, von tatsächlichen Gefühlsbewertungsinformationen, die eine Bewertung darstellen, die von einem oder mehreren einzelnen Bewertungsinformationen aus Gefühlsinformationen, biologischen Informationen und Verhaltensinformationen des Benutzers abgeleitet ist; Speichern der erfassten Verzehrinformationen, der Erreichungsmenge, der tatsächlichen Gefühlsbewertungsinformationen und von Identifikationsinformationen des Benutzers in einer Speichereinheit (12);
Erfassen von Informationen von dem Endgerät des Benutzers, die mit einem anderen Benutzer in Bezug auf das Lebensmittel geteilt werden, wobei die geteilten Informationen Informationen enthalten, die eine Zielverzehrmenge des Benutzers für die Nährstoffkomponente spezifizieren;
Speichern der Zielverzehrmenge in der Speichereinheit;
Auslesen der Zielverzehrmenge der Nährstoffkomponente und der Erreichungsmenge aus der Speichereinheit;
Erfassen einer möglichen Erreichungsmenge der Nährstoffkomponente, die durch das Essen des Lebensmittels erreicht werden kann;
Spezifizieren eines vorgeschlagenen Lebensmittels basierend auf den Zielverzehrinformationen, der Erreichungsmenge und der möglichen Erreichungsmenge der Nährstoffkomponente;
Erzeugen, basierend auf dem vorgeschlagenen Lebensmittel, der Beratungsinformationen, die Informationen in Bezug auf eine Korrelation zwischen den Verzehrinformationen und den tatsächlichen Gefühlsbewertungsinformationen und Informationen in Bezug auf ein Verzehrverfahren und ein Kochverfahren unter Verwendung des vorgeschlagenen Lebensmittels zum Erzielen der Zielverzehrmenge für die Nährstoffkomponente enthalten; und
Ausgeben der Beratungsinformationen an das Endgerät des Benutzers.

2. Computerprogrammprodukt nach Anspruch 1, wobei die Informationen in Bezug auf die Korrelation zwischen den Verzehrinformationen und den tatsächlichen Gefühlsbewertungsinformationen Informationen sind, die auf Daten basieren, die in einer Nation mit einem PDI-Wert innerhalb von ±20 in Bezug auf einen PDI-Wert einer Nation, zu der der Benutzer gehört, erhalten wurden.

3. Computerprogrammprodukt nach Anspruch 1 oder 2, wobei das Lebensmittel, das der Benutzer aufgenommen hat, ein Lebensmittel enthält, das zu relativ niedrigen tatsächlichen Gefühlsbewertungen führt, aber in Bezug auf eine Präferenz und/oder tägliche Gewohnheiten basierend auf den Informationen in Bezug auf die Korrelation zwischen den Verzehrinformationen und den tatsächlichen Gefühlsbewertungsinformationen hochgradig erforderlich ist.

4. Computerprogrammprodukt nach einem der Ansprüche 1 bis 3, wobei die tatsächlichen Gefühlsbewertungsinformationen Zeitreihendaten der Gefühlsinformationen enthalten.

5. Computerprogrammprodukt nach Anspruch 4, wobei die Zeitreihendaten Daten vor und nach der Aufnahme eines Lebensmittels durch den Benutzer enthalten.

6. Computerprogrammprodukt nach einem der Ansprüche 1 bis 5, wobei die Beratungsinformationen virtuelle Verzehrinformationen zum Erhöhen einer tatsächlichen Gefühlsbewertung pro Zeiteinheit basierend auf den Informationen in Bezug auf die Korrelation zwischen den Verzehrinformationen und den tatsächlichen Gefühlsbewertungsinformationen enthalten.

7. Computerprogrammprodukt nach Anspruch 6, wobei die Beratungsinformationen virtuelle Verzehrinformationen zum Erhöhen einer tatsächlichen Gefühlsbewertung pro Zeiteinheit einschließlich eines Lebensmittels enthalten, das zu einer relativ niedrigen tatsächlichen Gefühlsbewertung führt, aber in Bezug auf eine Präferenz und/oder tägliche Gewohnheiten hochgradig erforderlich ist, basierend auf den Informationen in Bezug auf die Korrelation zwischen den Verzehrinformationen und den tatsächlichen Gefühlsbewertungsinformationen.

8. Computerprogrammprodukt nach Anspruch 6 oder 7, wobei die Beratungsinformationen virtuelle Verzehrinformationen zum Erhöhen einer tatsächlichen Gefühlsbewertung pro Zeiteinheit ohne jegliche Änderung von Verhaltensinformationen basierend auf den Informationen in Bezug auf die Korrelation zwischen den Verzehrinformationen und den tatsächlichen Gefühlsbewertungsinformationen enthalten.

9. Computerprogrammprodukt nach Anspruch 8, wobei die Beratungsinformationen virtuelle Verhaltensinformationen zum Erhöhen einer tatsächlichen Gefühlsbewertung pro Zeiteinheit enthalten.

10. Computerprogrammprodukt nach einem der Ansprüche 1 bis 9, wobei die Beratungsinformationen entweder eines oder beides von einer Beratung zum Verbessern von täglichen Gewohnheiten und Beratung zum Lösen von entweder einem oder beidem von Informationen in Bezug auf Angst und Informationen in Bezug auf Stress aufgrund einer Änderung beim Verhalten des Benutzers enthalten.

11. Computerprogrammprodukt nach einem der Ansprüche 1 bis 10, wobei das Programm den Computer zu Folgendem veranlasst
Ausgeben von Beratungsinformationen zu einem willkürlichen Zeitpunkt und dann Erfassen einer oder mehrerer einzelner Informationen aus Gefühlsinformationen in Bezug auf ein Gefühl eines Benutzers, der den Beratungsinformationen gefolgt ist, oder eines Benutzers, der den Beratungsinformationen nicht gefolgt ist, biologischen Informationen und Verhaltensinformationen und/oder tatsächlichen Gefühlsbewertungsinformationen, die von solchen Informationen abgeleitet sind,
Ausgeben, an die Informationsverarbeitungseinheit, der erfassten Verzehrinformationen, einer oder mehrerer einzelner aus den Gefühlsinformationen, den biologischen Informationen und den Verhaltensinformationen und/oder den tatsächlichen Gefühlsbewertungsinformationen, die von solchen Informationen abgeleitet sind, und der Identifikationsinformationen des Benutzers und
Erfassen von Angemessenheitsprüfungsinformationen von der Informationsverarbeitungseinheit für Informationen in Bezug auf eine Korrelation, wenn die Beratungsinformationen ausgegeben werden.

12. Computerprogrammprodukt nach Anspruch 11, wobei das Programm den Computer veranlasst, eine Informationsverarbeitung unter Verwendung der einen oder mehreren einzelnen Informationen aus den Gefühlsinformationen in Bezug auf das Gefühl des Benutzers, der den Beratungsinformationen gefolgt ist, und des Benutzers, der den Beratungsinformationen nicht gefolgt ist, den biologischen Informationen und den Verhaltensinformationen und/oder den tatsächlichen Gefühlsbewertungsinformationen, die von solchen Informationen abgeleitet sind, basierend auf den erfassten Angemessenheitsprüfungsinformationen erneut durchzuführen.

13. Computerprogrammprodukt nach Anspruch 12, wobei das Programm den Computer veranlasst, von der Informationsverarbeitungseinheit erneut Beratungsinformationen zu erfassen, die Informationen in Bezug auf eine Korrelation zwischen den Verzehrinformationen und den tatsächlichen Gefühlsbewertungsinformationen enthalten.

14. Informationsverarbeitungsverfahren, das durch einen Computer (10) ausgeführt wird, umfassend:
Erfassen von Verzehrinformationen in Bezug auf ein Lebensmittel, das ein Benutzer aufgenommen hat, von einem Endgerät (20) eines Benutzers;
Spezifizieren einer Erreichungsmenge, die von der Nährstoffkomponente basierend auf den Verzehrinformationen bereits aufgenommen wurde;
Erfassen, von dem Endgerät des Benutzers, von tatsächlichen Gefühlsbewertungsinformationen, die eine Bewertung darstellen, die von einem oder mehreren einzelnen Bewertungsinformationen aus Gefühlsinformationen in Bezug auf ein Gefühl des Benutzers, biologischen Informationen und Verhaltensinformationen des Benutzers abgeleitet ist;
Speichern der erfassten Verzehrinformationen, der Erreichungsmenge, der tatsächlichen Gefühlsbewertungsinformationen und von Identifikationsinformationen des Benutzers in einer Speichereinheit (12);
Erfassen von Informationen von dem Endgerät des Benutzers, die mit einem anderen Benutzer in Bezug auf das Lebensmittel geteilt werden, wobei die geteilten Informationen Informationen enthalten, die eine Zielverzehrmenge des Benutzers für die Nährstoffkomponente spezifizieren;
Speichern der Zielverzehrmenge in der Speichereinheit;
Auslesen der Zielverzehrmenge der Nährstoffkomponente und der Erreichungsmenge aus der Speichereinheit;
Erfassen einer möglichen Erreichungsmenge der Nährstoffkomponente, die durch das Essen des Lebensmittels erreicht werden kann;
Spezifizieren eines vorgeschlagenen Lebensmittels basierend auf den Zielverzehrinformationen, der Erreichungsmenge und der möglichen Erreichungsmenge der Nährstoffkomponente;
Erzeugen, basierend auf dem vorgeschlagenen Lebensmittel, der Beratungsinformationen, die Informationen in Bezug auf eine Korrelation zwischen den Verzehrinformationen und den tatsächlichen Gefühlsbewertungsinformationen und Informationen in Bezug auf ein Verzehrverfahren und ein Kochverfahren unter Verwendung des vorgeschlagenen Lebensmittels zum Erzielen der Zielverzehrmenge für die Nährstoffkomponente enthalten; und
Ausgeben der Beratungsinformationen an das Endgerät des Benutzers.

15. Informationsverarbeitungsvorrichtung (10), umfassend:
eine Registrierungseinheit (12), die einen Benutzer registriert;
eine Erfassungseinheit (13), die von einem Endgerät (20) des registrierten Benutzers Verzehrinformationen in Bezug auf ein Lebensmittel, das der Benutzer aufgenommen hat, und tatsächliche Gefühlsbewertungsinformationen erfasst, die eine Bewertung darstellen, die von einer oder mehreren einzelnen Bewertungsinformationen aus Verzehrinformationen in Bezug auf ein Lebensmittel, das der Benutzer aufgenommen hat, Gefühlsinformationen in Bezug auf ein Gefühl des Benutzers, biologischen Informationen und Verhaltensinformationen abgeleitet ist;
eine Speichereinheit (12), die die erfassten Verzehrinformationen, die tatsächlichen Gefühlsbewertungsinformationen und Identifikationsinformationen des Benutzers auf eine zugeordnete Weise speichert;
eine Ausgabeeinheit (13), die Beratungsinformationen an das Endgerät des Benutzers ausgibt; und
eine Steuereinheit (11), die zum Spezifizieren einer Erreichungsmenge, die von der Nährstoffkomponente basierend auf den Verzehrinformationen bereits aufgenommen wurde, konfiguriert ist; wobei
die Erfassungseinheit zum Empfangen erfasster Informationen, die mit einem anderen Benutzer in Bezug auf das Lebensmittel geteilt werden, konfiguriert ist, wobei die geteilten Informationen Informationen enthalten, die eine Zielverzehrmenge des Benutzers für die Nährstoffkomponente spezifizieren, die Speichereinheit zum Speichern der Zielverzehrmenge und der Erreichungsmenge konfiguriert ist und
die Steuereinheit zu Folgendem konfiguriert ist:
Auslesen der Zielverzehrmenge der Nährstoffkomponente und der Erreichungsmenge aus der Speichereinheit,
Erfassen einer möglichen Erreichungsmenge der Nährstoffkomponente, die durch das Essen des Lebensmittels erreicht werden kann,
Spezifizieren eines vorgeschlagenen Lebensmittels basierend auf den Zielverzehrinformationen, der Erreichungsmenge und der möglichen Erreichungsmenge der Nährstoffkomponente und
Erzeugen, basierend auf dem vorgeschlagenen Lebensmittel, der Beratungsinformationen, die Informationen in Bezug auf eine Korrelation zwischen den Verzehrinformationen und den tatsächlichen Gefühlsbewertungsinformationen und Informationen in Bezug auf ein Verzehrverfahren und ein Kochverfahren unter Verwendung des vorgeschlagenen Lebensmittels zum Erzielen der Zielverzehrmenge für die Nährstoffkomponente enthalten.

## Revendications

1. Produit de programme informatique (12P) comprenant des instructions qui, lorsque le programme est exécuté par un ordinateur (10), amènent l'ordinateur à :
acquérir, à partir d'un terminal (20) d'un utilisateur, des informations de consommation concernant un aliment que l'utilisateur a consommé ;
spécifier une quantité d'aboutissement par laquelle un composant nutritif a déjà été consommé en fonction des informations de consommation ;
acquérir, à partir du terminal de l'utilisateur, des informations de score de ressenti réel, qui représentent un score dérivé d'une ou de plusieurs informations de score parmi des informations de ressenti, des informations biologiques et des informations de comportement de l'utilisateur ; stocker, sur une unité de stockage (12), les informations de consommation acquises, la quantité d'aboutissement, les informations de score de ressenti réel et des informations d'identification de l'utilisateur ;
acquérir, à partir du terminal de l'utilisateur, des informations partagées avec un autre utilisateur en ce qui concerne l'aliment, les informations partagées comprenant des informations spécifiant une quantité de consommation cible de l'utilisateur pour le composant nutritif ;
stocker, dans l'unité de stockage, la quantité de consommation cible ;
lire, à partir l'unité de stockage, la quantité de consommation cible du composant nutritif et la quantité d'aboutissement ;
acquérir une quantité d'aboutissement possible du composant nutritif qui peut être atteinte en mangeant l'aliment ;
spécifier un aliment proposé sur la base des informations de consommation cible, de la quantité d'aboutissement et de la quantité d'aboutissement possible du composant nutritif ;
générer, sur la base de l'aliment proposé, les informations de conseil comprenant des informations concernant une corrélation entre les informations de consommation et les informations de score de ressenti réel, et des informations concernant un procédé de consommation et un procédé de cuisson utilisant l'aliment proposé pour parvenir à la quantité de consommation cible pour le composant nutritif ; et
émettre en sortie, sur le terminal de l'utilisateur, les informations de conseil.

2. Produit de programme informatique selon la revendication 1, dans lequel les informations concernant la corrélation entre les informations de consommation et les informations de score de ressenti réel sont des informations basées sur des données obtenues dans une nation avec une valeur PDI à ±20 d'une valeur PDI d'une nation à laquelle l'utilisateur appartient.

3. Produit de programme informatique selon la revendication 1 ou 2, dans lequel l'aliment que l'utilisateur a consommé comprend un aliment conduisant à des scores de ressenti réel relativement bas, mais hautement requis en termes de préférence et/ou d'habitudes quotidiennes, sur la base des informations concernant la corrélation entre les informations de consommation et les informations de score de ressenti réel.

4. Produit de programme informatique selon l'une quelconque des revendications 1 à 3, dans lequel les informations de score de ressenti réel comprennent des données de série temporelle des informations de ressenti.

5. Produit de programme informatique selon la revendication 4, dans lequel les données de série temporelle comprennent des données avant et après la consommation d'un aliment par l'utilisateur.

6. Produit de programme informatique selon l'une quelconque des revendications 1 à 5, dans lequel les informations de conseil comprennent des informations de consommation virtuelle destinées à augmenter un score de ressenti réel par unité temporelle sur la base des informations concernant la corrélation entre les informations de consommation et les informations de score de ressenti réel.

7. Produit de programme informatique selon la revendication 6, dans lequel les informations de conseil comprennent des informations de consommation virtuelle destinées à augmenter un score de ressenti réel par unité temporelle comprenant un aliment conduisant à un score de ressenti réel relativement bas, mais hautement requis en termes de préférence et/ou d'habitudes quotidiennes, sur la base des informations concernant la corrélation entre les informations de consommation et les informations de score de ressenti réel.

8. Produit de programme informatique selon la revendication 6 ou 7, dans lequel les informations de conseil comprennent des informations de consommation virtuelle destinées à augmenter un score de ressenti réel par unité temporelle sans aucun changement d'informations de comportement, sur la base des informations concernant la corrélation entre les informations de consommation et les informations de score de ressenti réel.

9. Produit de programme informatique selon la revendication 8, dans lequel les informations de conseil comprennent des informations de comportement virtuel destinées à augmenter un score de ressenti réel par unité temporelle.

10. Produit de programme informatique selon l'une quelconque des revendications 1 à 9, dans lequel les informations de conseil comprennent l'un ou l'autre ou les deux parmi des conseils pour améliorer des habitudes quotidiennes et des conseils pour résoudre l'une ou l'autre ou les deux parmi des informations concernant de l'anxiété et des informations concernant un stress dû à un changement de comportement de l'utilisateur.

11. Produit de programme informatique selon l'une quelconque des revendications 1 à 10, dans lequel le programme amène l'ordinateur à
émettre en sortie des informations de conseil à un moment arbitraire, puis acquérir une ou plusieurs informations parmi des informations de ressenti concernant un ressenti d'un utilisateur qui a suivi les informations de conseil ou d'un utilisateur qui n'a pas suivi les informations de conseil, des informations biologiques et des informations de comportement et/ou des informations de score de ressenti réel dérivées de telles informations,
émettre en sortie, vers l'unité de traitement d'informations, les informations de consommation acquises, une ou plusieurs informations parmi les informations de ressenti, les informations biologiques et les informations de comportement et/ou les informations de score de ressenti réel dérivées de telles informations et des informations d'identification de l'utilisateur, et
acquérir, à partir de l'unité de traitement d'informations, des informations de vérification de pertinence pour des informations concernant une corrélation lorsque les informations de conseil sont émises en sortie.

12. Produit de programme informatique selon la revendication 11, dans lequel le programme amène l'ordinateur à réaliser nouvellement un traitement d'informations en utilisant une ou plusieurs informations parmi les informations de ressenti concernant le ressenti de l'utilisateur qui a suivi les informations de conseil et de l'utilisateur qui n'a pas suivi les informations de conseil, les informations biologiques et les informations de comportement et/ou les informations de score de ressenti réel dérivées de telles informations, sur la base des informations de vérification de pertinence acquises.

13. Produit de programme informatique selon la revendication 12, dans lequel le programme amène l'ordinateur à acquérir nouvellement, à partir de l'unité de traitement d'informations, des informations de conseil comprenant des informations concernant une corrélation entre les informations de consommation et les informations de score de ressenti réel.

14. Procédé de traitement d'informations qui est exécuté par un ordinateur (10), comprenant :
l'acquisition, à partir d'un terminal (20) d'un utilisateur, d'informations de consommation concernant un aliment qu'un utilisateur a consommé ;
la spécification d'une quantité d'aboutissement par laquelle un composant nutritif a déjà été consommé en fonction des informations de consommation ;
l'acquisition, à partir du terminal de l'utilisateur, d'informations de score de ressenti réel, qui représentent un score dérivé d'une ou de plusieurs informations de score parmi des informations de ressenti concernant un ressenti de l'utilisateur, des informations biologiques et des informations de comportement de l'utilisateur ;
le stockage, sur une unité de stockage (12), des informations de consommation acquises, la quantité d'aboutissement, les informations de score de ressenti réel et des informations d'identification de l'utilisateur ;
l'acquisition, à partir du terminal de l'utilisateur, d'informations partagées avec un autre utilisateur en ce qui concerne l'aliment, les informations partagées comprenant des informations spécifiant une quantité de consommation cible de l'utilisateur pour le composant nutritif ;
le stockage, dans l'unité de stockage, de la quantité de consommation cible ;
la lecture, à partir l'unité de stockage, de la quantité de consommation cible du composant nutritif et la quantité d'aboutissement ;
l'acquisition d'une quantité d'aboutissement possible du composant nutritif qui peut être atteinte en mangeant l'aliment ;
la spécification d'un aliment proposé sur la base des informations de consommation cible, de la quantité d'aboutissement et de la quantité d'aboutissement possible du composant nutritif ;
la génération, sur la base de l'aliment proposé, des informations de conseil comprenant des informations concernant une corrélation entre les informations de consommation et les informations de score de ressenti réel, et des informations concernant un procédé de consommation et un procédé de cuisson utilisant l'aliment proposé pour parvenir à la quantité de consommation cible pour le composant nutritif ; et
l'émission en sortie, sur le terminal de l'utilisateur, des informations de conseil.

15. Dispositif de traitement d'informations (10), comprenant :
une unité d'enregistrement (12) qui enregistre un utilisateur ;
une unité d'acquisition (13) qui acquiert, à partir d'un terminal (20) de l'utilisateur enregistré, des informations de consommation concernant un aliment que l'utilisateur a consommé, et des informations de score de ressenti réel, qui représentent un score dérivé d'une ou de plusieurs informations de score parmi des informations de consommation concernant un aliment que l'utilisateur a consommé, des informations de ressenti concernant un ressenti de l'utilisateur, des informations biologiques et des informations de comportement ;
une unité de stockage (12) qui stocke, de manière associée, les informations de consommation acquises, les informations de score de ressenti réel et des informations d'identification de l'utilisateur ;
une unité de sortie (13) qui émet en sortie, sur le terminal de l'utilisateur, des informations de conseil ; et
une unité de commande (11) configurée pour spécifier une quantité d'aboutissement par laquelle un composant nutritif a déjà été consommé en fonction des informations de consommation ; dans lequel
l'unité d'acquisition est configurée pour recevoir des informations acquises partagées avec un autre utilisateur concernant l'aliment, les informations partagées comprenant des informations spécifiant une quantité de consommation cible de l'utilisateur pour le composant nutritif, l'unité de stockage est configurée pour stocker la quantité de consommation cible et la quantité d'aboutissement, et
l'unité de commande est configurée pour :
lire, à partir l'unité de stockage, la quantité de consommation cible du composant nutritif et la quantité d'aboutissement,
acquérir une quantité d'aboutissement possible du composant nutritif qui peut être atteinte en mangeant l'aliment,
spécifier un aliment proposé sur la base des informations de consommation cible, de la quantité d'aboutissement et de la quantité d'aboutissement possible du composant nutritif, et
générer, sur la base de l'aliment proposé, les informations de conseil comprenant des informations concernant une corrélation entre les informations de consommation et les informations de score de ressenti réel, et des informations concernant un procédé de consommation et un procédé de cuisson utilisant l'aliment proposé pour parvenir à la quantité de consommation cible pour le composant nutritif.
